# EUROPEAN PATENT SPECIFICATION

(11) **EP 3 060 239 B1**
(45) Date of publication and mention of the grant of the patent: **13.05.2020**
(21) Application number: 14856666.4
(22) Date of filing: 21.10.2014
(51) Int. Cl.: A61K 38/27, A61K 38/24

(54) **LONG-ACTING POLYPEPTIDES AND METHODS OF PRODUCING AND ADMINISTERING SAME**
POLYPEPTIDE MIT LANGZEITWIRKUNG SOWIE VERFAHREN ZUR HERSTELLUNG UND VERABREICHUNG DAVON
POLYPEPTIDES À ACTION PROLONGÉE ET PROCÉDÉS DE PRODUCTION ET D'ADMINISTRATION DE CEUX-CI

(30) Priority: 21.10.2013 US 201314059134; 19.06.2014 US 201414309496
(43) Date of publication of application: 31.08.2016
(73) Proprietor: OPKO Biologics Ltd., Kiryat Gat, 8211804 (IL)
(72) Inventor: FARES, Fuad, 25155 Hourfish Village (IL); FIMA, Udi Eyal, 52330 Dvira (IL)
(74) Representative: Pearl Cohen Zedek Latzer Baratz UK LLP
(86) International application number: PCT/IL2014/050910
(87) International publication number: WO 2015/059695

(56) References cited:
- US-A1- 2010 081 614
- US-A1- 2013 184 207
- US-A1- 2013 184 207
- FARES, FUAD ET AL.: 'Designing a long-acting human growth hormone (hGH) by fusing the carboxyl-terminal peptide of human chorionic gonadotropin ?-subunit to the coding sequence of hGH.' ENDOCRINOLOGY vol. 151, no. 9, 21 July 2010, pages 4410 - 4417, XP002692130
- CAWLEY, PIPPA; ET AL.: 'Developing long?acting growth hormone formulations.' CLINICAL ENDOCRINOLOGY vol. 79, no. 3, 2013, pages 305 - 309., XP055185642

### SEQUENCE LISTING

<110> **OPKO BIOLOGICS LTD.**
   FARES, Fuad
   FIMA, Udi Eyal
<120> LONG-ACTING POLYPEPTIDES AND METHODS OF PRODUCING AND ADMINISTERING SAME
<130> P-9520-PC6
<150> 14/059,134
   <151> 2013-10-21
<150> 14/309,496
   <151> 2014-06-19
<160> 50
<170> PatentIn version 3.5
<210> 1
   <211> 221
   <212> PRT
   <213> Homo sapiens
<400> 1
   Met Gly Val His Glu Cys Pro Ala Trp Leu Trp Leu Leu Leu Ser Leu
   1               5                   10                  15
   Leu Ser Leu Pro Leu Gly Leu Pro Val Leu Gly Ala Pro Pro Arg Leu
               20                  25                  30
   Ile Cys Asp Ser Arg Val Leu Glu Arg Tyr Leu Leu Glu Ala Lys Glu
           35                  40                  45
   Ala Glu Asn Ile Thr Thr Gly Cys Ala Glu His Cys Ser Leu Asn Glu
       50                  55                  60
   Asn Ile Thr Val Pro Asp Thr Lys Val Asn Phe Tyr Ala Trp Lys Arg
   65                  70                  75                  80
   Met Glu Val Gly Gln Gln Ala Val Glu Val Trp Gln Gly Leu Ala Leu
                   85                  90                  95
   Leu Ser Glu Ala Val Leu Arg Gly Gln Ala Leu Leu Val Asn Ser Ser
               100                 105                 110
   Gln Pro Trp Glu Pro Leu Gln Leu His Val Asp Lys Ala Val Ser Gly
           115                 120                 125
   Leu Arg Ser Leu Thr Thr Leu Leu Arg Ala Leu Gly Ala Gln Lys Glu
       130                 135                 140
   Ala Ile Ser Pro Pro Asp Ala Ala Ser Ala Ala Pro Leu Arg Thr Ile
   145 150 155 160
   Thr Ala Asp Thr Phe Arg Lys Leu Phe Arg Val Tyr Ser Asn Phe Leu
   165 170 175
   Arg Gly Lys Leu Lys Leu Tyr Thr Gly Glu Ala Cys Arg Thr Gly Asp
   180 185 190
   Arg Ser Ser Ser Ser Lys Ala Pro Pro Pro Ser Leu Pro Ser Pro Ser
   195 200 205
   Arg Leu Pro Gly Pro Ser Asp Thr Pro Ile Leu Pro Gln
   210 215 220
<210> 2
   <211> 249
   <212> **PRT**
   <213> Homo sapiens
<400> 2
   Met Gly Val His Glu Cys Pro Ala Trp Leu Trp Leu Leu Leu Ser Leu
   1 5 10 15
   Leu Ser Leu Pro Leu Gly Leu Pro Val Leu Gly Ala Pro Pro Arg Leu
   20 25 30
   Ile Cys Asp Ser Arg Val Leu Glu Arg Tyr Leu Leu Glu Ala Lys Glu
   35 40 45
   Ala Glu Asn Ile Thr Thr Gly Cys Ala Glu His Cys Ser Leu Asn Glu
   50 55 60
   Asn Ile Thr Val Pro Asp Thr Lys Val Asn Phe Tyr Ala Trp Lys Arg
   65 70 75 80
   Met Glu Val Gly Gln Gln Ala Val Glu Val Trp Gln Gly Leu Ala Leu
   85 90 95
   Leu Ser Glu Ala Val Leu Arg Gly Gln Ala Leu Leu Val Asn Ser Ser
   100 105 110
   Gln Pro Trp Glu Pro Leu Gln Leu His Val Asp Lys Ala Val Ser Gly
   115 120 125
   Leu Arg Ser Leu Thr Thr Leu Leu Arg Ala Leu Gly Ala Gln Lys Glu
   130 135 140
   Ala Ile Ser Pro Pro Asp Ala Ala Ser Ala Ala Pro Leu Arg Thr Ile
   145                 150                 155                 160
   Thr Ala Asp Thr Phe Arg Lys Leu Phe Arg Val Tyr Ser Asn Phe Leu
                   165                 170                 175
   Arg Gly Lys Leu Lys Leu Tyr Thr Gly Glu Ala Cys Arg Thr Gly Asp
               180                 185                 190
   Arg Ser Ser Ser Ser Lys Ala Pro Pro Pro Ser Leu Pro Ser Pro Ser
           195                 200                 205
   Arg Leu Pro Gly Pro Ser Asp Thr Pro Ile Leu Pro Gln Ser Ser Ser
       210                 215                 220
   Ser Lys Ala Pro Pro Pro Ser Leu Pro Ser Pro Ser Arg Leu Pro Gly
   225                 230                 235                 240
   Pro Ser Asp Thr Pro Ile Leu Pro Gln
                   245
<210> 3
   <211> 277
   <212> PRT
   <213> Homo sapiens
<400> 3
   Met Gly Val His Glu Cys Pro Ala Trp Leu Trp Leu Leu Leu Ser Leu
   1               5                   10                  15
   Leu Ser Leu Pro Leu Gly Leu Pro Val Leu Gly Ser Ser Ser Ser Lys
               20                  25                  30
   Ala Pro Pro Pro Ser Leu Pro Ser Pro Ser Arg Leu Pro Gly Pro Ser
           35                  40                  45
   Asp Thr Pro Ile Leu Pro Gln Ala Pro Pro Arg Leu Ile Cys Asp Ser
       50                  55                  60
   Arg Val Leu Glu Arg Tyr Leu Leu Glu Ala Lys Glu Ala Glu Asn Ile
   65                  70                  75                  80
   Thr Thr Gly Cys Ala Glu His Cys Ser Leu Asn Glu Asn Ile Thr Val
                   85                  90                  95
   Pro Asp Thr Lys Val Asn Phe Tyr Ala Trp Lys Arg Met Glu Val Gly
               100                 105                 110
   Gln Gln Ala Val Glu Val Trp Gln Gly Leu Ala Leu Leu Ser Glu Ala
   115 120 125
   Val Leu Arg Gly Gln Ala Leu Leu Val Asn Ser Ser Gln Pro Trp Glu
   130 135 140
   Pro Leu Gln Leu His Val Asp Lys Ala Val Ser Gly Leu Arg Ser Leu
   145 150 155 160
   Thr Thr Leu Leu Arg Ala Leu Gly Ala Gln Lys Glu Ala Ile Ser Pro
   165 170 175
   Pro Asp Ala Ala Ser Ala Ala Pro Leu Arg Thr Ile Thr Ala Asp Thr
   180 185 190
   Phe Arg Lys Leu Phe Arg Val Tyr Ser Asn Phe Leu Arg Gly Lys Leu
   195 200 205
   Lys Leu Tyr Thr Gly Glu Ala Cys Arg Thr Gly Asp Arg Ser Ser Ser
   210 215 220
   Ser Lys Ala Pro Pro Pro Ser Leu Pro Ser Pro Ser Arg Leu Pro Gly
   225 230 235 240
   Pro Ser Asp Thr Pro Ile Leu Pro Gln Ser Ser Ser Ser Lys Ala Pro
   245 250 255
   Pro Pro Ser Leu Pro Ser Pro Ser Arg Leu Pro Gly Pro Ser Asp Thr
   260 265 270
   Pro Ile Leu Pro Gln
   275
<210> 4
   <211> 387
   <212> PRT
   <213> Homo sapiens
<400> 4
   Met Gly Val His Glu Cys Pro Ala Trp Leu Trp Leu Leu Leu Ser Leu
   1 5 10 15
   Leu Ser Leu Pro Leu Gly Leu Pro Val Leu Gly Ala Pro Pro Arg Leu
   20 25 30
   Ile Cys Asp Ser Arg Val Leu Glu Arg Tyr Leu Leu Glu Ala Lys Glu
   35 40 45
   Ala Glu Asn Ile Thr Thr Gly Cys Ala Glu His Cys Ser Leu Asn Glu
       50                  55                  60
   Asn Ile Thr Val Pro Asp Thr Lys Val Asn Phe Tyr Ala Trp Lys Arg
   65                  70                  75                  80
   Met Glu Val Gly Gln Gln Ala Val Glu Val Trp Gln Gly Leu Ala Leu
                   85                  90                  95
   Leu Ser Glu Ala Val Leu Arg Gly Gln Ala Leu Leu Val Asn Ser Ser
               100                 105                 110
   Gln Pro Trp Glu Pro Leu Gln Leu His Val Asp Lys Ala Val Ser Gly
           115                 120                 125
   Leu Arg Ser Leu Thr Thr Leu Leu Arg Ala Leu Gly Ala Gln Lys Glu
       130                 135                 140
   Ala Ile Ser Pro Pro Asp Ala Ala Ser Ala Ala Pro Leu Arg Thr Ile
   145                 150                 155                 160
   Thr Ala Asp Thr Phe Arg Lys Leu Phe Arg Val Tyr Ser Asn Phe Leu
                   165                 170                 175
   Arg Gly Lys Leu Lys Leu Tyr Thr Gly Glu Ala Cys Arg Thr Gly Asp
               180                 185                 190
   Arg Ser Ser Ser Ser Lys Ala Pro Pro Pro Ser Leu Pro Ser Pro Ser
           195                 200                 205
   Arg Leu Pro Gly Pro Ser Asp Thr Pro Ile Leu Pro Gln Ala Pro Pro
       210                 215                 220
   Arg Leu Ile Cys Asp Ser Arg Val Leu Glu Arg Tyr Leu Leu Glu Ala
   225                 230                 235                 240
   Lys Glu Ala Glu Asn Ile Thr Thr Gly Cys Ala Glu His Cys Ser Leu
                   245                 250                 255
   Asn Glu Asn Ile Thr Val Pro Asp Thr Lys Val Asn Phe Tyr Ala Trp
               260                 265                 270
   Lys Arg Met Glu Val Gly Gln Gln Ala Val Glu Val Trp Gln Gly Leu
           275                 280                 285
   Ala Leu Leu Ser Glu Ala Val Leu Arg Gly Gln Ala Leu Leu Val Asn
       290                 295                 300
   Ser Ser Gln Pro Trp Glu Pro Leu Gln Leu His Val Asp Lys Ala Val
   305 310 315 320
   Ser Gly Leu Arg Ser Leu Thr Thr Leu Leu Arg Ala Leu Gly Ala Gln
   325 330 335
   Lys Glu Ala Ile Ser Pro Pro Asp Ala Ala Ser Ala Ala Pro Leu Arg
   340 345 350
   Thr Ile Thr Ala Asp Thr Phe Arg Lys Leu Phe Arg Val Tyr Ser Asn
   355 360 365
   Phe Leu Arg Gly Lys Leu Lys Leu Tyr Thr Gly Glu Ala Cys Arg Thr
   370 375 380
   Gly Asp Arg
   385
<210> 5
   <211> 221
   <212> PRT
   <213> Homo sapiens
<400> 5
   Met Gly Val His Glu Cys Pro Ala Trp Leu Trp Leu Leu Leu Ser Leu
   1 5 10 15
   Leu Ser Leu Pro Leu Gly Leu Pro Val Leu Gly Ser Ser Ser Ser Lys
   20 25 30
   Ala Pro Pro Pro Ser Leu Pro Ser Pro Ser Arg Leu Pro Gly Pro Ser
   35 40 45
   Asp Thr Pro Ile Leu Pro Gln Ala Pro Pro Arg Leu Ile Cys Asp Ser
   50 55 60
   Arg Val Leu Glu Arg Tyr Leu Leu Glu Ala Lys Glu Ala Glu Asn Ile
   65 70 75 80
   Thr Thr Gly Cys Ala Glu His Cys Ser Leu Asn Glu Asn Ile Thr Val
   85 90 95
   Pro Asp Thr Lys Val Asn Phe Tyr Ala Trp Lys Arg Met Glu Val Gly
   100 105 110
   Gln Gln Ala Val Glu Val Trp Gln Gly Leu Ala Leu Leu Ser Glu Ala
   115 120 125
   Val Leu Arg Gly Gln Ala Leu Leu Val Asn Ser Ser Gln Pro Trp Glu
       130                 135                 140
   Pro Leu Gln Leu His Val Asp Lys Ala Val Ser Gly Leu Arg Ser Leu
   145                 150                 155                 160
   Thr Thr Leu Leu Arg Ala Leu Gly Ala Gln Lys Glu Ala Ile Ser Pro
                   165                 170                 175
   Pro Asp Ala Ala Ser Ala Ala Pro Leu Arg Thr Ile Thr Ala Asp Thr
               180                 185                 190
   Phe Arg Lys Leu Phe Arg Val Tyr Ser Asn Phe Leu Arg Gly Lys Leu
           195                 200                 205
   Lys Leu Tyr Thr Gly Glu Ala Cys Arg Thr Gly Asp Arg
       210                 215                 220
<210> 6
   <211> 249
   <212> **PRT**
   <213> Homo sapiens
<400> 6
   Met Gly Val His Glu Cys Pro Ala Trp Leu Trp Leu Leu Leu Ser Leu
   1               5                   10                  15
   Leu Ser Leu Pro Leu Gly Leu Pro Val Leu Gly Ser Ser Ser Ser Lys
               20                  25                  30
   Ala Pro Pro Pro Ser Leu Pro Ser Pro Ser Arg Leu Pro Gly Pro Ser
           35                  40                  45
   Asp Thr Pro Ile Leu Pro Gln Ala Pro Pro Arg Leu Ile Cys Asp Ser
       50                  55                  60
   Arg Val Leu Glu Arg Tyr Leu Leu Glu Ala Lys Glu Ala Glu Asn Ile
   65                  70                  75                  80
   Thr Thr Gly Cys Ala Glu His Cys Ser Leu Asn Glu Asn Ile Thr Val
                   85                  90                  95
   Pro Asp Thr Lys Val Asn Phe Tyr Ala Trp Lys Arg Met Glu Val Gly
               100                 105                 110
   Gln Gln Ala Val Glu Val Trp Gln Gly Leu Ala Leu Leu Ser Glu Ala
           115                 120                 125
   Val Leu Arg Gly Gln Ala Leu Leu Val Asn Ser Ser Gln Pro Trp Glu
       130                 135                 140
   Pro Leu Gln Leu His Val Asp Lys Ala Val Ser Gly Leu Arg Ser Leu
   145                 150                 155                 160
   Thr Thr Leu Leu Arg Ala Leu Gly Ala Gln Lys Glu Ala Ile Ser Pro
                   165                 170                 175
   Pro Asp Ala Ala Ser Ala Ala Pro Leu Arg Thr Ile Thr Ala Asp Thr
               180                 185                 190
   Phe Arg Lys Leu Phe Arg Val Tyr Ser Asn Phe Leu Arg Gly Lys Leu
           195                 200                 205
   Lys Leu Tyr Thr Gly Glu Ala Cys Arg Thr Gly Asp Arg Ser Ser Ser
       210                 215                 220
   Ser Lys Ala Pro Pro Pro Ser Leu Pro Ser Pro Ser Arg Leu Pro Gly
   225                 230                 235                 240
   Pro Ser Asp Thr Pro Ile Leu Pro Gln
                   245
<210> 7
   <211> 25
   <212> DNA
   <213> Artificial
<220>
   <223> Forward primer for EPO-CTP constructs
<400> 7
   aatctagagg tcatcatggg ggtgc 25
<210> 8
   <211> 32
   <212> DNA
   <213> Artificial
<220>
   <223> Forward primer for EPO-CTP constructs
<400> 8
   attgcggccg cggatccaga agacctttat tg 32
<210> 9
   <211> 25
   <212> DNA
   <213> Artificial
<220>
   <223> Reverse primer for EPO-CTP constructs
<400> 9
   taaatattgg ggtgtccgag ggccc 25
<210> 10
   <211> 32
   <212> DNA
   <213> Artificial
<220>
   <223> Forward primer for EPO-CTP constructs
<400> 10
   ccaatattac cacaagcccc accacgcctc at 32
<210> 11
   <211> 35
   <212> DNA
   <213> Artificial
<220>
   <223> Reverse primer for EPO-CTP constructs
<400> 11
   tgcggccgcg gatccttatc tgtcccctgt cctgc 35
<210> 12
   <211> 17
   <212> DNA
   <213> Artificial
<220>
   <223> Forward primer for EPO-CTP constructs
<400> 12
   gccctgctgt cggaagc 17
<210> 13
   <211> 32
   <212> DNA
   <213> Artificial
<220>
   <223> Reverse primer for EPO-CTP constructs
<400> 13
   attgcggccg cggatccaga agacctttat tg 32
<210> 14
   <211> 32
   <212> DNA
   <213> Artificial
<220>
   <223> Reverse primer for EPO-CTP constructs
<400> 14
   ctttgaggaa gaggagccca ggactgggag gc 32
<210> 15
   <211> 24
   <212> DNA
   <213> Artificial
<220>
   <223> Forward primer for EPO-CTP constructs
<400> 15
   cctgggctcc tcttcctcaa aggc 24
<210> 16
   <211> 193
   <212> PRT
   <213> Homo sapiens
<400> 16
   Met Gly Val His Glu Cys Pro Ala Trp Leu Trp Leu Leu Leu Ser Leu
   1               5                   10                  15
   Leu Ser Leu Pro Leu Gly Leu Pro Val Leu Gly Ala Pro Pro Arg Leu
               20                  25                  30
   Ile Cys Asp Ser Arg Val Leu Glu Arg Tyr Leu Leu Glu Ala Lys Glu
           35                  40                  45
   Ala Glu Asn Ile Thr Thr Gly Cys Ala Glu His Cys Ser Leu Asn Glu
       50                  55                  60
   Asn Ile Thr Val Pro Asp Thr Lys Val Asn Phe Tyr Ala Trp Lys Arg
   65                  70                  75                  80
   Met Glu Val Gly Gln Gln Ala Val Glu Val Trp Gln Gly Leu Ala Leu
                   85                  90                  95
   Leu Ser Glu Ala Val Leu Arg Gly Gln Ala Leu Leu Val Asn Ser Ser
               100                 105                 110
   Gln Pro Trp Glu Pro Leu Gln Leu His Val Asp Lys Ala Val Ser Gly
           115                 120                 125
   Leu Arg Ser Leu Thr Thr Leu Leu Arg Ala Leu Gly Ala Gln Lys Glu
       130                 135                 140
   Ala Ile Ser Pro Pro Asp Ala Ala Ser Ala Ala Pro Leu Arg Thr Ile
   145                 150                 155                 160
   Thr Ala Asp Thr Phe Arg Lys Leu Phe Arg Val Tyr Ser Asn Phe Leu
   165 170 175
   Arg Gly Lys Leu Lys Leu Tyr Thr Gly Glu Ala Cys Arg Thr Gly Asp
   180 185 190
   Arg
<210> 17
   <211> 34
   <212> **PRT**
   <213> Artificial
<220>
   <223> CTP amino acid sequence
<400> 17
   Asp Pro Arg Phe Gln Asp Ser Ser Ser Ser Lys Ala Pro Pro Pro Ser
   1 5 10 15
   Leu Pro Ser Pro Ser Arg Leu Pro Gly Pro Ser Asp Thr Pro Ile Leu
   20 25 30
   Pro Gln
<210> 18
   <211> 28
   <212> PRT
   <213> Artificial
<220>
   <223> CTP amino acid sequence
<400> 18
   Ser Ser Ser Ser Lys Ala Pro Pro Pro Ser Leu Pro Ser Pro Ser Arg
   1 5 10 15
   Leu Pro Gly Pro Ser Asp Thr Pro Ile Leu Pro Gln
   20 25
<210> 19
   <211> 27
   <212> PRT
   <213> Homo sapiens
<400> 19
   Met Gly Val His Glu Cys Pro Ala Trp Leu Trp Leu Leu Leu Ser Leu
   1 5 10 15
   Leu Ser Leu Pro Leu Gly Leu Pro Val Leu Gly
   20 25
<210> 20
   <211> 786
   <212> DNA
   <213> Homo sapiens
<400> 20
   tctagaggtc atcatggggg tgcacgaatg tcctgcctgg ctgtggcttc tcctgtccct 60
   tctgtcgctc cctctgggcc tcccagtcct gggctcctct tcctcaaagg cccctccccc 120
   gagccttcca agtccatccc gactcccggg gccctcggac accccaatat taccacaagc 180
   cccaccacgc ctcatctgtg acagccgagt cctggagagg tacctcttgg aggccaagga 240
   ggccgagaat atcacgacgg gctgtgctga acactgcagc ttgaatgaga atatcactgt 300
   cccagacacc aaagttaatt tctatgcctg gaagaggatg gaggtcgggc agcaggccgt 360
   agaagtctgg cagggcctgg ccctgctgtc ggaagctgtc ctgcggggcc aggccctgtt 420
   ggtcaactct tcccagccgt gggagcccct gcagctgcat gtggataaag ccgtcagtgg 480
   ccttcgcagc ctcaccactc tgcttcgggc tctgggagcc cagaaggaag ccatctcccc 540
   tccagatgcg gcctcagctg ctccactccg aacaatcact gctgacactt tccgcaaact 600
   cttccgagtc tactccaatt tcctccgggg aaagctgaag ctgtacacag gggaggcctg 660
   caggacaggg gacagatcct cttcctcaaa ggcccctccc ccgagccttc caagtccatc 720
   ccgactcccg gggccctcgg acaccccgat cctcccacaa taaaggtctt ctggatccgc 780
   ggccgc 786
<210> 21
   <211> 873
   <212> DNA
   <213> Homo sapiens
<400> 21
   tctagaggtc atcatggggg tgcacgaatg tcctgcctgg ctgtggcttc tcctgtccct        60
   tctgtcgctc cctctgggcc tcccagtcct gggctcctct tcctcaaagg cccctccccc       120
   gagccttcca agtccatccc gactcccggg gccctcggac accccaatat taccacaagc       180
   cccaccacgc ctcatctgtg acagccgagt cctggagagg tacctcttgg aggccaagga       240
   ggccgagaat atcacgacgg gctgtgctga acactgcagc ttgaatgaga atatcactgt       300
   cccagacacc aaagttaatt tctatgcctg gaagaggatg gaggtcgggc agcaggccgt       360
   agaagtctgg cagggcctgg ccctgctgtc ggaagctgtc ctgcggggcc aggccctgtt       420
   ggtcaactct tcccagccgt gggagcccct gcagctgcat gtggataaag ccgtcagtgg       480
   ccttcgcagc ctcaccactc tgcttcgggc tctgggagcc cagaaggaag ccatctcccc       540
   tccagatgcg gcctcagctg ctccactccg aacaatcact gctgacactt tccgcaaact       600
   cttccgagtc tactccaatt tcctccgggg aaagctgaag ctgtacacag gggaggcctg       660
   caggacaggg gacagatcct cttcctcaaa ggcccctccc ccgagccttc caagtccatc       720
   ccgactcccg gggccctccg acacaccaat cctgccacag agcagctcct ctaaggcccc       780
   tcctccatcc ctgccatccc cctcccggct gcctggcccc tctgacaccc ctatcctgcc       840
   tcagtgatga aggtcttctg gatccgcggc cgc                                    873
<210> 22
   <211> 221
   <212> PRT
   <213> Homo sapiens
<400> 22
   Met Gly Val His Glu Cys Pro Ala Trp Leu Trp Leu Leu Leu Ser Leu
   1               5                   10                  15
   Leu Ser Leu Pro Leu Gly Leu Pro Val Leu Gly Ala Pro Pro Arg Leu
               20                  25                  30
   Ile Cys Asp Ser Arg Val Leu Glu Arg Tyr Leu Leu Glu Ala Lys Glu
           35                  40                  45
   Ala Glu Asn Ile Thr Thr Gly Cys Ala Glu His Cys Ser Leu Asn Glu
       50                  55                  60
   Asn Ile Thr Val Pro Asp Thr Lys Val Asn Phe Tyr Ala Trp Lys Arg
   65                  70                  75                  80
   Met Glu Val Gly Gln Gln Ala Val Glu Val Trp Gln Gly Leu Ala Leu
                   85                  90                  95
   Leu Ser Glu Ala Val Leu Arg Ser Gln Ala Leu Leu Val Asn Ser Ser
               100                 105                 110
   Gln Pro Trp Glu Pro Leu Gln Leu His Val Asp Lys Ala Val Ser Gly
           115                 120                 125
   Leu Arg Ser Leu Thr Thr Leu Leu Arg Ala Leu Gly Ala Gln Lys Glu
       130                 135                 140
   Ala Ile Ser Pro Pro Asp Ala Ala Ser Ala Ala Pro Leu Arg Thr Ile
   145                 150                 155                 160
   Thr Ala Asp Thr Phe Arg Lys Leu Phe Arg Val Tyr Ser Asn Phe Leu
                   165                 170                 175
   Arg Gly Lys Leu Lys Leu Tyr Thr Gly Glu Ala Cys Arg Thr Gly Asp
               180                 185                 190
   Arg Ser Ser Ser Ser Lys Ala Pro Pro Pro Ser Leu Pro Ser Pro Ser
           195                 200                 205
   Arg Leu Pro Gly Pro Ser Asp Thr Pro Ile Leu Pro Gln
       210                 215                 220
<210> 23
   <211> 217
   <212> PRT
   <213> Homo sapiens
<400> 23
   Met Ala Thr Gly Ser Arg Thr Ser Leu Leu Leu Ala Phe Gly Leu Leu
   1               5                   10                  15
   Cys Leu Pro Trp Leu Gln Glu Gly Ser Ala Phe Pro Thr Ile Pro Leu
               20                  25                  30
   Ser Arg Leu Phe Asp Asn Ala Met Leu Arg Ala His Arg Leu His Gln
           35                  40                  45
   Leu Ala Phe Asp Thr Tyr Gln Glu Phe Glu Glu Ala Tyr Ile Pro Lys
       50                  55                  60
   Val Gln Lys Tyr Ser Phe Leu Gln Asn Pro Gln Thr Ser Leu Cys Phe
   65                  70                  75                  80
   Ser Glu Ser Ile Pro Thr Pro Ser Asn Arg Glu Glu Thr Gln Gln Lys
                   85                  90                  95
   Ser Asn Leu Glu Leu Leu Arg Ile Ser Leu Leu Leu Ile Gln Ser Trp
               100                 105                 110
   Leu Glu Pro Val Gln Phe Leu Arg Ser Val Phe Ala Asn Ser Leu Val
           115                 120                 125
   Tyr Gly Ala Ser Asp Ser Asn Val Tyr Asp Leu Leu Lys Asp Leu Glu
       130                 135                 140
   Glu Gly Ile Gln Thr Leu Met Gly Arg Leu Glu Asp Gly Ser Pro Arg
   145                 150                 155                 160
   Thr Gly Gln Ile Phe Lys Gln Thr Tyr Ser Lys Phe Asp Thr Asn Ser
                   165                 170                 175
   His Asn Asp Asp Ala Leu Leu Lys Asn Tyr Gly Leu Leu Tyr Cys Phe
               180                 185                 190
   Arg Lys Asp Met Asp Lys Val Glu Thr Phe Leu Arg Ile Val Gln Cys
           195                 200                 205
   Arg Ser Val Glu Gly Ser Cys Gly Phe
       210                 215
<210> 24
   <211> 166
   <212> **PRT**
   <213> Homo sapiens
<400> **24**
   Met Ser Tyr Asn Leu Leu Gly Phe Leu Gln Arg Ser Ser Asn Phe Gln
   1               5                   10                  15
   Ser Gln Lys Leu Leu Trp Gln Leu Asn Gly Arg Leu Glu Tyr Cys Leu
               20                  25                  30
   Lys Asp Arg Met Asn Phe Asp Ile Pro Glu Glu Ile Lys Gln Leu Gln
           35                  40                  45
   Gln Phe Gln Lys Glu Asp Ala Ala Leu Thr Ile Tyr Glu Met Leu Gln
       50                  55                  60
   Asn Ile Phe Ala Ile Phe Arg Gln Asp Ser Ser Ser Thr Gly Trp Asn
   65                  70                  75                  80
   Glu Thr Ile Val Glu Asn Leu Leu Ala Asn Val Tyr His Gln Ile Asn
                   85                  90                  95
   His Leu Lys Thr Val Leu Glu Glu Lys Leu Glu Lys Glu Asp Phe Thr
               100                 105                 110
   Arg Gly Lys Leu Met Ser Ser Leu His Leu Lys Arg Tyr Tyr Gly Arg
           115                 120                 125
   Ile Leu His Tyr Leu Lys Ala Lys Glu Tyr Ser His Cys Ala Trp Thr
       130                 135                 140
   Ile Val Arg Val Glu Ile Leu Arg Asn Phe Tyr Phe Ile Asn Arg Leu
   145                 150                 155                 160
   Thr Gly Tyr Leu Arg Asn
                   165
<210> 25
   <211> 30
   <212> PRT
   <213> Homo sapiens
<400> 25
   His Ala Glu Gly Thr Phe Thr Ser Asp Val Ser Ser Tyr Leu Glu Gly
   1               5                   10                  15
   Gln Ala Ala Lys Glu Phe Ile Ala Trp Leu Val Lys Gly Arg
               20                  25                  30
<210> 26
   <211> 21
   <212> PRT
   <213> Homo sapiens
<400> 26
   Met Thr Asn Lys Cys Leu Leu Gln Ile Ala Leu Leu Leu Cys Phe Ser
   1               5                   10                  15
   Thr Thr Ala Leu Ser
               20
<210> 27
   <211> 19
   <212> DNA
   <213> Artificial
<220>
   <223> XbaI Forward primer for HGH-CTP constructs
<400> 27
   ctctagagga catggccac 19
<210> 28
   <211> 24
   <212> DNA
   <213> Artificial
<220>
   <223> Reverse primer for HGH-CTP constructs
<400> 28
   acagggaggt ctgggggttc tgca 24
<210> 29
   <211> 26
   <212> DNA
   <213> Artificial
<220>
   <223> Forward primer for HGH-CTP constructs
<400> 29
   tgcagaaccc ccagacctcc ctgtgc 26
<210> 30
   <211> 22
   <212> DNA
   <213> Artificial
<220>
   <223> Reverse primer for HGH-CTP constructs
<400> 30
   ccaaactcat caatgtatct ta 22
<210> 31
   <211> 19
   <212> DNA
   <213> Artificial
<220>
   <223> XbaI Forward primer for HGH-CTP constructs
<400> 31
   ctctagagga catggccac 19
<210> 32
   <211> 25
   <212> DNA
   <213> Artificial
<220>
   <223> Reverse primer for HGH-CTP constructs
<400> 32
   cgaactcctg gtaggtgtca aaggc 25
<210> 33
   <211> 25
   <212> DNA
   <213> Artificial
<220>
   <223> Forward primer for HGH-CTP constructs
<400> 33
   gcctttgaca cctaccagga gttcg 25
<210> 34
   <211> 33
   <212> DNA
   <213> Artificial
<220>
   <223> NotI Reverse primer for HGH-CTP constructs
<400> 34
   acgcggccgc atccagacct tcatcactga ggc 33
<210> 35
   <211> 34
   <212> DNA
   <213> Artificial
<220>
   <223> Reverse primer for HGH-CTP constructs
<400> 35
   gcggccgcgg actcatcaga agccgcagct gccc 34
<210> 36
   <211> 217
   <212> PRT
   <213> Homo sapiens
<400> 36
<210> 37
   <211> 245
   <212> PRT
   <213> Homo sapiens
<400> 37
   Met Ala Thr Gly Ser Arg Thr Ser Leu Leu Leu Ala Phe Gly Leu Leu
   1               5                   10                  15
   Cys Leu Pro Trp Leu Gln Glu Gly Ser Ala Phe Pro Thr Ile Pro Leu
               20                  25                  30
   Ser Arg Leu Phe Asp Asn Ala Met Leu Arg Ala His Arg Leu His Gln
           35                  40                  45
   Leu Ala Phe Asp Thr Tyr Gln Glu Phe Glu Glu Ala Tyr Ile Pro Lys
       50                  55                  60
   Glu Gln Lys Tyr Ser Phe Leu Gln Asn Pro Gln Thr Ser Leu Cys Phe
   65                  70                  75                  80
   Ser Glu Ser Ile Pro Thr Pro Ser Asn Arg Glu Glu Thr Gln Gln Lys
                   85                  90                  95
   Ser Asn Leu Glu Leu Leu Arg Ile Ser Leu Leu Leu Ile Gln Ser Trp
               100                 105                 110
   Leu Glu Pro Val Gln Phe Leu Arg Ser Val Phe Ala Asn Ser Leu Val
           115                 120                 125
   Tyr Gly Ala Ser Asp Ser Asn Val Tyr Asp Leu Leu Lys Asp Leu Glu
       130                 135                 140
   Glu Gly Ile Gln Thr Leu Met Gly Arg Leu Glu Asp Gly Ser Pro Arg
   145                 150                 155                 160
   Thr Gly Gln Ile Phe Lys Gln Thr Tyr Ser Lys Phe Asp Thr Asn Ser
                   165                 170                 175
   His Asn Asp Asp Ala Leu Leu Lys Asn Tyr Gly Leu Leu Tyr Cys Phe
               180                 185                 190
   Arg Lys Asp Met Asp Lys Val Glu Thr Phe Leu Arg Ile Val Gln Cys
           195                 200                 205
   Arg Ser Val Glu Gly Ser Cys Gly Phe Ser Ser Ser Ser Lys Ala Pro
       210                 215                 220
   Pro Pro Ser Leu Pro Ser Pro Ser Arg Leu Pro Gly Pro Ser Asp Thr
   225                 230                 235                 240
   Pro Ile Leu Pro Gln
                   245
<210> 38
   <211> 273
   <212> PRT
   <213> Homo sapiens
<400> 38
   Met Ala Thr Gly Ser Arg Thr Ser Leu Leu Leu Ala Phe Gly Leu Leu
   1               5                   10                  15
   Cys Leu Pro Trp Leu Gln Glu Gly Ser Ala Phe Pro Thr Ile Pro Leu
               20                  25                  30
   Ser Arg Leu Phe Asp Asn Ala Met Leu Arg Ala His Arg Leu His Gln
           35                  40                  45
   Leu Ala Phe Asp Thr Tyr Gln Glu Phe Glu Glu Ala Tyr Ile Pro Lys
       50                  55                  60
   Glu Gln Lys Tyr Ser Phe Leu Gln Asn Pro Gln Thr Ser Leu Cys Phe
   65                  70                  75                  80
   Ser Glu Ser Ile Pro Thr Pro Ser Asn Arg Glu Glu Thr Gln Gln Lys
                   85                  90                  95
   Ser Asn Leu Glu Leu Leu Arg Ile Ser Leu Leu Leu Ile Gln Ser Trp
               100                 105                 110
   Leu Glu Pro Val Gln Phe Leu Arg Ser Val Phe Ala Asn Ser Leu Val
           115                 120                 125
   Tyr Gly Ala Ser Asp Ser Asn Val Tyr Asp Leu Leu Lys Asp Leu Glu
       130                 135                 140
   Glu Gly Ile Gln Thr Leu Met Gly Arg Leu Glu Asp Gly Ser Pro Arg
   145                 150                 155                 160
   Thr Gly Gln Ile Phe Lys Gln Thr Tyr Ser Lys Phe Asp Thr Asn Ser
                   165                 170                 175
   His Asn Asp Asp Ala Leu Leu Lys Asn Tyr Gly Leu Leu Tyr Cys Phe
               180                 185                 190
   Arg Lys Asp Met Asp Lys Val Glu Thr Phe Leu Arg Ile Val Gln Cys
           195                 200                 205
   Arg Ser Val Glu Gly Ser Cys Gly Phe Ser Ser Ser Ser Lys Ala Pro
       210                 215                 220
   Pro Pro Ser Leu Pro Ser Pro Ser Arg Leu Pro Gly Pro Ser Asp Thr
   225                 230                 235                 240
   Pro Ile Leu Pro Gln Ser Ser Ser Ser Lys Ala Pro Pro Pro Ser Leu
                   245                 250                 255
   Pro Ser Pro Ser Arg Leu Pro Gly Pro Ser Asp Thr Pro Ile Leu Pro
               260                 265                 270
   Gln
<210> 39
   <211> 301
   <212> PRT
   <213> Homo sapiens
<400> 39
   Met Ala Thr Gly Ser Arg Thr Ser Leu Leu Leu Ala Phe Gly Leu Leu
   1               5                   10                  15
   Cys Leu Pro Trp Leu Gln Glu Gly Ser Ala Ser Ser Ser Ser Lys Ala
               20                  25                  30
   Pro Pro Pro Ser Leu Pro Ser Pro Ser Arg Leu Pro Gly Pro Ser Asp
           35                  40                  45
   Thr Pro Ile Leu Pro Gln Phe Pro Thr Ile Pro Leu Ser Arg Leu Phe
       50                  55                  60
   Asp Asn Ala Met Leu Arg Ala His Arg Leu His Gln Leu Ala Phe Asp
   65                  70                  75                  80
   Thr Tyr Gln Glu Phe Glu Glu Ala Tyr Ile Pro Lys Glu Gln Lys Tyr
                   85                  90                  95
   Ser Phe Leu Gln Asn Pro Gln Thr Ser Leu Cys Phe Ser Glu Ser Ile
               100                 105                 110
   Pro Thr Pro Ser Asn Arg Glu Glu Thr Gln Gln Lys Ser Asn Leu Glu
           115                 120                 125
   Leu Leu Arg Ile Ser Leu Leu Leu Ile Gln Ser Trp Leu Glu Pro Val
       130                 135                 140
   Gln Phe Leu Arg Ser Val Phe Ala Asn Ser Leu Val Tyr Gly Ala Ser
   145                 150                 155                 160
   Asp Ser Asn Val Tyr Asp Leu Leu Lys Asp Leu Glu Glu Gly Ile Gln
                   165                 170                 175
   Thr Leu Met Gly Arg Leu Glu Asp Gly Ser Pro Arg Thr Gly Gln Ile
               180                 185                 190
   Phe Lys Gln Thr Tyr Ser Lys Phe Asp Thr Asn Ser His Asn Asp Asp
           195                 200                 205
   Ala Leu Leu Lys Asn Tyr Gly Leu Leu Tyr Cys Phe Arg Lys Asp Met
       210                 215                 220
   Asp Lys Val Glu Thr Phe Leu Arg Ile Val Gln Cys Arg Ser Val Glu
   225                 230                 235                 240
   Gly Ser Cys Gly Phe Ser Ser Ser Ser Lys Ala Pro Pro Pro Ser Leu
                   245                 250                 255
   Pro Ser Pro Ser Arg Leu Pro Gly Pro Ser Asp Thr Pro Ile Leu Pro
               260                 265                 270
   Gln Ser Ser Ser Ser Lys Ala Pro Pro Pro Ser Leu Pro Ser Pro Ser
           275                 280                 285
   Arg Leu Pro Gly Pro Ser Asp Thr Pro Ile Leu Pro Gln
       290                 295                 300
<210> 40
   <211> 285
   <212> PRT
   <213> Homo sapiens
<400> 40
   Met Ala Thr Gly Ser Arg Thr Ser Leu Leu Leu Ala Phe Gly Leu Leu
   1               5                   10                  15
   Cys Leu Pro Trp Leu Gln Glu Gly Ser Ala Ser Ser Ser Ser Lys Ala
               20                  25                  30
   Pro Pro Pro Ser Leu Pro Phe Pro Thr Ile Pro Leu Ser Arg Leu Phe
           35                  40                  45
   Asp Asn Ala Met Leu Arg Ala His Arg Leu His Gln Leu Ala Phe Asp
       50                  55                  60
   Thr Tyr Gln Glu Phe Glu Glu Ala Tyr Ile Pro Lys Glu Gln Lys Tyr
   65                  70                  75                  80
   Ser Phe Leu Gln Asn Pro Gln Thr Ser Leu Cys Phe Ser Glu Ser Ile
                   85                  90                  95
   Pro Thr Pro Ser Asn Arg Glu Glu Thr Gln Gln Lys Ser Asn Leu Glu
               100                 105                 110
   Leu Leu Arg Ile Ser Leu Leu Leu Ile Gln Ser Trp Leu Glu Pro Val
           115                 120                 125
   Gln Phe Leu Arg Ser Val Phe Ala Asn Ser Leu Val Tyr Gly Ala Ser
       130                 135                 140
   Asp Ser Asn Val Tyr Asp Leu Leu Lys Asp Leu Glu Glu Gly Ile Gln
   145                 150                 155                 160
   Thr Leu Met Gly Arg Leu Glu Asp Gly Ser Pro Arg Thr Gly Gln Ile
                   165                 170                 175
   Phe Lys Gln Thr Tyr Ser Lys Phe Asp Thr Asn Ser His Asn Asp Asp
               180                 185                 190
   Ala Leu Leu Lys Asn Tyr Gly Leu Leu Tyr Cys Phe Arg Lys Asp Met
           195                 200                 205
   Asp Lys Val Glu Thr Phe Leu Arg Ile Val Gln Cys Arg Ser Val Glu
       210                 215                 220
   Gly Ser Cys Gly Phe Ser Ser Ser Ser Lys Ala Pro Pro Pro Ser Leu
   225                 230                 235                 240
   Pro Ser Pro Ser Arg Leu Pro Gly Pro Ser Asp Thr Pro Ile Leu Pro
                   245                 250                 255
   Gln Ser Ser Ser Ser Lys Ala Pro Pro Pro Ser Leu Pro Ser Pro Ser
   260 265 270
   Arg Leu Pro Gly Pro Ser Asp Thr Pro Ile Leu Pro Gln
   275 280 285
<210> 41
   <211> 273
   <212> PRT
   <213> Homo sapiens
<400> 41
   Met Ala Thr Gly Ser Arg Thr Ser Leu Leu Leu Ala Phe Gly Leu Leu
   1 5 10 15
   Cys Leu Pro Trp Leu Gln Glu Gly Ser Ala Ser Ser Ser Ser Lys Ala
   20 25 30
   Pro Pro Pro Ser Leu Pro Ser Pro Ser Arg Leu Pro Gly Pro Ser Asp
   35 40 45
   Thr Pro Ile Leu Pro Gln Phe Pro Thr Ile Pro Leu Ser Arg Leu Phe
   50 55 60
   Asp Asn Ala Met Leu Arg Ala His Arg Leu His Gln Leu Ala Phe Asp
   65 70 75 80
   Thr Tyr Gln Glu Phe Glu Glu Ala Tyr Ile Pro Lys Glu Gln Lys Tyr
   85 90 95
   Ser Phe Leu Gln Asn Pro Gln Thr Ser Leu Cys Phe Ser Glu Ser Ile
   100 105 110
   Pro Thr Pro Ser Asn Arg Glu Glu Thr Gln Gln Lys Ser Asn Leu Glu
   115 120 125
   Leu Leu Arg Ile Ser Leu Leu Leu Ile Gln Ser Trp Leu Glu Pro Val
   130 135 140
   Gln Phe Leu Arg Ser Val Phe Ala Asn Ser Leu Val Tyr Gly Ala Ser
   145 150 155 160
   Asp Ser Asn Val Tyr Asp Leu Leu Lys Asp Leu Glu Glu Gly Ile Gln
   165 170 175
   Thr Leu Met Gly Arg Leu Glu Asp Gly Ser Pro Arg Thr Gly Gln Ile
   180 185 190
   Phe Lys Gln Thr Tyr Ser Lys Phe Asp Thr Asn Ser His Asn Asp Asp
   195 200 205
   Ala Leu Leu Lys Asn Tyr Gly Leu Leu Tyr Cys Phe Arg Lys Asp Met
   210 215 220
   Asp Lys Val Glu Thr Phe Leu Arg Ile Val Gln Cys Arg Ser Val Glu
   225 230 235 240
   Gly Ser Cys Gly Phe Ser Ser Ser Ser Lys Ala Pro Pro Pro Ser Leu
   245 250 255
   Pro Ser Pro Ser Arg Leu Pro Gly Pro Ser Asp Thr Pro Ile Leu Pro
   260 265 270
   Gln
<210> 42
   <211> 245
   <212> PRT
   <213> Homo sapiens
<400> 42
   Met Ala Thr Gly Ser Arg Thr Ser Leu Leu Leu Ala Phe Gly Leu Leu
   1 5 10 15
   Cys Leu Pro Trp Leu Gln Glu Gly Ser Ala Ser Ser Ser Ser Lys Ala
   20 25 30
   Pro Pro Pro Ser Leu Pro Ser Pro Ser Arg Leu Pro Gly Pro Ser Asp
   35 40 45
   Thr Pro Ile Leu Pro Gln Phe Pro Thr Ile Pro Leu Ser Arg Leu Phe
   50 55 60
   Asp Asn Ala Met Leu Arg Ala His Arg Leu His Gln Leu Ala Phe Asp
   65 70 75 80
   Thr Tyr Gln Glu Phe Glu Glu Ala Tyr Ile Pro Lys Glu Gln Lys Tyr
   85 90 95
   Ser Phe Leu Gln Asn Pro Gln Thr Ser Leu Cys Phe Ser Glu Ser Ile
   100 105 110
   Pro Thr Pro Ser Asn Arg Glu Glu Thr Gln Gln Lys Ser Asn Leu Glu
   115 120 125
   Leu Leu Arg Ile Ser Leu Leu Leu Ile Gln Ser Trp Leu Glu Pro Val
       130                 135                 140
   Gln Phe Leu Arg Ser Val Phe Ala Asn Ser Leu Val Tyr Gly Ala Ser
   145                 150                 155                 160
   Asp Ser Asn Val Tyr Asp Leu Leu Lys Asp Leu Glu Glu Gly Ile Gln
                   165                 170                 175
   Thr Leu Met Gly Arg Leu Glu Asp Gly Ser Pro Arg Thr Gly Gln Ile
               180                 185                 190
   Phe Lys Gln Thr Tyr Ser Lys Phe Asp Thr Asn Ser His Asn Asp Asp
           195                 200                 205
   Ala Leu Leu Lys Asn Tyr Gly Leu Leu Tyr Cys Phe Arg Lys Asp Met
       210                 215                 220
   Asp Lys Val Glu Thr Phe Leu Arg Ile Val Gln Cys Arg Ser Val Glu
   225                 230                 235                 240
   Gly Ser Cys Gly Phe
                   245
<210> 43
   <211> 12
   <212> **PRT**
   <213> Artificial
<220>
   <223> CTP amino acid sequence
<400> 43
   Ser Ser Ser Ser Lys Ala Pro Pro Pro Ser Leu Pro
   1               5                   10
<210> 44
   <211> 853
   <212> DNA
   <213> Homo sapiens
<400> 44
   tctagaggac atggccaccg gcagcaggac cagcctgctg ctggccttcg gcctgctgtg      60
   cctgccatgg ctgcaggagg gcagcgccag ctcttcttct aaggctccac ccccatctct     120
   gcccagcccc agcagactgc cgggccccag cgacacaccc attctgcccc agttccccac     180
   catccccctg agcaggctgt tcgacaacgc catgctgagg gctcacaggc tgcaccagct     240
   ggcctttgac acctaccagg agttcgagga agcctacatc cccaaggagc agaagtacag     300
   cttcctgcag aacccccaga cctccctgtg cttcagcgag agcatcccca cccccagcaa     360
   cagagaggag acccagcaga agagcaacct ggagctgctg aggatctccc tgctgctgat     420
   ccagagctgg ctggagcccg tgcagttcct gagaagcgtg ttcgccaaca gcctggtgta     480
   cggcgccagc gacagcaacg tgtacgacct gctgaaggac ctggaggagg gcatccagac     540
   cctgatgggc cggctggagg acggcagccc caggaccggc cagatcttca agcagaccta     600
   cagcaagttc gacaccaaca gccacaacga cgacgccctg ctgaagaact acgggctgct     660
   gtactgcttc agaaaggaca tggacaaggt ggagaccttc ctgaggatcg tgcagtgcag     720
   aagcgtggag ggcagctgcg gcttcagctc cagcagcaag gcccctcccc cgagcctgcc     780
   ctccccaagc aggctgcctg ggccctccga cacaccaatc ctgcctcagt gatgaaggtc     840
   tggatgcggc cgc                                                        853
<210> 45
   <211> 937
   <212> DNA
   <213> Homo sapiens
<400> 45
   tctagaggac atggccaccg gcagcaggac cagcctgctg ctggccttcg gcctgctgtg        60
   cctgccatgg ctgcaggagg gcagcgccag ctcttcttct aaggctccac ccccatctct       120
   gcccagcccc agcagactgc cgggccccag cgacacaccc attctgcccc agttccccac       180
   catccccctg agcaggctgt tcgacaacgc catgctgagg gctcacaggc tgcaccagct       240
   ggcctttgac acctaccagg agttcgagga agcctacatc cccaaggagc agaagtacag       300
   cttcctgcag aacccccaga cctccctgtg cttcagcgag agcatcccca cccccagcaa       360
   cagagaggag acccagcaga agagcaacct ggagctgctg aggatctccc tgctgctgat       420
   ccagagctgg ctggagcccg tgcagttcct gagaagcgtg ttcgccaaca gcctggtgta       480
   cggcgccagc gacagcaacg tgtacgacct gctgaaggac ctggaggagg gcatccagac       540
   cctgatgggc cggctggagg acggcagccc caggaccggc cagatcttca agcagaccta       600
   cagcaagttc gacaccaaca gccacaacga cgacgccctg ctgaagaact acgggctgct       660
   gtactgcttc agaaaggaca tggacaaggt ggagaccttc ctgaggatcg tgcagtgcag       720
   aagcgtggag ggcagctgcg gcttcagctc cagcagcaag gcccctcccc cgagcctgcc       780
   ctccccaagc aggctgcctg ggccctccga cacaccaatc ctgccacaga gcagctcctc       840
   taaggcccct cctccatccc tgccatcccc ctcccggctg cctggcccct ctgacacccc       900
   tatcctgcct cagtgatgaa ggtctggatg cggccgc                                937
<210> 46
   <211> 889
   <212> DNA
   <213> Homo sapiens
<400> 46
   tctagaggac atggccaccg gcagcaggac cagcctgctg ctggccttcg gcctgctgtg      60
   cctgccatgg ctgcaggagg gcagcgccag ctcttcttct aaggctccac ccccgagcct     120
   gcccttcccc accatccccc tgagcaggct gttcgacaac gccatgctga gggctcacag     180
   gctgcaccag ctggcctttg acacctacca ggagttcgag gaagcctaca tccccaagga       240
   gcagaagtac agcttcctgc agaaccccca gacctccctg tgcttcagcg agagcatccc       300
   cacccccagc aacagagagg agacccagca gaagagcaac ctggagctgc tgaggatctc     360
   cctgctgctg atccagagct ggctggagcc cgtgcagttc ctgagaagcg tgttcgccaa     420
   cagcctggtg tacggcgcca gcgacagcaa cgtgtacgac ctgctgaagg acctggagga     480
   gggcatccag accctgatgg gccggctgga ggacggcagc cccaggaccg gccagatctt     540
   caagcagacc tacagcaagt tcgacaccaa cagccacaac gacgacgccc tgctgaagaa     600
   ctacgggctg ctgtactgct tcagaaagga catggacaag gtggagacct tcctgaggat     660
   cgtgcagtgc agaagcgtgg agggcagctg cggcttcagc tccagcagca aggcccctcc     720
   cccgagcctg ccctccccaa gcaggctgcc tgggccctcc gacacaccaa tcctgccaca     780
   gagcagctcc tctaaggccc ctcctccatc cctgccatcc ccctcccggc tgcctggccc     840
   ctctgacacc cctatcctgc ctcagtgatg aaggtctgga tgcggccgc                 889
<210> 47
   <211> 217
   <212> PRT
   <213> Homo sapiens
<400> 47
<210> 48
   <211> 33
   <212> PRT
   <213> Homo sapiens
<400> 48
   Asp Pro Arg Phe Gln Asp Ser Ser Ser Ser Lys Ala Pro Pro Pro Ser
   1 5 10 15
   Leu Pro Ser Pro Ser Arg Leu Pro Gly Pro Ser Asp Thr Pro Ile Leu
   20 25 30
   Gln
<210> 49
   <211> 26
   <212> PRT
   <213> Homo sapiens
<400> 49
   Met Ala Thr Gly Ser Arg Thr Ser Leu Leu Leu Ala Phe Gly Leu Leu
   1 5 10 15
   Cys Leu Pro Trp Leu Gln Glu Gly Ser Ala
               20                  25
<210> 50
   <211> 17
   <212> DNA
   <213> Artificial Sequence
<220>
   <223> Reverse primer for EPO-CTP constructs
<400> 50
   gcttccgaca gcagggc 17

## Description

### FIELD OF INVENTION

The present invention relates to medical uses of CTP-modified human growth hormone polypeptides.

### BACKGROUND OF THE INVENTION

Polypeptides are susceptible to denaturation or enzymatic degradation in the blood, liver or kidney. Accordingly, polypeptides typically have short circulatory half-lives of several hours. Because of their low stability, peptide drugs are usually delivered in a sustained frequency so as to maintain an effective plasma concentration of the active peptide. Moreover, since peptide drugs are usually administered by infusion, frequent injection of peptide drugs causes considerable discomfort to a subject.

Unfavorable pharmacokinetics, such as a short serum half-life, can prevent the pharmaceutical development of many otherwise promising drug candidates. Serum half-life is an empirical characteristic of a molecule, and must be determined experimentally for each new potential drug. For example, with lower molecular weight polypeptide drugs, physiological clearance mechanisms such as renal filtration can make the maintenance of therapeutic levels of a drug unfeasible because of cost or frequency of the required dosing regimen. Conversely, a long serum half-life is undesirable where a drug or its metabolites have toxic side effects.

Thus, there is a need for technologies that will prolong the half-lives of therapeutic polypeptides while maintaining a high pharmacological efficacy thereof. Such desired peptide drugs should also meet the requirements of enhanced serum stability, high activity and a low probability of inducing an undesired immune response when injected into a subject. The present invention addresses this need by providing CTP-modified peptides having prolonged half-lives while maintaining a high pharmacological efficacy, and while having enhanced serum stability, high activity and low probability of inducing undesired immune responses in a subject.

### SUMMARY OF THE INVENTION

The present invention provides a chorionic gonadotropin carboxy terminal peptide (CTP)-modified polypeptide comprising a growth hormone (GH), wherein one CTP is attached to the amino terminus of the growth hormone, and two chorionic gonadotropin CTPs are attached in tandem to the carboxy terminus of the growth hormone, optionally including a signal peptide attached to the amino terminus of the one CTP, for use in maintaining insulin-like growth factor (IGF-1) levels within a normal therapeutic range of ±2 SDS in a human subject for six months, wherein the subject is a growth hormone deficient (GHD) child, and the CTP-modified growth hormone is administered once a week at a dose of 0.66 mg/kg/week.

In an embodiment, at least one CTP is glycosylated. In an embodiment, at least one CTP is truncated. In an embodiment, at least one CTP is attached to the growth hormone via a linker, wherein the linker may be a peptide bond.

In an embodiment, the administration results in an annualized height velocity increase of at least about 14.37 cm, based on an interim analysis after 6 months treatment.

In an embodiment, subsequent doses of the CTP-modified polypeptide may be modified from a starting dose based on IGF-1 levels as measured 4 days after the previous dose. In an embodiment, the subsequent doses of the CTP-modified polypeptide are provided weekly.

In an embodiment, the IGF-1 levels comprise IGF-1 levels of samples tested four days post-administration of the CTP-modified polypeptide.

In an embodiment, the growth hormone is a human growth hormone (hGH).

In an embodiment, the subject is a naive subject.

In an embodiment, the sequence of at least one CTP consists of the amino acid sequence of SEQ ID NO: 17 or SEQ ID NO: 18.

In an embodiment, the amino acid sequence of the signal peptide is set forth in SEQ ID NO: 49.

In an embodiment, the amino acid sequence of the CTP-modified polypeptide comprises the sequence set forth in amino acids 27-301 of SEQ ID NO: 39 or amino acids 27-285 of SEQ ID NO: 40. In an embodiment, the amino acid sequence of the CTP-modified polypeptide comprises the sequence set forth in SEQ ID NO: 39 or SEQ ID NO: 40.

In an embodiment, the CTP-modified polypeptide is encoded by the nucleic acid sequence set forth in SEQ ID NO: 44 or SEQ ID NO: 45.

In an embodiment, the CTP-modified polypeptide is administered in a step-wise dose increase at two-week intervals until a dose of 0.66 mg/kg/week is reached.

Other features and advantages of the present invention will become apparent from the following detailed description examples and figures.

### BRIEF DESCRIPTION OF THE DRAWINGS

The following drawings form part of the present specification and are included to further demonstrate certain aspects of the present disclosure, the inventions of which can be better understood by reference to one or more of these drawings in combination with the detailed description of specific embodiments presented herein.
FIG. 1 is a Western blot illustrating the molecular weight & identity of MOD-4020 (SEQ ID NO: 36), MOD-4021 (SEQ ID NO: 37), MOD-4022 (SEQ ID NO: 38), MOD-4023 (SEQ ID NO: 39) and MOD-4024 (SEQ ID NO: 40). A PAGE SDS gel was blotted and stained using monoclonal anti-hGH antibodies. The photograph indicates that like commercial and wild type hGH, MOD-7020-4 variants are recognized by anti-hGH antibodies.
FIG. 2 is a bar graph illustrating the weight gain of hypophysectomized rats following administration of GH-CTP polypeptides.
FIG. 3 includes two schemes (1) a map of CTP-hGH-CTP-CTP pCI-dhfr Plasmid and (2) structural protein formula of CTP-hGH-CTP-CTP.
FIG. 4 are graphs showing the mean plasma CTP-hGH-CTP-CTP or GH concentrations (pg/ml) following a single i.v. or s.c. dose of CTP-hGH-CTP-CTP or GH in rats (n=3-6 per dose/route).
FIG. 5 are graphs showing the mean incremental weight gain following a single s.c. doses of CTP-hGH-CTP-CTP (0.4, 0.8 and 4 mg/Kg) in hypophysectomized rats in comparison to daily GH injections (0.1 mg/Kg/Day) (n=10 per dose).
FIG. 6 is a graph showing the area Under the Curve following single injection of CTP-hGH-CTP-CTP correlates with Body Weight gain in Rats.
FIG. 7 is a graph showing the incremental weight gain following s.c. doses of CTP-hGH-CTP-CTP (0.4, 0.8 and 4 mg/Kg) 4 days apart in hypophysectomized rats in comparison to daily GH injections (0.1 mg/Kg/Day) (n=10 per dose).
FIG. 8 is a graph showing hGH serum concentration in hypophysectomized rat following SC injection of CTP-hGH-CTP-CTP and commercial hGH. Single dose of CTP-hGH-CTP-CTP 0.6 or 1.8 mg/Kg and Biotropin 0.35 or 1.05 mg/Kg were injected subcutaneously to hypophysectomized rats for determination of PK/PD profile. Serum hGH post injection was measured using specific ELISA kits.
FIG. 9 is a graph showing IGF-1 serum levels in hypophysectomized rats following SC injection of CTP-hGH-CTP-CTP and commercial hGH. Single dose of CTP-hGH-CTP-CTP 0.6 or 1.8 mg/Kg and Biotropin 0.35 or 1.05 mg/Kg were injected subcutaneously to hypophysectomized rats for determination of PK/PD profile. Serum IGF-1 post injection was measured using specific ELISA kits (Roche Diagnostics).
FIG. 10 shows an illustration of the phase II study design.
FIG. 11 shows IGF-1 SDS following 4th weekly dose - All Cohorts.
FIG. 12 shows mean change from baseline in IGF-1 plasma concentrations after subcutaneous administration of MOD-4023 to growth hormone deficient adults (Stage I; post 4^{th} injection).
FIG. 13 shows mean IGF-1 levels (determined on day 4 post dosing) during 4 month extension study (52 patients).
FIG. 14 is schematic representation of phase II clinical study of MOD-4023.
FIG. 15 is a graph showing (A); average MOD-4023 weekly PK profile, and (B); average hGH Daily PK Profile.
FIG. 16 is a graph showing MOD-4023 Pediatric Phase II, 6 months average IGF-1 SDS profile.
FIG. 17 is a graph showing MOD-4023 Pediatric Phase 2, IGF-1 BP-3 profile during the 2nd week at each final dose.
FIG. 18 is a graph showing MOD-4023 Pediatric Phase II-6 months average IGF-1 BP-3 Profile.
FIG. 19 is a graph showing MOD-4023 Pediatric Phase II HV Results 6 months Annualized Height Velocity for all patients completing 6m treatment.
FIG. 20 is a graph showing (A); MOD-4023 Pediatric Phase II Pre-Study HV SDS Results and (B); 6 months HV SDS Results.
FIG. 21 is a graph showing MOD-4023 Pediatric Phase II- PD (IGF-1 SDS) Profile at each final dose.
FIG. 22 is a graph showing ΔHeight SDS.
FIG. 23 is a graph showing MOD-4023 Pediatric Phase II- IGF-1 Change from Baseline at the Final Dose.
FIG. 24 is a table showing non-GMP and GMP batches produced in 10 mM Citrate, 147 mM NaCl at pH 6.
FIG. 25A is a graph showing Clone 2 MOD-4023 RP-HPLC Stability at -20°C.
FIG. 25B is a graph showing Clone 2 MOD-4023 RP-HPLC Stability at 5°C.
FIG. 26 is a graph showing Clone 2 MOD-4023 RP-HPLC Stability at 25°C.
FIG. 27A is a graph showing Clone 28 (Xcellerex) Stability at -20°C (RP-HPLC).
FIG. 27B is a graph showing Clone 28 (Xcellerex) Stability at 5°C (RP-HPLC).
FIG. 28A is a graph showing Clone 28 (Xcellerex) Stability at 25°C (RP-HPLC).
FIG. 28B is a graph showing comparison of Clones 2 and 28 Stability Profiles at 5°C (RP-HPLC).
FIG. 29 is a table showing the transfer of MOD-4023 Manufacturing from Xcellerex (XC) to Rentschler (RB).
FIG. 30A is a graph showing differences in RP-HPLC Stability results between Rentschler (RB) and Xcellerex (XCLX) - Main Peak.
FIG. 30B is a table showing main Peak Stability results of GMP1 (RB).
FIG. 30C is a table showing main Peak Stability results of XCLX (tested at RB).
FIG. 31A is a graph showing differences in RP-HPLC Stability results between Rentschler (RB) and Xcellerex (XCLX) - Peak 3.
FIG. 31B is a table showing peak 3 Stability results of GMP1 (RB).
FIG. 31C is a table showing peak 3 Stability results of XCLX (tested at RB).
FIG. 32A is a graph showing differences in RP-HPLC Stability results between Rentschler (RB) and Xcellerex (XCLX) - Peak 5.
FIG. 32B is a table showing peak 5 stability results of GMP1 (RB).
FIG. 32C is a table showing peak 5 Stability results of XCLX (tested at RB).
FIG. 33A is a graph showing stability results after 3 months at 25°CRP-HPLC, Batch GMP Xcellerex. Peak 7 was not observed at XC material.
FIG. 33B is a graph showing Stability results RP-HPLC, Batch GMP-1 at Rentschler. Peak 7 which was not observed at XC material appears after 2 weeks at 25°C.
FIG. 34 is a graph showing RP-HPLC Stability GMP-1 after 3 months, 25°C. Arrow points to new Peak (7) which was not observed at XC material.
FIG. 35A shows IEF Profile of MOD-4023 Batches. There's a similar band pattern in a pI-value range from 3.5 to 4.2 In one XCLX batch there are less faint isoforms in the high pI boundary. In RB batch there are more faint isoforms in the low pI boundary.
FIG. 35B shows Stability results (IEF) from RB and XCLX (3 months at 25°C). More diffused bands in XCLX sample.
FIG. 36A-D shows Effect of high temperatures on % of peaks (clone 2) (FIG. 36A-mail peak; FIG. 36D). The formation of both peaks (3 and 5) is temperature dependent and accelerates at high temperature.(FIG. 36B and FIG. 36C)
FIG. 37A-D shows Effect of pH on % of Peaks (clone 2) (FIG. 37A-main peak; FIG. 37D). Peak 3: No change in the % of the peak after incubation for up to 5 days at pH=4 and up to 2 h at pH=12 (FIG. 37B). Peak 5: No change in the % of the peak after incubation for up to 6 h at pH=4. However, following 6 h a sharp increase in the peak % was observed. At pH 12 incubation for up to 2 h - the peak disappears (FIG. 37C).
FIG. 38 shows Forced degradation studies at Rentschler (clone 28). Overlay of zooms of native (above) and stressed (below) MOD-4023 drug substance. A stressed sample of MOD-4023 (CTP-hGH-CTP-CTP) drug substance was prepared (65°C for about three days) for analysis of related form 5 in MOD-4023 drug substance as the peak is below the LOQ for the unstressed sample.
FIG. 39 shows pH effect on RP-HPLC related forms. Tested samples: RB - 40 mg/ml, pH=5.9; RB - 10 mg/ml, pH=6.2; XCLX - 40 mg/ml, pH=6.2.

### DETAILED DESCRIPTION OF THE INVENTION

The present invention provides a chorionic gonadotropin carboxy terminal peptide (CTP)-modified polypeptide comprising a growth hormone (GH), wherein one CTP is attached to the amino terminus of the growth hormone, and two chorionic gonadotropin CTPs are attached in tandem to the carboxy terminus of the growth hormone, optionally including a signal peptide attached to the amino terminus of the one CTP, for use in maintaining insulin-like growth factor (IGF-1) levels within a normal therapeutic range of ±2 SDS in a human subject for six months, wherein the subject is a growth hormone deficient (GHD) child, and the CTP-modified growth hormone is administered once a week at a dose of 0.66 mg/kg/week.

Also disclosed herein is a pharmaceutical formulation comprising a buffer, a tonicity agent, and a CTP-modified polypeptide consisting of a growth hormone and one chorionic gonadotropin carboxy terminal peptide (CTP) attached to the amino terminus of said growth hormone, and two chorionic gonadotropin CTPs attached to the carboxy terminus of said growth hormone.

Also disclosed herein is a process for making a pharmaceutical formulation for a once a week administration to a subject having a growth hormone deficiency, the process comprising the steps of:
a. modifying a growth hormone by attaching one chorionic gonadotropin carboxy terminal peptide (CTP) attached to the amino terminus of said growth hormone, and two chorionic gonadotropin CTPs attached to the carboxy terminus of said growth hormone;
b. mixing the modified growth hormone in step a. with said buffer, and said tonicity agent at a pH of 6.2-6.4; and,
c. pre-filling a syringe with said formulation.

Also disclosed herein is a process for filling a syringe with a formulation provided herein comprising the steps of a. formulating a once a week dosage form of said CTP-modified human growth hormone (hGH) having a pre-determined amount of CTP-modified hGH; and, b. filling the syringe with said formulation.

The present application describes long-acting polypeptides and methods of producing and using same. The long-acting polypeptides comprise carboxy terminal peptides (CTP) of human Chorionic Gonadotropin (hCG) which may act as a protectant against degradation of proteins or peptides derived therefrom, may extends circulatory half-lives of proteins or peptides derived therefrom, and may enhance the potency of proteins or peptides derived therefrom.

The terms "CTP peptide," "carboxy terminal peptide," "CTP sequence," and "chorionic gonadotropin C-terminal peptide" are used interchangeably herein. In another embodiment, the carboxy terminal peptide is a full-length CTP. In another embodiment, the carboxy terminal peptide is a truncated CTP.

The terms "signal sequence" and "signal peptide" are used interchangeably herein. In another embodiment, "sequence" when in reference to a polynucleotide can refer to a coding portion.

The terms "peptide of interest" and "polypeptide sequence of interest" are used interchangeably herein. In another embodiment, the peptide of interest is a full-length protein. In another embodiment, the peptide of interest is a protein fragment.

In another embodiment, the invention utilizes a pharmaceutical formulation comprising the CTP-modified polypeptide. In another embodiment, the pharmaceutical formulation further comprises a buffer and a tonicity agent. In another embodiment, the buffer is 10mM citrate and the tonicity agent is 147 mM NaCl. In one embodiment, the formulation is at about a pH of 6.0. In another embodiment, the formulation is at about a pH of 6.2. In another embodiment, the formulation is at about a pH of 6.4. In another embodiment, the formulation is at about a pH range of 6.0-6.4. In one embodiment, the buffer is 10mM citrate, the tonicity agent is 147 mM NaCl, and the pH is 6.0. In another embodiment, the formulation is a liquid formulation.

Also disclosed herein is a once weekly dosage form comprising the pharmaceutical formulation provided herein.

In another embodiment, a growth hormone comprising one CTP attached to its amino terminus and two CTPs attached to its carboxy terminus has enhanced *in vivo* biological activity compared the same growth hormone without CTPs.

In one embodiment, the subject is male. In another embodiment, the subject is female. In this invention, the subject is a pre-pubertal growth hormone deficient (GHD) human child. As demonstrated herein, various doses of MOD-4023 (CTP-hGH-CTP-CTP) provided a good catch-up growth response in pre-pubescent children (see Example 10).

The phrase "polypeptide sequence of interest" refers to a polypeptide sequence comprising a biological activity which in this invention is a growth hormone (GH). In another embodiment, the polypeptide is a human growth hormone (hGH).

In one embodiment, the configuration of CTP- growth hormone-CTP-CTP as described herein comprises a growth hormone or an active fragment thereof connected via a linker to at least one of the CTP units. In one embodiment, the linker is a peptide bond. In another embodiment, the configuration of CTP- growth hormone-CTP-CTP as described herein comprises a growth hormone or an active fragment thereof connected via a peptide bond to at least one CTP unit. In another embodiment, a CTP- growth hormone -CTP-CTP as described herein comprises a growth hormone or an active fragment thereof connected via a peptide bond to at least one CTP unit which is connected to an additional CTP unit via a peptide bond. In another embodiment, one nucleic acid molecule encodes a polypeptide as described herein comprising a growth hormone and/or fragments thereof and CTP units and/or fragments thereof.

In another embodiment, the linker which connects the CTP sequence to the polypeptide sequence of interest is a covalent bond. In another embodiment, the linker which connects the CTP sequence to the polypeptide sequence of interest is a peptide bond. In another embodiment, the linker which connects the CTP sequence to the polypeptide sequence of interest is a substituted peptide bond.

In another embodiment, the carboxy-terminal peptide (CTP) sequence comprises an amino acid sequence as set forth in SEQ ID NO: 48. SEQ ID NO: 48 comprises the following amino acid (AA) sequence: DPRFQDSSSSKAPPPSLPSPSRLPGPSDTPILQ (SEQ ID NO: 48).

In another embodiment, the carboxy terminal peptide (CTP) of human Chorionic Gonadotropin (hCG) is fused to a growth hormone protein or peptide. In one embodiment, the protein or peptide thereof is a human growth hormone.

As disclosed herein, in some embodiments, a CTP sequence at both the amino terminal end of a polypeptide and at the carboxy terminal end of the polypeptide provide enhanced protection against degradation of a protein. In some embodiments, CTP sequences at both the amino terminal end of a polypeptide and at the carboxy terminal end of the polypeptide provide an extended half-life to the attached protein.

As disclosed herein, in some embodiments, a CTP sequence at the amino terminal end of a polypeptide, a CTP sequence at the carboxy terminal end of the polypeptide, and at least one additional CTP sequence attached in tandem to the CTP sequence at the carboxy terminus provide enhanced protection against degradation of a protein. In some embodiments, a CTP sequence at the amino terminal end of a polypeptide, a CTP sequence at the carboxy terminal end of the polypeptide, and at least one additional CTP sequence attached in tandem to the CTP sequence at the carboxy terminus provide an extended half-life to the attached protein. In some embodiments, a CTP sequence at the amino terminal end of a polypeptide, a CTP sequence at the carboxy terminal end of the polypeptide, and at least one additional CTP sequence attached in tandem to the CTP sequence at the carboxy terminus provide enhanced activity of the attached protein.

As disclosed herein, in some embodiments, a CTP sequence at the amino terminal end of a polypeptide, a CTP sequence at the carboxy terminal end of the polypeptide, and at least one additional CTP sequence attached in tandem to the CTP sequence at the amino terminus provide enhanced protection against degradation of the attached protein. In some embodiments, a CTP sequence at the amino terminal end of a polypeptide, a CTP sequence at the carboxy terminal end of the polypeptide, and at least one additional CTP sequence attached in tandem to the CTP sequence at the amino terminus provide an extended half-life to the attached protein. In some embodiments, a CTP sequence at the amino terminal end of a polypeptide, a CTP sequence at the carboxy terminal end of the polypeptide, and at least one additional CTP sequence attached in tandem to the CTP sequence at the amino terminus provide enhanced activity the attached protein.

As disclosed herein, in some embodiments, CTP sequences at both the amino terminal end of a growth hormone and at the carboxy terminal end of the growth hormone provide enhanced protection against degradation of a growth hormone. In another embodiment, at least one CTP sequence at the amino terminal end of a growth hormone and two CTP units in the carboxy terminal end of a growth hormone provide enhanced protection against clearance. In another embodiment, at least one CTP sequence at the amino terminal end of a growth hormone and two CTP units in the carboxy terminal end of a growth hormone provide prolonged clearance time. In another embodiment, at least one CTP sequence at the amino terminal end of a growth hormone and two CTP units in the carboxy terminal end of a growth hormone enhance Cₘₐₓ of a growth hormone. In another embodiment, at least one CTP sequence at the amino terminal end of a growth hormone and two CTP units in the carboxy terminal end of a growth hormone enhance Tₘₐₓ of a growth hormone. In another embodiment, at least one CTP sequence at the amino terminal end of a growth hormone and two CTP units in the carboxy terminal end of a growth hormone enhanced T1/2.

As disclosed herein, in some embodiments, CTP sequences at both the amino terminal end of a growth hormone and at the carboxy terminal end of the growth hormone extend the half-life of the modified growth hormone. In another embodiment, at least a single CTP sequence at the amino terminal end of a growth hormone and at least two CTP sequences at the carboxy terminal end of the growth hormone provide an extended half-life to the modified growth hormone. In another embodiment, a single CTP sequence at the amino terminal end of a growth hormone and two CTP sequences at the carboxy terminal end of the growth hormone provide extended half-life to the attached growth hormone. As utilized in this invention, a single CTP sequence at the amino terminal end of a growth hormone and two CTP sequences in tandem at the carboxy terminal end of the growth hormone provide extended half-life to the modified growth hormone.

As disclosed herein, in some embodiments, a CTP sequence at the amino terminal end of a polypeptide, a CTP sequence at the carboxy terminal end of the growth hormone, and at least one additional CTP sequence attached in tandem to the CTP sequence at the carboxy terminus provide enhanced protection against degradation to a growth hormone. In some embodiments, a CTP sequence at the amino terminal end of a growth hormone, a CTP sequence at the carboxy terminal end of the growth hormone, and at least one additional CTP sequence attached in tandem to the CTP sequence at the carboxy terminus extend the half-life of the growth hormone. In some embodiments, a CTP sequence at the amino terminal end of a growth hormone, a CTP sequence at the carboxy terminal end of the growth hormone, and at least one additional CTP sequence attached in tandem to the CTP sequence at the carboxy terminus enhance the biological activity of the growth hormone.

In one embodiment, the sequence of at least one CTP consists of an amino acid sequence selected from the group consisting of: SEQ ID NO: 17 and SEQ ID NO: 18. In another embodiment, the carboxy terminal peptide (CTP) peptide comprises the amino acid sequence from amino acid 112 to position 145 of human chorionic gonadotropin, as set forth in SEQ ID NO: 17. In another embodiment, the CTP sequence comprises the amino acid sequence from amino acid 118 to position 145 of human chorionic gonadotropin, as set forth in SEQ ID NO: 18. In another embodiment, the CTP sequence also commences from any position between positions 112-118 and terminates at position 145 of human chorionic gonadotropin. In some embodiments, the CTP sequence peptide is 28, 29, 30, 31, 32, 33 or 34 amino acids long and commences at position 112, 113, 114, 115, 116, 117 or 118 of the CTP amino acid sequence.

In another embodiment, the CTP peptide is a variant of chorionic gonadotropin CTP which differs from the native CTP by 1-5 conservative amino acid substitutions as described in U.S. Pat. No. 5,712,122. In another embodiment, the CTP peptide is a variant of chorionic gonadotropin CTP which differs from the native CTP by 1 conservative amino acid substitution. In another embodiment, the CTP peptide is a variant of chorionic gonadotropin CTP which differs from the native CTP by 2 conservative amino acid substitutions. In another embodiment, the CTP peptide is a variant of chorionic gonadotropin CTP which differs from the native CTP by 3 conservative amino acid substitutions. In another embodiment, the CTP peptide is a variant of chorionic gonadotropin CTP which differs from the native CTP by 4 conservative amino acid substitutions. In another embodiment, the CTP peptide is a variant of chorionic gonadotropin CTP which differs from the native CTP by 5 conservative amino acid substitutions. In another embodiment, the CTP peptide amino acid sequence is at least 70% homologous to the native CTP amino acid sequence or a peptide thereof. In another embodiment, the CTP peptide amino acid sequence is at least 80% homologous to the native CTP amino acid sequence or a peptide thereof. In another embodiment, the CTP peptide amino acid sequence is at least 90% homologous to the native CTP amino acid sequence or a peptide thereof. In another embodiment, the CTP peptide amino acid sequence is at least 95% homologous to the native CTP amino acid sequence or a peptide thereof.

In another embodiment, the CTP peptide DNA sequence is at least 70% homologous to the native CTP DNA sequence. In another embodiment, the CTP peptide DNA sequence is at least 80% homologous to the native CTP DNA sequence. In another embodiment, the CTP peptide DNA sequence is at least 90% homologous to the native CTP DNA sequence. In another embodiment, the CTP peptide DNA sequence is at least 95% homologous to the native CTP DNA sequence.

In one embodiment, at least one of the chorionic gonadotropin CTP amino acid sequences is truncated. In another embodiment, 2 of the chorionic gonadotropin CTP amino acid sequences are truncated. In another embodiment, all of the chorionic gonadotropin CTP amino acid sequences are truncated. In one embodiment, the truncated CTP comprises the first 10 amino acids of SEQ ID NO: 43. In one embodiment, the truncated CTP comprises the first 11 amino acids of SEQ ID NO: 43. In one embodiment, the truncated CTP comprises the first 12 amino acids of SEQ ID NO: 43. In one embodiment, the truncated CTP comprises the first 13 amino acids of SEQ ID NO: 43. In one embodiment, the truncated CTP comprises the first 14 amino acids of SEQ ID NO: 43. In one embodiment, the truncated CTP comprises the first 15 amino acids of SEQ ID NO: 43. In one embodiment, the truncated CTP comprises the first 16 amino acids of SEQ ID NO: 43. In one embodiment, the truncated CTP comprises the last 14 amino acids of SEQ ID NO: 43.

In one embodiment, at least one of the chorionic gonadotropin CTP amino acid sequences is glycosylated. In another embodiment, 2 of the chorionic gonadotropin CTP amino acid sequences are glycosylated. In another embodiment, all of the chorionic gonadotropin CTP amino acid sequences are glycosylated. In one embodiment, the CTP sequence comprises at least one glycosylation site. In one embodiment, the CTP sequence comprises 2 glycosylation sites. In one embodiment, the CTP sequence comprises 3 glycosylation sites. In one embodiment, the CTP sequence comprises 4 glycosylation sites.

In some embodiments, "homology" as used herein also encompasses deletions, insertions, or substitution variants, including an amino acid substitution thereof, and biologically active polypeptide fragments thereof.

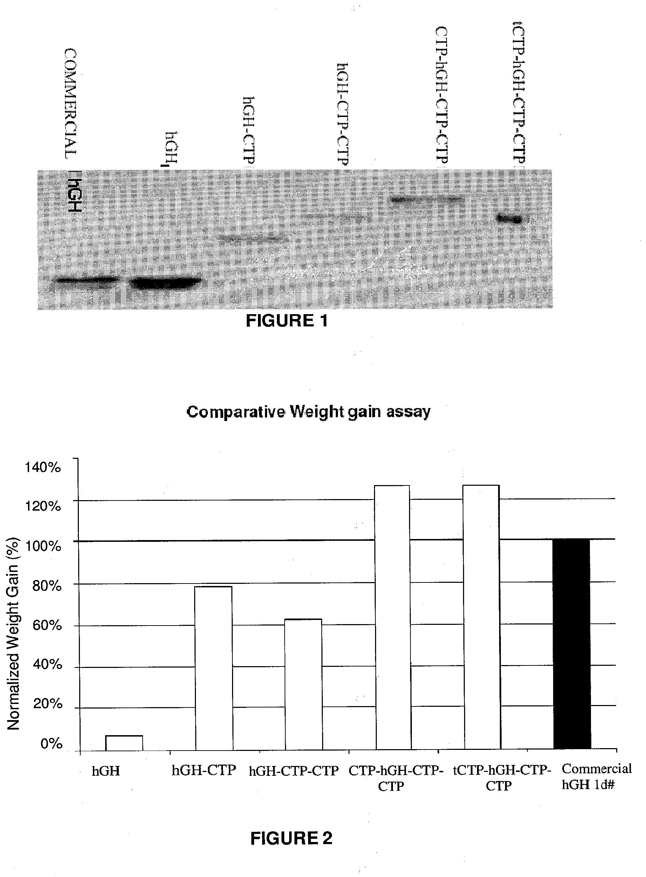

In some embodiments, human growth hormone (hGH) is utilized according to the teachings of the present invention. In some embodiments, the attachment of CTP sequence to both the amino and carboxy termini of the hGH protein results in increased potency (Figures 2, 3, 5, 7 and 9). In some embodiments, the attachment of CTP sequence to both the amino and carboxy termini of the hGH protein results in prolonged in-vivo activity. In one embodiment, CTP-hGH polypeptides utilized in the present invention are set forth in SEQ ID NO: 39 and 40.

As provided herein, growth gain was demonstrated in hypophysectomized rats (which have no growth hormone secretion) following injections of CTP-hGH. As further provided herein, growth grain with excellent correlation to the patients' catch up growth was demonstrated in pre-pubertal growth hormone deficient children (see Example 10, herein).

Also disclosed herein is a method of achieving normal growth recovery of a pre-pubertal growth hormone deficient child, the method comprising administering a pharmaceutical composition comprising a CTP-modified growth hormone provided herein. Also disclosed herein is a method of achieving growth recovery of a pre-pubertal growth hormone deficient child, the method comprising administering a pharmaceutical composition comprising a CTP-modified growth hormone provided herein.

In one embodiment, the phrase "human growth hormone" (hGH) refers to a polypeptide, such as set forth in Genbank Accession No. P01241 (SEQ ID NO: 47), exhibiting hGH activity (i.e. stimulation of growth).

In another embodiment, "human growth hormone" (hGH) refers to a polypeptide, such as set forth in Genbank Accession No. P01241, exhibiting hGH activity (i.e. stimulation of growth). In another embodiment, "GH" also refers to homologues. In another embodiment, a GH amino acid sequence for use in the present invention is at least 50% homologous to a GH sequence set forth herein as determined using BlastP software of the National Center of Biotechnology Information (NCBI) using default parameters. In another embodiment, the percent homology is 60%. In another embodiment, the percent homology is 70%. In another embodiment, the percent homology is 80%. In another embodiment, the percent homology is 90%. In another embodiment, the percent homology is at least 95%. In another embodiment, the percent homology is greater than 95%.

Exemplary CTP-GH polypeptides and CTP-hGH polypeptides utilized in the present invention are set forth in SEQ ID NO: 39 and SEQ ID NO: 40.

As disclosed herein, a GH peptide having additionally one CTP amino acid peptide on the N-terminus and two CTP amino acid peptides on the C-terminus may be for use in for stimulating muscle growth.

Also disclosed herein is a nucleic acid sequence encoding a GH peptide having additionally one CTP amino acid peptide on the N-terminus and two CTP amino acid peptides on the C-terminus for stimulating muscle growth. In another embodiment, the methods of the present disclosure provide a nucleic acid of SEQ ID NO: 45 encoding a GH peptide comprising one CTP amino acid peptide on the N-terminus and two CTP amino acid peptides on the C-terminus for stimulating muscle growth.

In one embodiment, administering to said subject a therapeutically effective amount of a polypeptide consisting of a growth hormone, one chorionic gonadotropin carboxy terminal peptide (CTP) attached to the amino terminus of said growth hormone, and two chorionic gonadotropin CTPs attached to the carboxy terminus of said growth hormone, and wherein said polypeptide optionally consists of a signal peptide attached to the amino terminus of said one CTP, thereby reduces the dosing frequency of the growth hormone in the subject.

In another embodiment, administering to said subject a therapeutically effective amount of a polypeptide consisting of a growth hormone, one chorionic gonadotropin carboxy terminal peptide (CTP) attached to the amino terminus of said growth hormone, and two chorionic gonadotropin CTPs attached to the carboxy terminus of said growth hormone, and wherein said polypeptide optionally consists of a signal peptide attached to the amino terminus of said one CTP, thereby improves the area under the curve (AUC) of the growth hormone in the subject.

As disclosed herein a formulation comprising a polypeptide consisting of a growth hormone, one chorionic gonadotropin carboxy terminal peptide (CTP) attached to the amino terminus of said growth hormone, and two chorionic gonadotropin CTPs attached to the carboxy terminus of said growth hormone, and wherein said polypeptide optionally consists of a signal peptide attached to the amino terminus of said one CTP, has increased stability. In one embodiment, the formulation is stable for at least one year. In another embodiment, the formulation is stable for at least two years.

In one embodiment, the present disclosure provides a method of treating a subject in need of GH therapy, comprising administering to said subject a therapeutically effective amount of a polypeptide consisting of a growth hormone, one chorionic gonadotropin carboxy terminal peptide (CTP) attached to the amino terminus of said growth hormone, and two chorionic gonadotropin CTPs attached to the carboxy terminus of said growth hormone, and wherein said polypeptide optionally consists of a signal peptide attached to the amino terminus of said one CTP, thereby reducing the dosing frequency of a growth hormone in a subject.

In another embodiment, administering to said subject a therapeutically effective amount of a polypeptide consisting of a growth hormone, one chorionic gonadotropin carboxy terminal peptide (CTP) attached to the amino terminus of said growth hormone, and two chorionic gonadotropin CTPs attached to the carboxy terminus of said growth hormone, and wherein said polypeptide optionally consists of a signal peptide attached to the amino terminus of said one CTP, thereby increases insulin-like growth factor (IGF-1) levels in the subject.

In another embodiment, administering to said subject a therapeutically effective amount of a polypeptide consisting of a growth hormone, one chorionic gonadotropin carboxy terminal peptide (CTP) attached to the amino terminus of said growth hormone, and two chorionic gonadotropin CTPs attached to the carboxy terminus of said growth hormone, and wherein said polypeptide optionally consists of a signal peptide attached to the amino terminus of said one CTP, thereby maintains insulin-like growth factor (IGF-1) levels in the subject. In another embodiment, the IGF-1 levels are kept in a defined range, as further provided herein.

In another embodiment, administering to said subject a therapeutically effective amount of a polypeptide consisting of a growth hormone, one chorionic gonadotropin carboxy terminal peptide (CTP) attached to the amino terminus of said growth hormone, and two chorionic gonadotropin CTPs attached to the carboxy terminus of said growth hormone, and wherein said polypeptide optionally consists of a signal peptide attached to the amino terminus of said one CTP, thereby increases and maintains insulin-like growth factor (IGF-1) levels within a defined range in the subject.

In another embodiment, the defined range is a therapeutic dose range achieved by administering a CTP-modified growth hormone provided herein. In another embodiment, the defined range is one in which the Cmax and Ctrough of the sinusoidal behavior of IGF-1 are maintained following consecutive administrations of a CTP-modified growth hormone as further provided herein (see Example 9). In another embodiment, the defined range is a therapeutic dose range for consecutively administering a CTP-modified growth hormone provided herein with excellent responsiveness in a subject and with minimal need for dose modification. In another embodiment, the defined range is comparable to the range of IGF-1 levels in individuals that are considered to be normal. In another embodiment, the defined range is the normal range of IGF-1 levels/values in normal individuals. In this invention, , the defined range is within the normal range when IGF-1 SDS values are within ±2 SDS.

It is disclosed that the CTP-modified GH peptides described herein may be for use in stimulating bone growth.

It is also disclosed that a nucleic acid sequence encoding the CTP-modified GH peptides described herein may be for use in stimulating bone growth.

In another embodiment, conjugated growth hormones of this invention are used in the same manner as unmodified growth hormones. In another embodiment, conjugated growth hormones of this invention have an increased circulating half-life and plasma residence time, decreased clearance, and increased clinical activity *in vivo.* In another embodiment, due to the improved properties of the conjugated growth hormones as described herein, these conjugates are administered less frequently than unmodified growth hormones. In another embodiment, conjugated growth hormones as described herein are administered once a week. In another embodiment, decreased frequency of administration will result in improved patient compliance leading to improved treatment outcomes, as well as improved patient quality of life. In another embodiment, compared to conventional conjugates of growth hormones linked to poly(ethylene glycol) it has been found that growth hormone CTP conjugates having the molecular weight and linker structure of the conjugates of this invention have an improved potency, improved stability, elevated AUC levels, enhanced circulating half-life. In another embodiment, compared to conventional conjugates of growth hormones linked to poly(ethylene glycol) it has been found that growth hormones having the molecular weight and linker structure of the conjugates of this invention have an improved potency, improved stability, elevated AUC levels, enhanced circulating half-life. In another embodiment, a therapeutically effective amount of a conjugated growth hormone is the amount of conjugate necessary for the *in vivo* measurable expected biological activity. In another embodiment, a growth hormone utilized according to the teachings of the present invention exhibits increased potency. In some embodiments, the attachment of CTP sequence to both the amino and carboxy termini of a growth hormone results in prolonged in-vivo activity.

In another embodiment, a therapeutically effective amount of a conjugated growth hormone is determined according to factors as the exact type of condition being treated, the condition of the patient being treated, as well as the other ingredients in the composition. In this invention, a therapeutically effective amount of a conjugated growth hormone is 0.66 mg per kg body weight administered once a week. In another embodiment, a pharmaceutical composition comprising a conjugated growth hormone is formulated at strength effective for administration by various means to a human patient.

In another embodiment, the growth hormone is any growth hormone known to one of skill in the art. In another embodiment, the growth hormone is a human growth hormone. In another embodiment, the growth hormone is a non-human growth hormone. In another embodiment, the nucleotide sequence and/or the amino acid sequence of a growth hormone is available in a gene bank database. In another embodiment, the growth hormone is a homologue. In another embodiment, a homologue also refers to a deletion, insertion, or substitution variant, including an amino acid substitution, thereof and biologically active polypeptide fragments thereof.

In another embodiment, the growth hormone is variant of hGH missing exons 2, 3, 4, or any combination thereof. In another embodiment, the growth hormone comprises a signal peptide. In another embodiment, the growth hormone comprises a signal cleavage site. In another embodiment, polypeptides comprising GH modified by CTPs for use in the present invention comprise recombinant GH.

It is disclosed that the CTP-modified GH peptide utilized in the present invention act by maintaining muscle quality .

It is also disclosed that the CTP-modified GH utilized in the present invention may maintain bone quality.

It is also disclosed that the CTP-modified GH peptides described herein may be for use in \treating a wasting disease.

It is also disclosed that the CTP-modified GH peptides described herein may be for use in increasing cardiac function.

It is also disclosed that the CTP-modified GH peptides described herein may be for use in increasing lipolysis.

It is also disclosed that the CTP-modified GH peptides described herein may be for use in improving fluid balance.

In another embodiment, a growth hormone of the invention comprises the gene bank amino acid deposited sequence under accession no. AAA72260. In another embodiment, a growth hormone of the invention comprises the gene bank amino acid deposited sequence under accession no. AAK69708. In another embodiment, a growth hormone of the invention comprises the gene bank amino acid deposited sequence under accession no. CAA01435. In another embodiment, a growth hormone of the invention comprises the gene bank amino acid deposited sequence under accession no. CAA01329. In another embodiment, a growth hormone of the invention comprises the gene bank amino acid deposited sequence under accession no. CAA00380. In another embodiment, a growth hormone of the invention comprises the gene bank amino acid deposited sequence under accession no. AAA72555. In another embodiment, a growth hormone of the invention comprises the gene bank amino acid deposited sequence under accession no. NP_000506.2. In another embodiment, a growth hormone of the invention comprises the gene bank amino acid deposited sequence under accession no. NP_072053.1. In another embodiment, a growth hormone of the invention comprises the gene bank amino acid deposited sequence under accession no. NP_072054.1. In another embodiment, a growth hormone of the invention comprises the gene bank amino acid deposited sequence under accession no. NP_072055.1. In another embodiment, a growth hormone of the invention comprises the gene bank amino acid deposited sequence under accession no. NP_072056.1.

It is also disclosed that the CTP-modified GH peptide utilized in the present invention may improve exercise capacity.

It is also disclosed that a GH peptide having additionally one CTP amino acid peptide on the N-terminus and two CTP amino acid peptides on the C-terminus may improve lung function.

It is also disclosed that a GH peptide having additionally one CTP amino acid peptide on the N-terminus and two CTP amino acid peptides on the C-terminus may improve..

It is also disclosed that a GH peptide having additionally one CTP amino acid peptide on the N-terminus and two CTP amino acid peptides on the C-terminus may be for regrowing vital organs.

It is also disclosed that a GH peptide as utilized in the present invention may increase sense of well-being.

It is also disclosed that a GH peptide having additionally one CTP amino acid peptide on the N-terminus and two CTP amino acid peptides on the C-terminus may restore REM sleep.

It is also disclosed that a nucleic acid sequence encoding polypeptide comprising hGH modified by CTPs may be for stimulating muscle growth, increasing cardiac function, stimulating bone growth, maintaining muscle integrity, balancing muscle metabolism, inducing muscle buildup, inducing de-novo muscle build-up, enhancing bone load, treating symptoms associated with osteoporosis, treating a wasting disease, increasing lipolysis, improving fluid balance, treating osteoporosis, improving lung function, improving immunity, regrowing a vital organ, increasing sense of well-being, restoring REM sleep, or any combination thereof.

In some embodiments, "homology" also encompasses deletions, insertions, or substitution variants, including an amino acid substitution, thereof and biologically active polypeptide fragments thereof. In one embodiment the substitution variant is one in which the glutamine in position 65 of hGH is substituted by a valine [Gellerfors et al., J Pharm Biomed Anal 1989, 7:173-83].

In another embodiment, the nucleic acid molecule encoding a growth hormone as described herein encodes any amino acid sequence of a growth hormone known to one of skill in the art. In another embodiment, the nucleic acid molecule encoding a growth hormone as described herein encodes an hGH. In another embodiment, the nucleic acid molecule encoding a growth hormone comprises the gene bank nucleic acid deposited sequence under accession no. NM_000515.3. In another embodiment, the nucleic acid molecule encoding a growth hormone comprises the gene bank nucleic acid deposited sequence under accession no. NM_022559.2. In another embodiment, the nucleic acid molecule encoding a growth hormone comprises the gene bank nucleic acid deposited sequence under accession no. NM_022560.2. In another embodiment, the nucleic acid molecule encoding a growth hormone comprises the gene bank nucleic acid deposited sequence under accession no. NM_022561.2. In another embodiment, the nucleic acid molecule encoding a growth hormone comprises the gene bank nucleic acid deposited sequence under accession no. NM_022562.2.

In one embodiment, the homologue also refers to a deletion, insertion, or substitution variant, including an amino acid substitution, thereof and biologically active polypeptide fragments thereof.

In another embodiment the polypeptide sequence of interest is an hGH. In another embodiment the polypeptide sequence of interest is a peptide or a protein including any modified form.

It is disclosed that hGH having additionally at least one CTP amino acid peptide on the N-terminus and at least one CTP amino acid peptide on the C-terminus may be for the treatment of wasting disease, AIDS, cachexia, or hGH deficiency.

In some embodiments, the CTP sequences modification is advantageous in permitting lower dosages to be used.

In some embodiments, "polypeptide" as used herein encompasses native polypeptides (either degradation products, synthetically synthesized polypeptides or recombinant polypeptides) and peptidomimetics (typically, synthetic polypeptides), as well as peptoids and semipeptoids which are polypeptide analogs, which have, in some embodiments, modifications rendering the polypeptides even more stable while in a body or more capable of penetrating into cells.

In some embodiments, modifications include, but are not limited to N terminus modification, C terminus modification, polypeptide bond modification, including, but not limited to, CH2-NH, CH2-S, CH2-S=O, O=C-NH, CH2-O, CH2-CH2, S=C-NH, CH=CH or CF=CH, backbone modifications, and residue modification. Methods for preparing peptidomimetic compounds are well known in the art and are specified, for example, in Quantitative Drug Design, C.A. Ramsden Gd., Chapter 17.2, F. Choplin Pergamon Press (1992). Further details in this respect are provided hereinbelow.

In some embodiments, polypeptide bonds (-CO-NH-) within the polypeptide are substituted. In some embodiments, the polypeptide bonds are substituted by N-methylated bonds (-N(CH3)-CO-). In some embodiments, the polypeptide bonds are substituted by ester bonds (-C(R)H-C-O-O-C(R)-N-). In some embodiments, the polypeptide bonds are substituted by ketomethylen bonds (-CO-CH2-). In some embodiments, the polypeptide bonds are substituted by α-aza bonds (-NH-N(R)-CO-), wherein R is any alkyl, e.g., methyl, carba bonds (-CH2-NH-). In some embodiments, the polypeptide bonds are substituted by hydroxyethylene bonds (-CH(OH)-CH2-). In some embodiments, the polypeptide bonds are substituted by thioamide bonds (-CS-NH-). In some embodiments, the polypeptide bonds are substituted by olefinic double bonds (-CH=CH-). In some embodiments, the polypeptide bonds are substituted by retro amide bonds (-NH-CO-). In some embodiments, the polypeptide bonds are substituted by polypeptide derivatives (-N(R)-CH2-CO-), wherein R is the "normal" side chain, naturally presented on the carbon atom. In some embodiments, these modifications occur at any of the bonds along the polypeptide chain and even at several (2-3 bonds) at the same time.

In some embodiments, natural aromatic amino acids of the polypeptide such as Trp, Tyr and Phe, be substituted for synthetic non-natural acid such as Phenylglycine, TIC, naphthylelanine (Nol), ring-methylated derivatives of Phe, halogenated derivatives of Phe or o-methyl-Tyr. In some embodiments, the polypeptides include one or more modified amino acid or one or more non-amino acid monomers (e.g. fatty acid, complex carbohydrates etc).

In one embodiment, "amino acid" or "amino acid" is understood to include the 20 naturally occurring amino acid; those amino acid often modified post-translationally *in vivo,* including, for example, hydroxyproline, phosphoserine and phosphothreonine; and other unusual amino acid including, but not limited to, 2-aminoadipic acid, hydroxylysine, isodesmosine, nor-valine, nor-leucine and ornithine. In one embodiment, "amino acid" includes both D- and L-amino acid.

In some embodiments, the polypeptides are utilized in therapeutics which requires the polypeptides to be in a soluble form. In some embodiments, the polypeptides include one or more non-natural or natural polar amino acid, including but not limited to serine and threonine which are capable of increasing polypeptide solubility due to their hydroxyl-containing side chain.

In some embodiments, the polypeptides are utilized in a linear form, although it will be appreciated by one skilled in the art that in cases where cyclization does not severely interfere with polypeptide characteristics, cyclic forms of the polypeptide can also be utilized.

In some embodiments, the polypeptides are biochemically synthesized such as by using standard solid phase techniques. In some embodiments, these biochemical methods include exclusive solid phase synthesis, partial solid phase synthesis, fragment condensation, or classical solution synthesis. In some embodiments, these methods are used when the polypeptide is relatively short (about 5-15kDa) and/or when it cannot be produced by recombinant techniques (i.e., not encoded by a nucleic acid sequence) and therefore involves different chemistry.

Solid phase polypeptide synthesis procedures are well known to one skilled in the art and further described by John Morrow Stewart and Janis Dillaha Young, Solid Phase Polypeptide Syntheses (2nd Ed., Pierce Chemical Company, 1984). In some embodiments, synthetic polypeptides are purified by preparative high performance liquid chromatography [Creighton T. (1983) Proteins, structures and molecular principles. WH Freeman and Co. N.Y.] and the composition of which can be confirmed via amino acid sequencing by methods known to one skilled in the art.

In some embodiments, recombinant protein techniques are used to generate the polypeptides for use in the present invention. In some embodiments, recombinant protein techniques are used for generation of relatively long polypeptides (e.g., longer than 18-25 amino acid). In some embodiments, recombinant protein techniques are used for the generation of large amounts of the polypeptide. Recombinant techniques are described by Bitter et al., (1987) Methods in Enzymol. 153:516-544, Studier et al. (1990) Methods in Enzymol. 185:60-89, Brisson et al. (1984) Nature 310:511-514, Takamatsu et al. (1987) EMBO J. 6:307-311, Coruzzi et al. (1984) EMBO J. 3:1671-1680 and Brogli et al, (1984) Science 224:838-843, Gurley et al. (1986) Mol. Cell. Biol. 6:559-565 and Weissbach & Weissbach, 1988, Methods for Plant Molecular Biology, Academic Press, NY, Section VIII, pp 421-463.

In one embodiment, a polypeptide for use in the present invention is synthesized using a polynucleotide encoding the polypeptide. In some embodiments, the polynucleotide encoding the polypeptide is ligated into an expression vector, comprising a transcriptional control of a cis-regulatory sequence (e.g., promoter sequence). In some embodiments, the cis-regulatory sequence is suitable for directing constitutive expression of the polypeptide. In some embodiments, the cis-regulatory sequence is suitable for directing tissue specific expression of the polypeptide. In some embodiments, the cis-regulatory sequence is suitable for directing inducible expression of the polypeptide.

In some embodiments, polynucleotides which express the polypeptides for use in the present invention are as set forth in SEQ ID NOs: 44 and 45.

In some embodiments, suitable tissue-specific promoters include sequences which are functional in specific cell population, for example promoters such as albumin that is liver specific [Pinkert et al., (1987) Genes Dev. 1:268-277], lymphoid specific promoters [Calame et al., (1988) Adv. Immunol. 43:235-275]; in particular promoters of T-cell receptors [Winoto et al., (1989) EMBO J. 8:729-733] and immunoglobulins; [Banerji et al. (1983) Cell 33729-740], neuron-specific promoters such as the neurofilament promoter [Byrne et al. (1989) Proc. Natl. Acad. Sci. USA 86:5473-5477], pancreas-specific promoters [Edlunch et al. (1985) Science 230:912-916] or mammary gland-specific promoters such as the milk whey promoter (U.S. Pat. No. 4,873,316 and European Application Publication No. 264,166). Inducible promoters include for example the tetracycline-inducible promoter (Srour, M.A., et al., 2003. Thromb. Haemost. 90: 398-405).

In one embodiment, the phrase "a polynucleotide" refers to a single or double stranded nucleic acid sequence which be isolated and provided in the form of an RNA sequence, a complementary polynucleotide sequence (cDNA), a genomic polynucleotide sequence and/or a composite polynucleotide sequences (e.g., a combination of the above).

In one embodiment, "complementary polynucleotide sequence" refers to a sequence, which results from reverse transcription of messenger RNA using a reverse transcriptase or any other RNA dependent DNA polymerase. In one embodiment, the sequence can be subsequently amplified *in vivo* or *in vitro* using a DNA polymerase.

In one embodiment, "genomic polynucleotide sequence" refers to a sequence derived (isolated) from a chromosome and thus it represents a contiguous portion of a chromosome.

In one embodiment, "composite polynucleotide sequence" refers to a sequence, which is at least partially complementary and at least partially genomic. In one embodiment, a composite sequence can include some exonal sequences required to encode the polypeptide, as well as some intronic sequences interposing there between. In one embodiment, the intronic sequences can be of any source, including of other genes, and typically will include conserved splicing signal sequences. In one embodiment, intronic sequences include cis acting expression regulatory elements.

In one embodiment, the polynucleotides encoding the polypeptides for use in the present invention further comprise a signal sequence encoding a signal peptide for the secretion of the polypeptides. In some embodiments, signal sequences include the endogenous signal sequence for EPO as set forth in SEQ ID NO: 19 or the endogenous signal sequence for IFN-β1 as set forth in SEQ ID NO: 26. In another embodiment, the signal sequence is N-terminal to the CTP sequence that is in turn N-terminal to the polypeptide sequence of interest; e.g. the sequence is (a) signal sequence- (b) CTP- (c) sequence of interest- (d) optionally 1 or more additional CTP sequences. In another embodiment, 1 or more CTP sequences is inserted between the signal sequence of a polypeptide sequence of interest and the polypeptide sequence of interest itself, thus interrupting the wild-type sequence of interest.

In another embodiment, the growth hormone further comprises a signal peptide. In some embodiments, signal sequences include, but are not limited to the endogenous signal sequence. In some embodiments, signal sequences include, but are not limited to the endogenous signal sequence of any known growth hormone or growth hormones. In another embodiment, the polypeptides for use in the present invention provide a growth hormone having additionally a signal peptide comprising the following amino acid sequence: MATGSRTSLLLAFGLLCLPWLQEGSA (SEQ ID NO: 49).

In one embodiment, following expression and secretion, the signal peptides are cleaved from the precursor proteins resulting in the mature proteins.

In some embodiments, polynucleotides encoding the polypeptides for use in the present invention are prepared using PCR techniques using procedures and methods known to one skilled in the art. In some embodiments, the procedure involves the legation of two different DNA sequences (See, for example, "Current Protocols in Molecular Biology", eds. Ausubel et al., John Wiley & Sons, 1992).

In one embodiment, the polynucleotides are inserted into expression vectors (i.e., a nucleic acid construct) to enable expression of the recombinant polypeptide. In one embodiment, the expression vector includes additional sequences which render this vector suitable for replication and integration in prokaryotes. In one embodiment, the expression vector includes additional sequences which render this vector suitable for replication and integration in eukaryotes. In one embodiment, the expression vector includes a shuttle vector which renders this vector suitable for replication and integration in both prokaryotes and eukaryotes. In some embodiments, cloning vectors comprise transcription and translation initiation sequences (e.g., promoters, enhancer) and transcription and translation terminators (e.g., polyadenylation signals).

In one embodiment, a variety of prokaryotic or eukaryotic cells can be used as host-expression systems to express the polypeptides for use in the present invention. In some embodiments, these include, but are not limited to, microorganisms, such as bacteria transformed with a recombinant bacteriophage DNA, plasmid DNA or cosmid DNA expression vector containing the polypeptide coding sequence; yeast transformed with recombinant yeast expression vectors containing the polypeptide coding sequence; plant cell systems infected with recombinant virus expression vectors (e.g., cauliflower mosaic virus, CaMV; tobacco mosaic virus, TMV) or transformed with recombinant plasmid expression vectors, such as Ti plasmid, containing the polypeptide coding sequence.

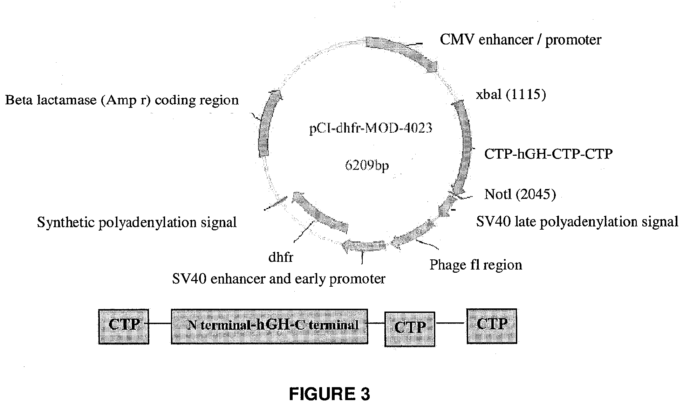

In some embodiments, non-bacterial expression systems are used (e.g. mammalian expression systems such as CHO cells) to express the polypeptide for use in the present invention. In one embodiment, the expression vector used to express polynucleotides in mammalian cells is pCI-DHFR vector comprising a CMV promoter and a neomycin resistance gene. Construction of the pCI-dhfr vector is described, according to one embodiment, in Example 1 and Figure 3.

In some embodiments, in bacterial systems, a number of expression vectors can be advantageously selected depending upon the use intended for the polypeptide expressed. In one embodiment, large quantities of polypeptide are desired. In one embodiment, vectors that direct the expression of high levels of the protein product, possibly as a fusion with a hydrophobic signal sequence, which directs the expressed product into the periplasm of the bacteria or the culture medium where the protein product is readily purified are desired. In one embodiment, certain fusion protein engineered with a specific cleavage site to aid in recovery of the polypeptide. In one embodiment, vectors adaptable to such manipulation include, but are not limited to, the pET series of *E. coli* expression vectors [Studier et al., Methods in Enzymol. 185:60-89 (1990)].

In one embodiment, yeast expression systems are used. In one embodiment, a number of vectors containing constitutive or inducible promoters can be used in yeast as disclosed in U.S. Patent. No: 5,932,447. In another embodiment, vectors which promote integration of foreign DNA sequences into the yeast chromosome are used.

In one embodiment, the expression vector can further include additional polynucleotide sequences that allow, for example, the translation of several proteins from a single mRNA such as an internal ribosome entry site (IRES) and sequences for genomic integration of the promoter-chimeric polypeptide.

In some embodiments, mammalian expression vectors include, but are not limited to, pcDNA3, pcDNA3.1(+/-), pGL3, pZeoSV2(+/-), pSecTag2, pDisplay, pEF/myc/cyto, pCMV/myc/cyto, pCR3.1, pSinRep5, DH26S, DHBB, pNMT1, pNMT41, pNMT81, which are available from Invitrogen, pCI which is available from Promega, pMbac, pPbac, pBK-RSV and pBK-CMV which are available from Stratagene, pTRES which is available from Clontech, and their derivatives.

In some embodiments, expression vectors containing regulatory elements from eukaryotic viruses such as retroviruses are used. SV40 vectors include pSVT7 and pMT2. In some embodiments, vectors derived from bovine papilloma virus include pBV-1MTHA, and vectors derived from Epstein Bar virus include pHEBO, and p205. Other exemplary vectors include pMSG, pAV009/A⁺, pMTO10/A⁺, pMAMneo-5, baculovirus pDSVE, and any other vector allowing expression of proteins under the direction of the SV-40 early promoter, SV-40 later promoter, metallothionein promoter, murine mammary tumor virus promoter, Rous sarcoma virus promoter, polyhedrin promoter, or other promoters shown effective for expression in eukaryotic cells.

In some embodiments, recombinant viral vectors are useful for *in vivo* expression of the polypeptides since they offer advantages such as lateral infection and targeting specificity. In one embodiment, lateral infection is inherent in the life cycle of, for example, retrovirus and is the process by which a single infected cell produces many progeny virions that bud off and infect neighboring cells. In one embodiment, the result is that a large area becomes rapidly infected, most of which was not initially infected by the original viral particles. In one embodiment, viral vectors are produced that are unable to spread laterally. In one embodiment, this characteristic can be useful if the desired purpose is to introduce a specified gene into only a localized number of targeted cells.

In one embodiment, various methods can be used to introduce the expression vector into cells. Such methods are generally described in Sambrook et al., Molecular Cloning: A Laboratory Manual, Cold Springs Harbor Laboratory, New York (1989, 1992), in Ausubel et al., Current Protocols in Molecular Biology, John Wiley and Sons, Baltimore, Md. (1989), Chang et al., Somatic Gene Therapy, CRC Press, Ann Arbor, Mich. (1995), Vega et al., Gene Targeting, CRC Press, Ann Arbor Mich. (1995), Vectors: A Survey of Molecular Cloning Vectors and Their Uses, Butterworths, Boston Mass. (1988) and Gilboa et at. [Biotechniques 4 (6): 504-512, 1986] and include, for example, stable or transient transfection, lipofection, electroporation and infection with recombinant viral vectors. In addition, see U.S. Patent Nos. 5,464,764 and 5,487,992 for positive-negative selection methods.

In some embodiments, introduction of nucleic acid by viral infection offers several advantages over other methods such as lipofection and electroporation, since higher transfection efficiency can be obtained due to the infectious nature of viruses.

In one embodiment of the disclosure, it will be appreciated that the polypeptides can also be expressed from a nucleic acid construct administered to the individual employing any suitable mode of administration, described hereinabove (i.e., in-vivo gene therapy). In one embodiment, the nucleic acid construct is introduced into a suitable cell via an appropriate gene delivery vehicle/method (transfection, transduction, homologous recombination, etc.) and an expression system as needed and then the modified cells are expanded in culture and returned to the individual (i.e., ex-vivo gene therapy).

In one embodiment, plant expression vectors are used. In one embodiment, the expression of a polypeptide coding sequence is driven by a number of promoters. In some embodiments, viral promoters such as the 35S RNA and 19S RNA promoters of CaMV [Brisson et al., Nature 310:511-514 (1984)], or the coat protein promoter to TMV [Takamatsu et al., EMBO J. 6:307-311 (1987)] are used. In another embodiment, plant promoters are used such as, for example, the small subunit of RUBISCO [Coruzzi et al., EMBO J. 3:1671-1680 (1984); and Brogli et al., Science 224:838-843 (1984)] or heat shock promoters, e.g., soybean hsp17.5-E or hsp17.3-B [Gurley et al., Mol. Cell. Biol. 6:559-565 (1986)]. In one embodiment, constructs are introduced into plant cells using Ti plasmid, Ri plasmid, plant viral vectors, direct DNA transformation, microinjection, electroporation and other techniques well known to the skilled artisan. See, for example, Weissbach & Weissbach [Methods for Plant Molecular Biology, Academic Press, NY, Section VIII, pp 421-463 (1988)]. Other expression systems such as insects and mammalian host cell systems, which are well known in the art, can also be used.

It will be appreciated that other than containing the necessary elements for the transcription and translation of the inserted coding sequence (encoding the polypeptide), the expression construct can also include sequences engineered to optimize stability, production, purification, yield or activity of the expressed polypeptide.

Various methods, in some embodiments, can be used to introduce the expression vector into the host cell system. In some embodiments, such methods are generally described in Sambrook et al., Molecular Cloning: A Laboratory Manual, Cold Springs Harbor Laboratory, New York (1989, 1992), in Ausubel et al., Current Protocols in Molecular Biology, John Wiley and Sons, Baltimore, Md. (1989), Chang et al., Somatic Gene Therapy, CRC Press, Ann Arbor, Mich. (1995), Vega et al., Gene Targeting, CRC Press, Ann Arbor Mich. (1995), Vectors: A Survey of Molecular Cloning Vectors and Their Uses, Butterworths, Boston Mass. (1988) and Gilboa et at. [Biotechniques 4 (6): 504-512, 1986] and include, for example, stable or transient transfection, lipofection, electroporation and infection with recombinant viral vectors. In addition, see U.S. Patent Nos. 5,464,764 and 5,487,992 for positive-negative selection methods.

In some embodiments, transformed cells are cultured under effective conditions, which allow for the expression of high amounts of recombinant polypeptide. In some embodiments, effective culture conditions include, but are not limited to, effective media, bioreactor, temperature, pH and oxygen conditions that permit protein production. In one embodiment, an effective medium refers to any medium in which a cell is cultured to produce the recombinant polypeptide. In some embodiments, a medium typically includes an aqueous solution having assimilable carbon, nitrogen and phosphate sources, and appropriate salts, minerals, metals and other nutrients, such as vitamins. In some embodiments, cells can be cultured in conventional fermentation bioreactors, shake flasks, test tubes, microtiter dishes and petri plates. In some embodiments, culturing is carried out at a temperature, pH and oxygen content appropriate for a recombinant cell. In some embodiments, culturing conditions are within the expertise of one of ordinary skill in the art.

In some embodiments, depending on the vector and host system used for production, resultant polypeptides either remain within the recombinant cell, secreted into the fermentation medium, secreted into a space between two cellular membranes, such as the periplasmic space in *E. coli;* or retained on the outer surface of a cell or viral membrane.

In one embodiment, following a predetermined time in culture, recovery of the recombinant polypeptide is effected.

In one embodiment, the phrase "recovering the recombinant polypeptide" used herein refers to collecting the whole fermentation medium containing the polypeptide and need not imply additional steps of separation or purification.

In one embodiment, polypeptides for use in the present invention are purified using a variety of standard protein purification techniques, such as, but not limited to, affinity chromatography, ion exchange chromatography, filtration, electrophoresis, hydrophobic interaction chromatography, gel filtration chromatography, reverse phase chromatography, concanavalin A chromatography, chromatofocusing and differential solubilization.

In one embodiment, to facilitate recovery, the expressed coding sequence can be engineered to encode the polypeptide and fused cleavable moiety. In one embodiment, a fusion protein can be designed so that the polypeptide can be readily isolated by affinity chromatography; e.g., by immobilization on a column specific for the cleavable moiety. In one embodiment, a cleavage site is engineered between the polypeptide and the cleavable moiety and the polypeptide can be released from the chromatographic column by treatment with an appropriate enzyme or agent that specifically cleaves the fusion protein at this site [e.g., see Booth et al., Immunol. Lett. 19:65-70 (1988); and Gardella et al., J. Biol. Chem. 265:15854-15859 (1990)].

In one embodiment, the polypeptide for use in the present invention is retrieved in "substantially pure" form.

In one embodiment, the phrase "substantially pure" refers to a purity that allows for the effective use of the protein in the applications described herein.

In one embodiment, the polypeptide for use in the present invention can also be synthesized using *in vitro* expression systems. In one embodiment, *in vitro* synthesis methods are well known in the art and the components of the system are commercially available.

In some embodiments, the recombinant polypeptides are synthesized and purified; their therapeutic efficacy can be assayed in *vivo* or *in vitro.* In one embodiment, production of GH modified by CTPs using recombinant DNA technology is performed.

In some embodiments, the recombinant polypeptides are synthesized and purified; their therapeutic efficacy can be assayed either *in vivo* or *in vitro.* In one embodiment, the binding activities of the recombinant GH modified by CTPs can be ascertained using various assays.

The present invention utilizes CTP-GH-CTP-CTP polypeptides. In one embodiment, recombinant DNA technology methods are used for the production of CTP-GH-CTP-CTP polypeptides, as illustrated in Example 1 and Figure 1. In one embodiment, the therapeutic efficacy of the CTP-GH-CTP-CTP polypeptides is assayed *in vivo.* In one embodiment, the therapeutic efficacy of the CTP-GH-CTP-CTP polypeptides is assayed *in vitro.* In one embodiment, the binding activities of the recombinant GH polypeptides are measured using Nb2 (a prolactin-dependent rat lymphoma cell line (ECACC Cell Bank)) or a FCD-P1 murine cell line, previously transfected with human growth hormone receptor. In one embodiment, binding of GH to these receptors induces cell proliferation which in one embodiment is measured by the levels of MTT cellular stain as a function of GH activity. In one embodiment, *in vivo* activity is deduced by measuring weight gain over time in treated growth hormone deficient animals.

In one embodiment, administering to the subject a therapeutically effective amount of a polypeptide comprising a growth hormone, one chorionic gonadotropin carboxy terminal peptide (CTP) attached to an amino terminus of said growth hormone, and two chorionic gonadotropin CTPs attached to a carboxy terminus of the growth hormone, thereby induces growth or weight gain in a subject.

It is disclosed that the present invention may act by decreasing fat deposits in a subject. In another embodiment, the present invention may act by increasing muscle mass in a subject. In another embodiment, the present invention may act by promoting muscle growth in a subject. In another embodiment, the present invention may act by increasing muscle to fat ratio. In another embodiment, the present invention may act by decreasing body mass index (BMI) or Quetelet index.

In another embodiment, growth is measured by weight gain. In another embodiment, growth is measured by height gain. In another embodiment, growth is measured by muscle mass gain. In another embodiment, growth is measured by bone mass gain. In another embodiment, the weight gain is due to bone and/or muscle mass gain. In another embodiment, growth is measured by any known measure known to one of skill in the art.

As disclosed herein, human growth hormone polypeptides of the present disclosure can be used to treat a subject, with conditions related to growth and weight, such as a growth deficiency disorder, AIDS wasting, aging, impaired immune function of HIV-infected subjects, a catabolic illness, surgical recovery, a congestive cardiomyopathy, liver transplantation, liver regeneration after hepatectomy, chronic renal failure, renal osteodystrophy, osteoporosis, achondroplasia/hypochondroplasia, skeletal dysplasia, a chronic inflammatory or nutritional disorder such as Crohn's disease, short bowel syndrome, juvenile chronic arthritis, cystic fibrosis, male infertility, X-linked hypophosphatemic rickets, Down's syndrome, Spina bifida, Noonan Syndrome, obesity, impaired muscle strength and fibromyalgia.

In one embodiment, the polypeptides utilized in the present invention can be provided to the individual *per se.* In one embodiment, the polypeptides utilized in the present invention can be provided to the individual as part of a pharmaceutical composition where it is mixed with a pharmaceutically acceptable carrier.

In one embodiment, a "pharmaceutical composition" refers to a preparation of one or more of the active ingredients described herein with other chemical components such as physiologically suitable carriers and excipients. The purpose of a pharmaceutical composition is to facilitate administration of a compound to an organism.

In one embodiment, "active ingredient" refers to the polypeptide sequence of interest, which is accountable for the biological effect.

In one embodiment, the pharmaceutical composition for use in the present invention may be provided as combined preparations. In one embodiment, "a combined preparation" defines especially a "kit of parts" in the sense that the combination partners as defined above can be dosed independently or by use of different fixed combinations with distinguished amounts of the combination partners i.e., simultaneously, concurrently, separately or sequentially. In some embodiments, the parts of the kit of parts can then, e.g., be administered simultaneously or chronologically staggered, that is at different time points and with equal or different time intervals for any part of the kit of parts. The ratio of the total amounts of the combination partners, in some embodiments, can be administered in the combined preparation. In one embodiment, the combined preparation can be varied, e.g., in order to cope with the needs of a patient subpopulation to be treated or the needs of the single patient which different needs can be due to a particular disease, severity of a disease, age, sex, or body weight as can be readily made by a person skilled in the art.

In one embodiment, the phrases "physiologically acceptable carrier" and "pharmaceutically acceptable carrier" which can be used interchangeably used refer to a carrier or a diluent that does not cause significant irritation to an organism and does not abrogate the biological activity and properties of the administered compound. An adjuvant is included under these phrases. In one embodiment, one of the ingredients included in the pharmaceutically acceptable carrier can be for example polyethylene glycol (PEG), a biocompatible polymer with a wide range of solubility in both organic and aqueous media (Mutter et al. (1979).

In one embodiment, "excipient" refers to an inert substance added to a pharmaceutical composition to further facilitate administration of an active ingredient. In one embodiment, excipients include calcium carbonate, calcium phosphate, various sugars and types of starch, cellulose derivatives, gelatin, vegetable oils and polyethylene glycols.

Techniques for formulation and administration of drugs are found in "Remington's Pharmaceutical Sciences," Mack Publishing Co., Easton, PA, latest edition.

In one embodiment, suitable routes of administration, for example, include oral, rectal, transmucosal, transnasal, intestinal or parenteral delivery, including intramuscular, subcutaneous and intramedullary injections as well as intrathecal, direct intraventricular, intravenous, intraperitoneal, intranasal, or intraocular injections.

In one embodiment, the preparation is administered in a local rather than systemic manner, for example, via injection of the preparation directly into a specific region of a patient's body.

In one embodiment, where the pharmaceutical formulation or the pharmaceutical composition is administered via injection to a subject, it is done so using a syringe or a PEN device.

In another embodiment, polypeptides comprising GH modified by CTPs may be administered in a dose of 5 milligrams (mg) in 1 ml solution, or 10 mg in 1 ml solution, or 20 mg in 1 ml solution.

In one embodiment, administration is by intramuscular (IM) injection. In one embodiment, administration is by subcutaneous (SC) injection. In one embodiment, administration is by intravenous (IV) injection.

The dosage of the GH modified by CTPs in this invention is 0.66 mg/kg/week.

It is also disclosed that the GH dosage given to a subject may be 50% of the standard dosage given to a reference subject from the same population of subjects (e.g. children, elderly, men, women, GH deficient, specific nationality, etc). In another disclosure the dosage may be 30% of the dosage given to a subject from a specific population of subjects. In another disclosure the dosage may be 45% of the dosage given to a subject from a specific population of subjects. In another disclosure the dosage may be 100% of the dosage given to a subject from a specific population of subjects.

In another disclosure the dosage is 1-5 mg/administration. In another disclosure the dosage is 2 mg/administration. In another disclosure the dosage is 4 mg/administration. In another disclosure the dosage is 1.2 mg/administration. In another disclosure the dosage is 1.8 mg/administration. In this invention, the composition is administered once a week.

In one embodiment, GH modified by CTPs is formulated in a liquid formulation.

In another embodiment, GH modified by CTPs is formulated in an intranasal dosage form. In another embodiment, GH modified by CTPs is formulated in an injectable dosage form.

In another disclosure a GH modified by CTPs is administered to a subject in a dose ranging from 2 mg to 6 mg. In another disclosure a GH modified by CTPs is administered to a subject in a dose ranging from 4 mg to 10 mg. In another disclosure a GH modified by CTPs is administered to a subject in a dose ranging from 5 mg and 15 mg.

In another embodiment, a GH modified by CTPs is injected into the muscle (intramuscular injection). In another embodiment, a GH modified by CTPs is injected below the skin (subcutaneous injection). In another embodiment, a GH modified by CTPs is injected into the muscle. In another embodiment, a GH modified by CTPs is injected below the skin.

In another embodiment, providing to a subject in need thereof a GH modified by CTPs thereby increases compliance in the use of growth hormone therapy.

In another embodiment, protein drugs of molecular weight lower than 50,000 Daltons, such as GH modified by CTPs as used in the present invention, are in general short-lived species *in vivo,* having short circulatory half-lives of several hours. In another embodiment, the subcutaneous route of administration in general provides slower release into the circulation. In another embodiment, the CTP modified polypeptide prolongs the half-life of protein drugs of molecular weight lower than 50,000 Daltons, such as GH. In another embodiment, the CTP modified polypeptide enable interferons to exert their beneficial effects for a longer period of time.

In another embodiment, it is disclosed that the immunogenicity of a CTP modified polypeptide comprising a GH modified by CTPs may be equal to an isolated GH. In another embodiment, the immunogenicity of a CTP modified polypeptide comprising a GH modified by CTPs is comparable to an isolated GH. In another embodiment, modifying a GH as described herein with CTP peptides reduces the immunogenicity of the GH. In another embodiment, the CTP modified polypeptide comprising a GH is as active as an isolated GH protein. In another embodiment, the CTP modified polypeptide comprising a GH is more active than an isolated GH. In another embodiment, the CTP modified polypeptide comprising a GH maximizes the growth hormone's protective ability against degradation while minimizing reductions in bioactivity.

In another embodiment, the invention increases the compliance of subjects afflicted with chronic illnesses that are in need of a GH therapy. In another embodiment, the invention enables reduction in the dosing frequency of a GH by modifying the GH with CTPs as described hereinabove. In another embodiment, the term compliance comprises adherence. In another embodiment, the invention includes increasing the compliance of patients in need of a GH therapy by reducing the frequency of administration of the GH. In another embodiment, reduction in the frequency of administration of the GH is achieved due to the CTP modifications which render the CTP-modified GH more stable. In another embodiment, reduction in the frequency of administration of the GH is achieved as a result of increasing T1/2 of the growth hormone. In another embodiment, reduction in the frequency of administration of the GH is achieved as a result of increasing the clearance time of the GH. In another embodiment, reduction in the frequency of administration of the growth hormone is achieved as a result of increasing the AUC measure of the growth hormone.

In another embodiment, administering to said subject a therapeutically effective amount of a polypeptide comprising a growth hormone, one chorionic gonadotropin carboxy terminal peptide (CTP) attached to the amino terminus of said growth hormone, and two chorionic gonadotropin CTPs attached to the carboxy terminus of said growth hormone, thereby increases IGF-1 levels in said subject.

It is disclosed that increasing IGF-1 levels in a human subject may be effective in treating, preventing or suppressing type 1 diabetes, type 2 diabetes, amyotrophic lateral sclerosis (ALS aka "Lou Gehrig's Disease"), severe burn injury and myotonic muscular dystrophy (MMD).

Oral administration, in one embodiment, comprises a unit dosage form comprising tablets, capsules, lozenges, chewable tablets, suspensions, emulsions and the like. Such unit dosage forms comprise a safe and effective amount of the desired compound, or compounds. The pharmaceutically-acceptable carriers suitable for the preparation of unit dosage forms for peroral administration are well-known in the art. In some embodiments, tablets typically comprise conventional pharmaceutically-compatible adjuvants as inert diluents, such as calcium carbonate, sodium carbonate, mannitol, lactose and cellulose; binders such as starch, gelatin and sucrose; disintegrants such as starch, alginic acid and croscarmelose; lubricants such as magnesium stearate, stearic acid and talc. In one embodiment, glidants such as silicon dioxide can be used to improve flow characteristics of the powder-mixture. In one embodiment, coloring agents, such as the FD&C dyes, can be added for appearance. Sweeteners and flavoring agents, such as aspartame, saccharin, menthol, peppermint, and fruit flavors, are useful adjuvants for chewable tablets. Capsules typically comprise one or more solid diluents disclosed above. In some embodiments, the selection of carrier components depends on secondary considerations like taste, cost, and shelf stability, which are not critical for the purposes of this invention, and can be readily made by a person skilled in the art.

In one embodiment, the oral dosage form comprises predefined release profile. In one embodiment, the oral dosage form comprises an extended release tablets, capsules, lozenges or chewable tablets. In one embodiment, the oral dosage form comprises a slow release tablets, capsules, lozenges or chewable tablets. In one embodiment, the oral dosage form comprises an immediate release tablets, capsules, lozenges or chewable tablets. In one embodiment, the oral dosage form is formulated according to the desired release profile of the pharmaceutical active ingredient as known to one skilled in the art.

Peroral compositions, in some embodiments, comprise liquid solutions, emulsions, suspensions, and the like. In some embodiments, pharmaceutically-acceptable carriers suitable for preparation of such compositions are well known in the art. In some embodiments, liquid oral compositions comprise from about 0.012% to about 0.933% of the desired compound or compounds, or in another embodiment, from about 0.033% to about 0.7%.

In some embodiments, compositions for use in this invention comprise solutions or emulsions, which in some embodiments are aqueous solutions or emulsions comprising a safe and effective amount of the CTP-modified GH compounds and optionally, other compounds, intended for topical intranasal administration. In some embodiments, compositions comprise from about 0.01% to about 10.0% w/v of a subject compound, more preferably from about 0.1% to about 2.0, which is used for systemic delivery of the compounds by the intranasal route.

In another embodiment, the pharmaceutical compositions are administered by intravenous, intra-arterial, or intramuscular injection of a liquid preparation. In some embodiments, liquid formulations include solutions, suspensions, dispersions, emulsions, oils and the like. In one embodiment, the pharmaceutical compositions are administered intravenously, and are thus formulated in a form suitable for intravenous administration. In another embodiment, the pharmaceutical compositions are administered intra-arterially, and are thus formulated in a form suitable for intra-arterial administration. In another embodiment, the pharmaceutical compositions are administered intramuscularly, and are thus formulated in a form suitable for intramuscular administration.

In another embodiment, the pharmaceutical compositions are administered topically to body surfaces, and are thus formulated in a form suitable for topical administration. Suitable topical formulations include gels, ointments, creams, lotions, drops and the like. For topical administration, the compounds are combined with an additional appropriate therapeutic agent or agents, prepared and applied as solutions, suspensions, or emulsions in a physiologically acceptable diluent with or without a pharmaceutical carrier.

In one embodiment, pharmaceutical compositions are manufactured by processes well known in the art, e.g., by means of conventional mixing, dissolving, granulating, dragee-making, levigating, emulsifying, encapsulating, entrapping or lyophilizing processes.

In one embodiment, pharmaceutical compositions for use in the present invention is formulated in conventional manner using one or more physiologically acceptable carriers comprising excipients and auxiliaries, which facilitate processing of the active ingredients into preparations which, can be used pharmaceutically. In one embodiment, formulation is dependent upon the route of administration chosen.

In one embodiment, injectables are formulated in aqueous solutions. In one embodiment, injectables are formulated in physiologically compatible buffers such as Hank's solution, Ringer's solution, or physiological salt buffer. In some embodiments, for transmucosal administration, penetrants appropriate to the barrier to be permeated are used in the formulation. Such penetrants are generally known in the art.

In one embodiment, the preparations described herein are formulated for parenteral administration, e.g., by bolus injection or continuous infusion. In some embodiments, formulations for injection are presented in unit dosage form, e.g., in ampoules or in multidose containers with optionally, an added preservative. In some embodiments, compositions are suspensions, solutions or emulsions in oily or aqueous vehicles, and contain formulatory agents such as suspending, stabilizing and/or dispersing agents.

The compositions also comprise, in some embodiments, preservatives, such as benzalkonium chloride and thimerosal and the like; chelating agents, such as edetate sodium and others; buffers such as phosphate, citrate and acetate; tonicity agents such as sodium chloride, potassium chloride, glycerin, mannitol and others; antioxidants such as ascorbic acid, acetylcystine, sodium metabisulfite and others; aromatic agents; viscosity adjustors, such as polymers, including cellulose and derivatives thereof; and polyvinyl alcohol and acid and bases to adjust the pH of these aqueous compositions as needed. The compositions also comprise, in some embodiments, local anesthetics or other actives. The compositions can be used as sprays, mists, drops, and the like.

In some embodiments, pharmaceutical compositions for parenteral administration include aqueous solutions of the active preparation in water-soluble form. Additionally, suspensions of the active ingredients, in some embodiments, are prepared as appropriate oily or water based injection suspensions. Suitable lipophilic solvents or vehicles include, in some embodiments, fatty oils such as sesame oil, or synthetic fatty acid esters such as ethyl oleate, triglycerides or liposomes. Aqueous injection suspensions contain, in some embodiments, substances, which increase the viscosity of the suspension, such as sodium carboxymethyl cellulose, sorbitol or dextran. In another embodiment, the suspension also contain suitable stabilizers or agents which increase the solubility of the active ingredients to allow for the preparation of highly concentrated solutions.

In another embodiment, the active compound can be delivered in a vesicle, in particular a liposome (see Langer, Science 249:1527-1533 (1990); Treat et al., in Liposomes in the Therapy of Infectious Disease and Cancer, Lopez- Berestein and Fidler (eds.), Liss, New York, pp. 353-365 (1989); Lopez-Berestein, ibid., pp. 317-327; see generally ibid).

In another embodiment, the pharmaceutical composition delivered in a controlled release system is formulated for intravenous infusion, implantable osmotic pump, transdermal patch, a syringe, liposomes, or other modes of administration. In one embodiment, a pump is used (see Langer, supra; Sefton, CRC Crit. Ref. Biomed. Eng. 14:201 (1987); Buchwald et al., Surgery 88:507 (1980); Saudek et al., N. Engl. J. Med. 321:574 (1989). In another embodiment, polymeric materials can be used. In yet another embodiment, a controlled release system can be placed in proximity to the therapeutic target, i.e., the brain, thus requiring only a fraction of the systemic dose (see, e.g., Goodson, in Medical Applications of Controlled Release, supra, vol. 2, pp. 115-138 (1984). Other controlled release systems are discussed in the review by Langer (Science 249:1527-1533 (1990).

In some embodiments, the active ingredient is in powder form for constitution with a suitable vehicle, e.g., sterile, pyrogen-free water based solution, before use. Compositions are formulated, in some embodiments, for atomization and inhalation administration. In another embodiment, compositions are contained in a container with attached atomizing means.

In one embodiment, the preparation is formulated in rectal compositions such as suppositories or retention enemas, using, e.g., conventional suppository bases such as cocoa butter or other glycerides.

In one embodiment, the preparation is formulated in liquid formulations for injection via a syringe or Pen device. In some embodiments, pharmaceutical compositions suitable for use in context of the present invention include compositions wherein the active ingredients are contained in an amount effective to achieve the intended purpose. In some embodiments, a therapeutically effective amount means an amount of active ingredients effective to prevent, alleviate or ameliorate symptoms of disease or prolong the survival of the subject being treated.

In one embodiment, determination of a therapeutically effective amount is well within the capability of those skilled in the art.

In one embodiment, the formulations provided herein also comprise preservatives, such as benzalkonium chloride and thimerosal and the like; chelating agents, such as edetate sodium and others; buffers such as phosphate, citrate and acetate; tonicity agents such as sodium chloride, potassium chloride, glycerin, mannitol and others; antioxidants such as ascorbic acid, acetylcystine, sodium metabisulfite and others; aromatic agents; viscosity adjustors, such as polymers, including cellulose and derivatives thereof; and polyvinyl alcohol and acid and bases to adjust the pH of these aqueous compositions as needed. The compositions also comprise local anesthetics or other actives. The compositions can be used as sprays, mists, drops, and the like.

Some examples of substances which can serve as pharmaceutically-acceptable carriers or components thereof are sugars, such as lactose, glucose and sucrose; starches, such as corn starch and potato starch; cellulose and its derivatives, such as sodium carboxymethyl cellulose, ethyl cellulose, and methyl cellulose; powdered tragacanth; malt; gelatin; talc; solid lubricants, such as stearic acid and magnesium stearate; calcium sulfate; vegetable oils, such as peanut oil, cottonseed oil, sesame oil, olive oil, corn oil and oil of theobroma; polyols such as propylene glycol, glycerine, sorbitol, mannitol, and polyethylene glycol; alginic acid; emulsifiers, such as the Tween™ brand emulsifiers; wetting agents, such sodium lauryl sulfate; coloring agents; flavoring agents; tableting agents, stabilizers; antioxidants; preservatives; pyrogen-free water; isotonic saline; and phosphate buffer solutions. The choice of a pharmaceutically-acceptable carrier to be used in conjunction with the compound is basically determined by the way the compound is to be administered. If the subject compound is to be injected, in one embodiment, the pharmaceutically-acceptable carrier is sterile, physiological saline, with a blood-compatible suspending agent, the pH of which has been adjusted to about 7.4.

In addition, the formulations further comprise binders (e.g. acacia, cornstarch, gelatin, carbomer, ethyl cellulose, guar gum, hydroxypropyl cellulose, hydroxypropyl methyl cellulose, povidone), disintegrating agents (e.g. cornstarch, potato starch, alginic acid, silicon dioxide, croscarmelose sodium, crospovidone, guar gum, sodium starch glycolate), buffers (e.g., Tris-HCI., acetate, phosphate) of various pH and ionic strength, additives such as albumin or gelatin to prevent absorption to surfaces, detergents (e.g., Tween 20, Tween 80, Pluronic F68, bile acid salts), protease inhibitors, surfactants (e.g. sodium lauryl sulfate), permeation enhancers, solubilizing agents (e.g., glycerol, polyethylene glycerol), anti-oxidants (e.g., ascorbic acid, sodium metabisulfite, butylated hydroxyanisole), stabilizers (e.g. hydroxypropyl cellulose, hydroxypropylmethyl cellulose), viscosity increasing agents(e.g. carbomer, colloidal silicon dioxide, ethyl cellulose, guar gum), sweeteners (e.g. aspartame, citric acid), preservatives (e.g., Thimerosal, benzyl alcohol, parabens), lubricants (e.g. stearic acid, magnesium stearate, polyethylene glycol, sodium lauryl sulfate), flow-aids (e.g. colloidal silicon dioxide), plasticizers (e.g. diethyl phthalate, triethyl citrate), emulsifiers (e.g. carbomer, hydroxypropyl cellulose, sodium lauryl sulfate), polymer coatings (e.g., poloxamers or poloxamines), coating and film forming agents (e.g. ethyl cellulose, acrylates, polymethacrylates) and/or adjuvants.

Typical components of carriers for syrups, elixirs, emulsions and suspensions include ethanol, glycerol, propylene glycol, polyethylene glycol, liquid sucrose, sorbitol and water. For a suspension, typical suspending agents include methyl cellulose, sodium carboxymethyl cellulose, cellulose (e.g. Avicel™, RC-591), tragacanth and sodium alginate; typical wetting agents include lecithin and polyethylene oxide sorbitan (e.g. polysorbate 80). Typical preservatives include methyl paraben and sodium benzoate. In another embodiment, peroral liquid compositions also contain one or more components such as sweeteners, flavoring agents and colorants disclosed above.

The formulations provided herein also include incorporation of the active material into or onto particulate preparations of polymeric compounds such as polylactic acid, polyglycolic acid, hydrogels, etc, or onto liposomes, microemulsions, micelles, unilamellar or multilamellar vesicles, erythrocyte ghosts, or spheroplasts. Such compositions will influence the physical state, solubility, stability, rate of *in vivo* release, and rate of *in vivo* clearance.

Also comprehended are particulate compositions coated with polymers (e.g. poloxamers or poloxamines) and the compound coupled to antibodies directed against tissue-specific receptors, ligands or antigens or coupled to ligands of tissue-specific receptors.

In some embodiments, compounds modified by the covalent attachment of water-soluble polymers such as polyethylene glycol, copolymers of polyethylene glycol and polypropylene glycol, carboxymethyl cellulose, dextran, polyvinyl alcohol, polyvinylpyrrolidone or polyproline. In another embodiment, the modified compounds exhibit substantially longer half-lives in blood following intravenous injection than do the corresponding unmodified compounds. In one embodiment, modifications also increase the compound's solubility in aqueous solution, eliminate aggregation, enhance the physical and chemical stability of the compound, and greatly reduce the immunogenicity and reactivity of the compound. In another embodiment, the desired *in vivo* biological activity is achieved by the administration of such polymer-compound abducts less frequently or in lower doses than with the unmodified compound.

In some embodiments, preparation of an effective amount or dose can be estimated initially from *in vitro* assays. In one embodiment, a dose can be formulated in animal models and such information can be used to more accurately determine useful doses in humans.

In one embodiment, toxicity and therapeutic efficacy of the active ingredients described herein can be determined by standard pharmaceutical procedures *in vitro,* in cell cultures or experimental animals. In one embodiment, the data obtained from these *in vitro* and cell culture assays and animal studies can be used in formulating a range of dosage for use in human. In one embodiment, the dosages vary depending upon the dosage form employed and the route of administration utilized. In one embodiment, the exact formulation, route of administration and dosage can be chosen by the individual physician in view of the patient's condition. [See e.g., Fingl, et al., (1975) "The Pharmacological Basis of Therapeutics", Ch. 1 p.1].

In one embodiment, the amount of a composition or formulation to be administered will, of course, be dependent on the subject being treated, the severity of the affliction, the manner of administration, the judgment of the prescribing physician, etc.

In one embodiment, compositions formulated in a compatible pharmaceutical carrier are also be prepared, placed in an appropriate container, and labeled for treatment of an indicated condition.

In another embodiment, a GH modified by CTPs is administered via systemic administration. In another embodiment, a growth hormone as described herein is administered by intravenous, intramuscular or subcutaneous injection. In another embodiment, a GH modified by CTPs is lyophilized (i.e., freeze-dried) preparation in combination with complex organic excipients and stabilizers such as nonionic surface active agents (i.e., surfactants), various sugars, organic polyols and/or human serum albumin. In another embodiment, a pharmaceutical composition comprises a lyophilized GH modified by CTPs as described in sterile water for injection. In another embodiment, a pharmaceutical composition comprises a lyophilized growth hormone as described in sterile PBS for injection. In another embodiment, a pharmaceutical composition comprises a lyophilized growth hormone as described in sterile 0.9% NaCl for injection.

In another embodiment, the pharmaceutical composition comprising a GH modified by CTPs as described herein is further formulated to comprise complex carriers such as human serum albumin, polyols, sugars, and anionic surface active stabilizing agents. See, for example, WO 89/10756 (Hara et al.- containing polyol and p-hydroxybenzoate). In another embodiment, the pharmaceutical composition comprises a growth hormone as described herein and is further formulated to comprise lactobionic acid and an acetate/glycine buffer. In another embodiment, the pharmaceutical composition comprising a GH modified by CTPs as described herein is further formulated to comprise amino acids, such as arginine or glutamate that increase the solubility of interferon compositions in water. In another embodiment, the pharmaceutical composition comprises a lyophilized GH modified by CTPs as described herein and is further formulated to comprise glycine or human serum albumin (HSA), a buffer (e g. acetate) and an isotonic agent (e.g. NaCl). In another embodiment, the pharmaceutical composition comprises a lyophilized GH modified by CTPs as described herein and is further formulated to comprise a phosphate buffer, glycine and HSA.

In another embodiment, the pharmaceutical composition comprising a GH modified by CTPs as described herein is stabilized when placed in buffered solutions having a pH between about 4 and 7.2. In another embodiment, the pharmaceutical composition comprising a GH modified by CTPs as described herein is stabilized when placed in buffered solutions having a pH between about 6 and 6.4. In another embodiment, the pharmaceutical composition comprising a GH modified by CTPs as described herein is stabilized when placed in buffered solutions having a pH of 6.0. In another embodiment, the pharmaceutical composition comprising a GH modified by CTPs as described herein is stabilized when placed in buffered solutions having a pH of 6.2. In another embodiment, the pharmaceutical composition comprising a GH modified by CTPs as described herein is stabilized when placed in buffered solutions having a pH of 6.4. In another embodiment, the pharmaceutical composition comprising a GH modified by CTPs as described herein is stabilized with an amino acid as a stabilizing agent and in some cases a salt (if the amino acid does not contain a charged side chain). In one embodiment, the pharmaceutical composition is stabilized at room temperature. In another embodiment, the pharmaceutical composition is stabilized at 4°C. In another embodiment, the pharmaceutical composition is stabilized at 5°C. In another embodiment, the pharmaceutical composition is stabilized at - 20°C. In one embodiment, the pharmaceutical composition is stabilized for at least three months. In one embodiment, the pharmaceutical composition is stabilized for at least six months. In one embodiment, the pharmaceutical composition is stabilized for at least one year. In one embodiment, the pharmaceutical composition is stabilized for at least two years.

In another embodiment, the pharmaceutical composition comprising a GH modified by CTPs as described herein is formulated in a liquid composition comprising a stabilizing agent at between about 0.3% and 5% by weight which is an amino acid.

In another embodiment, the pharmaceutical composition comprising a GH modified by CTPs as described herein provides dosing accuracy and product safety. In another embodiment, the pharmaceutical composition comprising a GH modified by CTPs as described herein provides a biologically active, stable liquid formulation for use in injectable applications. In another embodiment, the pharmaceutical composition comprises a non-lyophilized GH modified by CTPs as described herein.

In another embodiment, the pharmaceutical composition comprising a GH modified by CTPs as described herein provides a liquid formulation permitting storage for a long period of time in a liquid state facilitating storage and shipping prior to administration.

In another embodiment, the pharmaceutical composition comprising a GH modified by CTPs as described herein comprises solid lipids as matrix material. In another embodiment, the injectable pharmaceutical composition comprising a GH modified by CTPs as described herein comprises solid lipids as matrix material. In another embodiment, the production of lipid microparticles by spray congealing was described by Speiser (Speiser and al., Pharm. Res. 8 (1991) 47-54) followed by lipid nanopellets for peroral administration (Speiser EP 0167825 (1990)). In another embodiment, lipids, which are used, are well tolerated by the body (e. g. glycerides composed of fatty acids which are present in the emulsions for parenteral nutrition).

In another embodiment, the pharmaceutical composition comprising a GH modified by CTPs as described herein is in the form of liposomes (J. E. Diederichs and al., Pharm./nd. 56 (1994) 267- 275).

In another embodiment, the pharmaceutical composition comprising a GH modified by CTPs as described herein comprises polymeric microparticles. In another embodiment, the injectable pharmaceutical composition comprising a GH modified by CTPs as described herein comprises polymeric microparticles. In another embodiment, the pharmaceutical composition comprising a GH modified by CTPs as described herein comprises nanoparticles. In another embodiment, the pharmaceutical composition comprising a GH modified by CTPs as described herein comprises liposomes. In another embodiment, the pharmaceutical composition comprising a GH modified by CTPs as described herein comprises lipid emulsion. In another embodiment, the pharmaceutical composition comprising a GH modified by CTPs as described herein comprises microspheres. In another embodiment, the pharmaceutical composition comprising a GH modified by CTPs as described herein comprises lipid nanoparticles. In another embodiment, the pharmaceutical composition comprising a GH modified by CTPs as described herein comprises lipid nanoparticles comprising amphiphilic lipids. In another embodiment, the pharmaceutical composition comprising a GH modified by CTPs as described herein comprises lipid nanoparticles comprising a drug, a lipid matrix and a surfactant. In another embodiment, the lipid matrix has a monoglyceride content which is at least 50% w/w.

In one embodiment, compositions for use in the present invention are presented in a pack or dispenser device, such as an FDA approved kit, which contain one or more unit dosage forms containing the active ingredient. In one embodiment, the pack, for example, comprises metal or plastic foil, such as a blister pack. In one embodiment, the pack or dispenser device is accompanied by instructions for administration. In one embodiment, the pack or dispenser is accommodated by a notice associated with the container in a form prescribed by a governmental agency regulating the manufacture, use or sale of pharmaceuticals, which notice is reflective of approval by the agency of the form of the compositions for human administration. Such notice, in one embodiment, is labeling approved by the U.S. Food and Drug Administration for prescription drugs or of an approved product insert.

In one embodiment, it will be appreciated that the GH modified by CTPs for use in the present invention can be provided to the individual with additional active agents to achieve an improved therapeutic effect as compared to treatment with each agent by itself. In another embodiment, measures (e.g., dosing and selection of the complementary agent) are taken to minimize adverse side effects which are associated with combination therapies.

Additional objects, advantages, and novel features of the present invention will become apparent to one ordinarily skilled in the art upon examination of the following examples, which are not intended to be limiting. Additionally, each of the various embodiments and aspects of the present invention as delineated hereinabove and as claimed in the claims section below finds experimental support in the following examples.

### EXAMPLES

Generally, the nomenclature used herein and the laboratory procedures utilized include molecular, biochemical, microbiological and recombinant DNA techniques. Such techniques are thoroughly explained in the literature. See, for example, "Molecular Cloning: A laboratory Manual" Sambrook et al., (1989); "Current Protocols in Molecular Biology" Volumes I-III Ausubel, R. M., ed. (1994); Ausubel et al., "Current Protocols in Molecular Biology", John Wiley and Sons, Baltimore, Maryland (1989); Perbal, "A Practical Guide to Molecular Cloning", John Wiley & Sons, New York (1988); Watson et al., "Recombinant DNA", Scientific American Books, New York; Birren et al. (eds) "Genome Analysis: A Laboratory Manual Series", Vols. 1-4, Cold Spring Harbor Laboratory Press, New York (1998); methodologies as set forth in U.S. Pat. Nos. 4,666,828; 4,683,202; 4,801,531; 5,192,659 and 5,272,057; "Cell Biology: A Laboratory Handbook", Volumes I-III Cellis, J. E., ed. (1994); "Culture of Animal Cells - A Manual of Basic Technique" by Freshney, Wiley-Liss, N. Y. (1994), Third Edition; "Current Protocols in Immunology" Volumes I-III Coligan J. E., ed. (1994); Stites et al. (eds), "Basic and Clinical Immunology" (8th Edition), Appleton & Lange, Norwalk, CT (1994); Mishell and Shiigi (eds), "Selected Methods in Cellular Immunology", W. H. Freeman and Co., New York (1980); available immunoassays are extensively described in the patent and scientific literature, see, for example, U.S. Pat. Nos. 3,791,932; 3,839,153; 3,850,752; 3,850,578; 3,853,987; 3,867,517; 3,879,262; 3,901,654; 3,935,074; 3,984,533; 3,996,345; 4,034,074; 4,098,876; 4,879,219; 5,011,771 and 5,281,521; "Oligonucleotide Synthesis" Gait, M. J., ed. (1984); "Nucleic Acid Hybridization" Hames, B. D., and Higgins S. J., eds. (1985); "Transcription and Translation" Hames, B. D., and Higgins S. J., eds. (1984); "Animal Cell Culture" Freshney, R. I., ed. (1986); "Immobilized Cells and Enzymes" IRL Press, (1986); "A Practical Guide to Molecular Cloning" Perbal, B., (1984) and "Methods in Enzymology" Vol. 1-317, Academic Press; "PCR Protocols: A Guide To Methods And Applications", Academic Press, San Diego, CA (1990); Marshak et al., "Strategies for Protein Purification and Characterization - A Laboratory Course Manual" CSHL Press (1996). Other general references are provided throughout this document.

### EXAMPLE 1

### Generation of hGH constructs

### MATERIALS AND METHODS

Four hGH clones (variants of 20kD protein) were synthesized. Xbal -Not I fragments containing hGH sequences from the four variants were ligated into the eukaryotic expression vector pCI-dhfr previously digested with XbaI-NotI. DNA from the 4 clones (401-0, 1, 2, 3 and 4) was prepared. Another partial hGH clone (1-242 bp) from 22kD protein was also synthesized (0606114). Primers were ordered from Sigma-Genosys. The primer sequences used to generate the hGH-CTP polypeptides for use in the present invention are summarized in Table 1 hereinbelow.

**Table 1**

| Primer number | SEQ ID NO | sequence | Restriction site (underlined in sequence) |
|---|---|---|---|
| 25 | 27 | 5' CTCTAGAGGACATGGCCAC 3' | XbaI |
| 32 ^{R} | 28 | 5' ACAGGGAGGTCTGGGGGTTCTGCA 3' | |
| 33 | 29 | 5' TGCAGAACCCCCAGACCTCCCTGTGC 3' | |
| 4 ^{R} | 30 | 5' CCAAACTCATCAATGTATCTTA 3' | |
| 25 | 31 | 5' CTCTAGAGGACATGGCCAC 3' | XbaI |
| 35 ^{R} | 32 | 5' CGAACTCCTGGTAGGTGTCAAAGGC 3' | |
| 34 | 33 | 5' GCCTTTGACACCTACCAGGAGTTCG 3' | |
| 37 ^{R} | 34 | 5'ACGCGGCCGCATCCAGACCTTCATCACTGAGGC 3' | NotI |
| 39 ^{R} | 35 | 5' GCGGCCGCGGACTCATCAGAAGCCGCAGCTGCCC 3' | |

***Construction of 402-0-p69-1* (*hGH*)** ***SEQ** I**D NO: 36:*** MOD-4020 is the wild type recombinant human growth hormone (without CTP) which was prepared for use as control in the below described experiments.

Three PCR reactions were performed. The first reaction was conducted with primer 25 and primer 32^{R} and plasmid DNA of 0606114 (partial clone of hGH 1-242 bp) as a template; as a result of the PCR amplification, a 245 bp product was formed.

The second reaction was conducted with primer 33 and primer 4^{R} and plasmid DNA of 401-0-p57-2 as a template; as a result of the PCR amplification, a 542 bp product was formed.

The last reaction was conducted with primers 25 and 4^{R} and a mixture of the products of the previous two reactions as a template; as a result of the PCR amplification, a 705 bp product was formed and ligated into the TA cloning vector (Invitrogen, catalog K2000-01). The Xbal -NotI fragment containing hGH-0 sequence was ligated into the eukaryotic expression vector pCI-dhfr. The vector was transfected into DG-44 CHO cells. Cells were grown in protein-free medium.

***Construction of 402-1-p83-5* (*hGH-CTP*) - *SEQ ID NO: 37 and 402-2-p72-3*(*hGH-CTPx2*) - *SEQ ID NO: 38:*** MOD-4021 is a recombinant human growth hormone which was fused to 1 copy of the C-terminal peptide of the beta chain of human Chorionic Gonadotropin (CTP). The CTP cassette of MOD-4021 was attached to the C-terminus (one cassette). MOD-4022 is a recombinant human growth hormone which was fused to 2 copies of the C-terminal peptide of the beta chain of human Chorionic Gonadotropin (CTP). The two CTP cassettes of MOD-4022 were attached to the C-terminus (two cassettes).

Construction of hGH-CTP and hGH-CTP-CTP was performed in the same way as the construction of hGH-0. pCI-dhfr-401-1-p20-1 (hGH*-ctp) and pCI-dhfr-401-2-p21-2 (hGH*-ctp x2) were used as templates in the second PCR reaction.

MOD-4021 and MOD-4022 were expressed in DG-44 CHO cells. Cells were grown in protein-free medium. The molecular weight of MOD-4021 is ∼30.5Kd since hGH has a MW of 22 Kd while each "CTP cassette" contributes 8.5 Kd to the overall molecular weight (see Figure 1). The molecular weight of MOD-4022 is -39 Kd (see Figure 1).

***Construction of 402-3-p81-4* (*CTP-hGH-CTP-CTP*) - *SEQ ID NO: 39 and 402-4-p82-9*(*CTP*hGH-CTP-CTP*) -** ***SEQ ID NO: 40:*** MOD-4023 is a recombinant human growth hormone which was fused to 3 copies of the C-terminal peptide of the beta chain of human Chorionic Gonadotropin (CTP). The three CTP cassettes of MOD-4023 were attached to both N-terminus (one cassette) and the C-terminus (two cassettes). MOD-4024 is a recombinant human growth hormone which is fused to 1 truncated and 2 complete copies of the C-terminal peptide of the beta chain of human Chorionic Gonadotropin (CTP). The truncated CTP cassette of MOD-4024 was attached to the N-terminus and two CTP cassettes were attached to the C-terminus (two cassettes).

Three PCR reactions were performed. The first reaction was conducted with primer 25 and primer 35^{R} and plasmid DNA of p401-3-p12-5 or 401-4-p22-1as a template; as a result of the PCR amplification, a 265 or 220 bp product was formed. The second reaction was conducted with primer 34 and primer 37^{R} and plasmid DNA of TA-hGH-2-q65-1 as a template; as a result of the PCR amplification, a 695 bp product was formed. The last reaction was conducted with primers 25 and 37^{R} and a mixture of the products of the previous two reactions as a template; as a result of the PCR amplification, a 938 or 891bp product was formed and ligated into TA cloning vector (Invitrogen, catalog K2000-01). Xba I -Not I fragment containing hGH sequence was ligated into our eukaryotic expression vector pCI-dhfr. (Figure 3)

MOD-4023 and MOD-4024 were expressed in DG-44 CHO cells. Cells were grown in protein-free medium. The molecular weight of MOD-4023 is ∼47.5Kd (see Figure 1) and the molecular weight of MOD-4024 is ∼43.25Kd (Figure 1).

***Construction of 402-6-p95a-8 (CTP-hGH-CTP)* -** ***SEQ ID NO: 41:*** Construction of hGH-6 was performed in the same way as the construction of hGH-3. pCI-dhfr-402-1-p83-5 (hGH-ctp) was used as a template in the second PCR reaction.

***Construction of 402-5-p96-4 (CTP-hGH)* - *SEQ ID NO: 42*:** PCR reaction was performed using primer 25 and primer 39^{R} and plasmid DNA of pCI-dhfr- ctp-EPO-ctp (402-6-p95a-8) as a template; as a result of the PCR amplification , a 763 bp product was formed and ligated into TA cloning vector (Invitrogen, catalog K2000-01). Xba I -Not I fragment containing ctp-hGH sequence was ligated into our eukaryotic expression vector pCI-dhfr to yield 402-5-p96-4 clone.

### EXAMPLE 2

### In vivo bioactivity tests of hGH-CTP polypeptides

The following experiment was performed in order to test the potential long acting biological activity of hGH-CTP polypeptides in comparison with commercial recombinant human GH and MOD-4020.

### MATERIALS AND METHODS

Female hypophysectomized rats (60 -100 g) received a weekly S.C. injection of 21.7 µg hGH-CTP polypeptides or a once daily 5 µg S.C. injection of control commercial rhGH.

Weight was measured in all animals before treatment, 24 hours following first injection and then every other day until the end of the study on day 21. Each point represents the group's average weight gain percentage ((Weight day 0-weight last day)/Weight day 0). Average weight gain was normalized against once-daily injection of commercial hGH. The treatment schedule is summarized in Table 2.

**Table 2**

| ***No.*** | ***Drug*** | ***N*** | ***Route*** | ***Treatment Schedule*** | ***Equimolar Dose (µg*/*rat)*** | ***Accumulate Dosage (µglrat)*** | ***Dose Vol.*(*ml*)** |
|---|---|---|---|---|---|---|---|
| ***1*** | Vehicle | 7 | s.c. | days 1, 7 and 13; 1/W | NA | NA | 0.25 |
| **2** | Mock | 7 | s.c | days 1, 7 and 13;1/W | NA | NA | 0.25 |
| ***3*** | MOD-4020 SEQ ID NO: 36 | 7 | s.c | days 1, 7 and 13; 1/W | 21.7 | 65 | 0.25 |
| ***4*** | MOD-4021 SEQ ID NO: 37 | 7 | s.c. | days 1, 7 and 13; 1/W | 21.7 | 65 | 0.25 |
| ***5*** | MOD-4022 SEQ ID NO: 38 | 7 | s.c. | days 1, 7 and 13; 1/W | 21.7 | 65 | 0.25 |
| ***6*** | MOD-4023 SEQ ID NO: 39 | 7 | s.c. | days 1, 7 and 13; 1/W | 21.7 | 65 | 0.25 |
| ***7*** | MOD-4024 SEQ ID NO: 40 | 7 | s.c. | days 1, 7 and 13; 1/W | 21.7 | 65 | 0.25 |
| ***8*** | Commercial hGH v.1 | 7 | s.c. | days 1, 7 and 13; 1/W | 21.7 | 65 | 0.25 |
| ***9*** | Commercial hGH v. 1 | 7 | s.c. | days 1-13; d/W | 5 | 65 | 0.25 |

### RESULTS

Results are summarized in Figure 2. These results show that MOD-4023 (SEQ ID NO: 39) and MOD-4024 (SEQ ID NO: 40) induced over 120% weight gain compared to commercial rhGH which induced 100% weight gain.

### CONCLUSION

Three weekly doses (Days of injections ;1, 7, and 13) of 21.7µg of MOD-4023 (SEQ ID NO: 39) and MOD-4024 (SEQ ID NO: 40) induced a 30 % greater weight increase in hypophysectomised rats compared to commercial rhGH injected at the same accumulated dose which was administered once per day at a dose of 5 µg for 13 days.

### EXAMPLE 3

### Pharmacokinetic studies of CTP-modified GH

Single-dose pharmacokinetic studies were conducted in Sprague-Dawley rats. All animal experimentation was conducted in accordance with the Animal Welfare Act, the Guide for the Care and Use of Laboratory Animals, and under the supervision and approval of the Institutional Animal Care and Use Committees of Modigene, Biotechnology General Ltd. Rats were housed either individually or two per cage in rooms with a 12-h light/dark cycle. Access to water (municipal supply) and noncertified rodent chow was provided ad libitum.

To compare the pharmacokinetics of CTP-hGH-CTP-CTP and GH in rats, four groups of Sprague-Dawley rats (270-290 g), three to six male rats per group. The rats were randomly assigned to four treatment groups (see Table 3). Rats were administered a single s.c. or i.v. injection of GH (50 µg/kg i.v. or s.c.) or CTP-hGH-CTP-CTP (108 µg/kg i.v. or s.c.). With the exception of the predose sample, which was collected under isoflurane anesthesia, blood collection was performed in unanesthetized animals. Blood samples (approximately 0.25 ml) were collected in EDTA-coated microtainers for ELISA analyses of CTP-hGH-CTP-CTP plasma concentration at the times outlined in Table 11. After each sampling, the blood volume was replaced with an equal volume of sterile 0.9% saline. Samples were stored on ice for up to 1 h prior to centrifugation and plasma harvest. Plasma samples were stored at approximately -20 °C prior to analysis.

**Table 3. Experimental design of rat pharmacokinetic study**

| Trt. Grp. | Test Article | No. of animals/group/timepoint | Dose Route | Gender | Dose Level (µg/kg) | Injected Vol. (µl) | Concentration (µg/ml)/Total vol. (ml) | Time-Points ^{∗} (hours post-dose) |
|---|---|---|---|---|---|---|---|---|
| 1 | Biotropin | 6# | sc | Male | 50 | 500 | 20/5 | 0 (Pre-dose) 0.5,2, 4, 8, 24, 48 |
| 2 | CTP-hGH-CTP-CTP | 6# | sc | Male | 108 | 500 | 43.2/5 | 0.5, 2, 4, 8, 24, 48, 72, 96 |
| 3 | Biotropin | 6# | IV | Male | 50 | 300 | 20/3 | 0, 0.12, 2, 4, 8, 24 |
| 4 | CTP-hGH-CTP-CTP | 6# | IV | Male | 108 | 300 | 43.2/3 | 0.12, 2, 4, 8, 24, 48,72 |
| | | | | | | | | |
| Volume of blood sample/time point - 500 µl | | | | | | | | Terminal blood samples |

| | | | | | | | | |
|---|---|---|---|---|---|---|---|---|
| # 3 rats per time point. | | | | | | | | |

A commercial sandwich ELISA kit specific for detection of human growth hormone (Roche Diagnostics) was used for evaluation of the rat plasma samples. This kit detects human growth hormone in plasma by means of an antibody sandwich ELISA format. This kit was initially used to measure the concentration of CTP-hGH-CTP-CTP in rat plasma. For these plasma samples, a CTP-hGH-CTP-CTP standard curve (1.2-100 ng/ml) was used and the concentrations of CTP-hGH-CTP-CTP in rat plasma were interpolated from this curve.

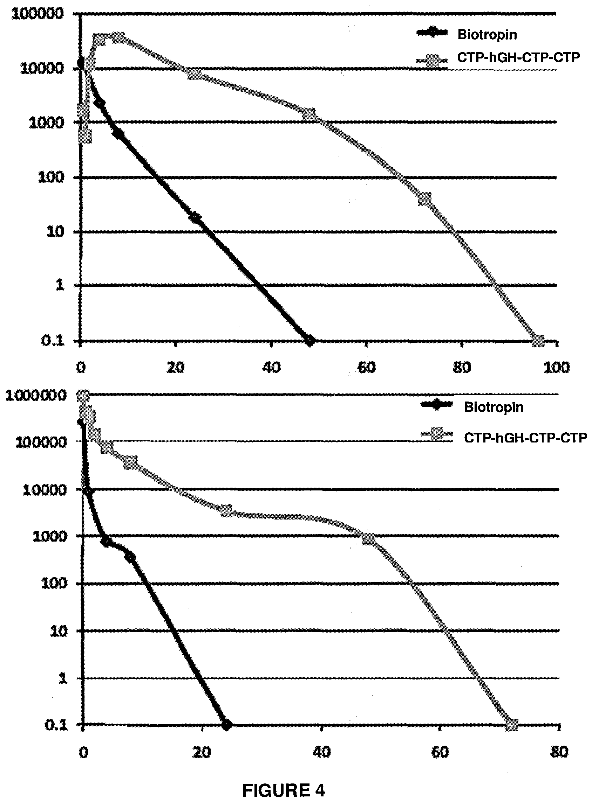

Standard pharmacokinetic parameters, including clearance (CL or CL/F), volume of distribution (Vd or Vd/F), half-life (*t*_{1/2})*,* area under the plasma concentration versus time curve (AUC), maximal observed plasma concentration (Cₘₐₓ) and time to maximal observed plasma concentration (*T*ₘₐₓ), were obtained from plasma albutropin or GH concentration/time curves by noncompartmental analysis using the modeling program WinNonlin (Pharsight, version 3.1). Plasma CTP-hGH-CTP-CTP or GH concentration data were uniformly weighted for this analysis. The AUC was calculated using the log-linear trapezoidal analysis for the i.v. data and the linear-up/log-down trapezoidal method for the s.c. data. Plasma concentration profiles for each rat (with the exception of the s.c. albutropin data) or monkey were analyzed individually, and mean±standard error of the mean (S.E.M.) values for the pharmacokinetic parameters are reported in Table 4 and Figure 4.

CTP-hGH-CTP-CTP is a single chain protein of 275 amino acids and up to twelve O-linked carbohydrates. The structure consists of modified human Growth Hormone (Somatropin) attached to three copies of the C-terminal peptide (CTP) of the beta chain of human Chorionic Gonadotropin (hCG); one copy at the N-terminus and two copies (in tandem) at the C terminus. Human Growth Hormone is comprised of 191 amino acids. CTP is comprised of 28 amino acids and four O-linked sugar chains.

### EXAMPLE 4

### Pharmacokinetics of CTP-modified GH in SD rats

The pharmacokinetics of CTP-hGH-CTP-CTP was evaluated and compared to that of commercial hGH (Biotropin).

Table 4. Mean pharmacokinetic parameters following single-dose i.v. and s.c. administration of CTP-hGH-CTP-CTP and GH (Biotropin) in Sprague-Dawley rats.

| **PK Statistics** | | | | | |
|---|---|---|---|---|---|
| | | **SC** | | **IV** | |
| **Parameters** | **Units** | **Biotropin** | **CTP-hGH-CTP-CTP** | **Biotropin** | **CTP-hGH-CTP-CTP** |
| Dose | mg/Kg | 50 | 50 | 50 | 50 |
| AUClast | hr^{∗}ng/mL | 41 | 680 | 162.7 | 1568.3 |
| Cmax | ng/ml | 13 | 36.8 | 275.8 | 926 |
| Tmax | hr | 0.5 | 8 | 0 | 0 |
| MRT | hr | 2.5 | 12.9 | 0.5 | 9.9 |
| T1/2 **alpha** | hr | | 1.58 | | 0.74 |
| T1/2 **beta** | hr | 1.73 | 9 | 0.5 | 6.9 |

### Data statistical analysis

Analysis of serum samples was performed in order to determine specific concentration levels for each sample. Concentration and time-point data were processed using WinNonLin noncompartmental analysis.

Parameters that were determined included: AUC, MRT, t1/2, Cmax, and Tmax. Figure 4 demonstrates the superior pharmacokinetic profile of CTP-hGH-CTP-CTP plasma concentration compared to GH concentrations (pg/ml) following a single i.v. or s.c. dose of CTP-hGH-CTP-CTP or GH in rats (n=3-6 per dose/route).

Following a single S.C. injection of 50 µg/kg, clearance of CTP-hGH-CTP-CTP from SD rat's blood was significantly slower than that of CTP-hGH-CTP and of Biotropin. The corresponding calculated half-life times and AUCs were:

| | |
|---|---|
| Biotropin | T1/2 1.7h, AUC 41 hr^{∗}ng/mL |
| CTP-hGH-CTP | T1/2 8.5h, AUC 424 hr^{∗}ng/mL |
| CTP-hGH-CTP-CTP | T1/2 9.0h, AUC 680 hr^{∗}ng/mL |

*Conclusion:* CTP-hGH-CTP-CTP was chosen as the final candidate out of 6 other variants. CTP-hGH-CTP-CTP demonstrated superior performance in terms of biological activity and pharmacokinetics.

### EXAMPLE 5

### Weight Gain Assay (WGA ) for Single dose/Repeated dose of CTP-modified GH

Hypophysectomized (interaural method) male rats, 3-4 weeks of age, were obtained from CRL Laboratories. During a post-surgical acclimation period of 3 weeks, rats were examined and weighed twice weekly to eliminate animals deemed to have incomplete hypophysectomy evidenced by weight gain similar to that of sham-operated rats. Those rats with incomplete hypophysectomized were eliminated from the study. The average body weights of the hypophysectomized were 70-90 grams, at the time of the experiment. This is the standard USP and EP bioassay for hGH. Hypophysectomized rats (rats from which the pituitary gland was removed) lose their ability to gain weight. Injections of hGH (and of CTP-hGH-CTP-CTP) to these rats result in weight gain. Based on the measured weight gain along a defined period of time and the amount of hGH injected, the specific activity of hGH (and CTP-hGH-CTP-CTP) is determined. Rats were administered either a single s.c. doses of 0.4, 0.8 and 4 mg/Kg or repeated s.c. doses of 0.6 and 1.8 mg/Kg 4 days apart for 3 weeks. Individual body weights of all animals are determined at randomization, prior to the first dosing, thereafter every two days or in case of decedents at the time of death, and prior to sacrifice.

### Single dose and repeated dose weight gain assay

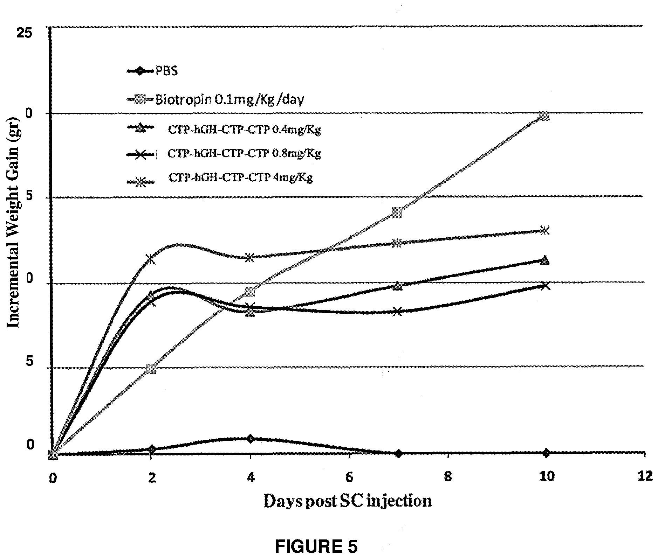

The results comparing whole body growth response following different dosing patterns of CTP-hGH-CTP-CTP in hypophysectomized rats are demonstrated in Figure 5. The results demonstrate that a single injection of 0.4 & 0.8 mg/Kg/day doses of hGH-CTP were equivalent to 4 daily injections of 0.1 mg/Kg/day of Biotropin. The peak of the hGH-CTP effect was after 2 days.

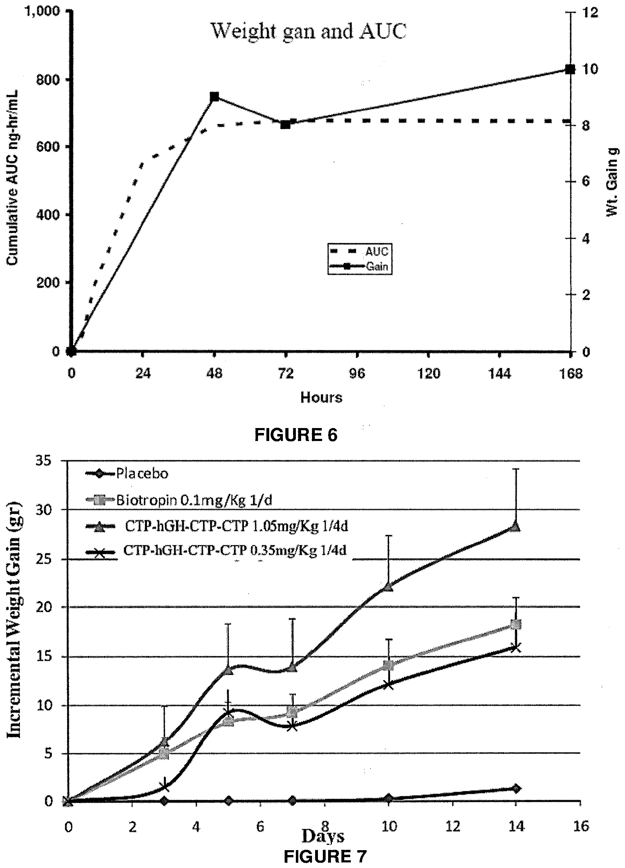

Figure 6 further demonstrates that the area under the curve following single injection of CTP-hGH-CTP-CTP correlates with Body Weight gain in Rats. Thus, the collective data demonstrates that body weight gain is closely correlated with cumulative AUC.

The hGH-CTP construct administered 4 days apart promotes the same weight gain as daily injections of Biotropin as demonstrated in Figure 7. Half-life of hGH in humans is expected to be 5x better than in rats - indicating potential peak effect in humans after 10 days for one single injection. These results support administration of hGH-CTP construct, CTP-hGH-CTP-CTP, once weekly or bi-weekly in humans.

### EXAMPLE 6

### Pharmacodynamics/Pharmacokinetics studies of CTP-modified GH

Hypophysectomized (interaural method) male rats, 3-4 weeks of age, were obtained from CRL Laboratories. During a post-surgical acclimation period of 3 weeks, rats were examined and weighed twice weekly to eliminate animals deemed to have incomplete hypophysectomy evidenced by weight gain similar to that of sham-operated rats. Those rats with incomplete hypophysectomized were eliminated from the study. The average body weights of the hypophysectomized and sham rats were 70 and 150 g, respectively, at the time of the experiment.

Rats were administered a single s.c. with CTP-hGH-CTP-CTP, vehicle, human growth hormone CTP-hGH-CTP-CTP or human growth hormone (20 µg/rat) was administered s.c. in an injection volume of 0.2 ml/rat. The dose of GH was 0.35 and 1.05 µg/Kg, a dose of growth hormone that was equimolar with the amount of GH in a corresponding 0.6 and 1.8 µg/Kg dose of CTP-hGH-CTP-CTP. The treatment groups are summarized in Table 5. Following injection, plasma samples for IGF-1 analyses were obtained at the times described in Table 5. Samples were analyzed for IGF-1 concentration using a commercial ELISA (R&D systems).

**Table 5. Treatment schedule for hypophysectomized rat study**

| Trt. Grp. | Test Article | No. of animals/ group/ timepoint | Dose Route | Eq. Dose (mg/rat) | Eq. Dosage (mg/Kg) | CTP-hGH-CTP-CTP Conc. mg/ml | **Dose Vol. (ml)** | Time-Points * (hours post-dose) |
|---|---|---|---|---|---|---|---|---|
| M7 | Biotropin | 9 | SC | 0.032 | 0.35 | **0.16** | 0.2 | 0 (Pre-dose) 0.5,2, 4, 8, 24, 48, 72, 96 |
| M8 | Biotropin | 9 | sc | 0.095 | 1.05 | **0.475** | 0.2 | 0 (Pre-dose) 0.5,2, 4, 8, 24, 48, 72, 96 |
| M9 | EN648-01-08-005 | 12 | sc | 0.032 (0.055) | 0.35 (0.6) | **0.275** | 0.2 | 1, 2, 4, 8, 24, 48, 72, 96 |
| M10 | EN648-01-08-005 | 12 | sc | 0.095 (0.165) | 1.05 (1.8) | **0.825** | 0.2 | 1,2,4,8, 24, 48, 72, 96 |
| Volume of blood sample/time point - 500 µl | | | | | | | | Terminal blood samples |

Non-compartmental pharmacokinetic analysis was performed on the mean serum concentration versus time curves for each group. CTP-hGH-CTP-CTP Cmax was significantly higher than Biotropin Cmax. The terminal half-live of CTP-hGH-CTP-CTP was 6 times higher than Biotropin.

**Table 6: Pharmacokinetic Parameter Estimates of CTP-hGH-CTP-CTP and Biotropin Following a Single Subcutaneous Injection in hypophysectomized Rats**

| **Group** | **Dose** | **Gender** | **Cmax** | **Tmax** | **AUC_{0-∞}** | **AUC₀₋ₜ** | **CL/F** | **T_{1/2}** |
|---|---|---|---|---|---|---|---|---|
| | **mg/kg** | | **ng/mL** | **hr** | **ng-hr/mL** | **ng-hr/mL** | **mL/hr/kg** | **hr** |
| CTP-hGH-CTP-CTP | 1.8 | M | 2,150 | 8 | 37,713 | 37,695 | 0.928 | 6.86 |
| | 0.6 | M | 681 | 8 | 11,505 | 11,489 | 3.042 | 6.8 |
| hGH | 1.05 | M | 1,078 | 0.5 | 3,541 | 3,540 | 9.884 | 1 |
| | 0.35 | M | 439 | 0.5 | 1,279 | 1,279 | 27.36 | 1 |

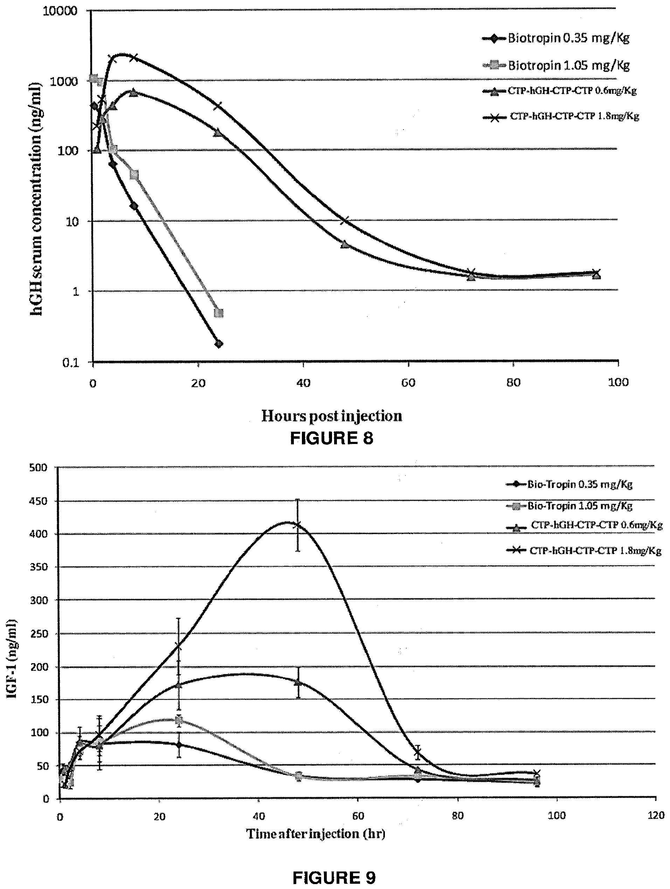

The AUCo-t and the AUC_{0-∞} were very similar suggesting the duration of sampling was adequate to characterize the pharmacokinetic profiles. AUC of CTP-hGH-CTP-CTP was 10 times higher than of Biotropin. Moreover, Cmax was generally proportional to dose and for CTP-hGH-CTP-CTP and it was twice higher than Cmax of Biotropin. However, as shown in Figure 8, Tmax of CTP-hGH-CTP-CTP was 8 hr as compare to 0.5 hr of Biotropin, and the terminal half-lives did not appear to vary with dose level. T1/2 of CTP-hGH-CTP-CTP was 6.8 times longer than of Biotropin.

Indirect effects of GH are mediated primarily by an insulin-like growth factor-I (IGF-1), a hormone that is secreted from the liver and other tissues in response to growth hormone. A majority of the growth promoting effects of growth hormone is actually due to IGF-1 acting on its target cells. Accordingly, the effect of the CTP-hGH construct, CTP-hGH-CTP-CTP, on IGF-1 serum levels in Hypophysectimized Rats was measured. Figure 9 presents results of IGF-1 serum levels in Hypophysectimized Rats Following SC injection of CTP-hGH-CTP-CTP and commercial hGH.

Single dose of CTP-hGH-CTP-CTP 0.6 or 1.8 mg/Kg and Biotropin 0.35 or 1.05 mg/Kg were injected subcutaneously to hypophysectomised rats for determination of PK/PD profile. Serum IGF-1 post injection was measured using specific ELISA kits (Roche Diagnostics).

The cumulative serum levels of IGF-1 following injection of CTP-hGH-CTP-CTP was significantly higher than following injection of Biotropin. Cmax was generally proportional to dose and for CTP-hGH-CTP-CTP it was 3-4 times higher than Cmax of Biotropin. Tmax of CTP-hGH-CTP-CTP was 36-48 hr as compare to 20-24 hr of Biotropin. In conclusion, higher hGH levels and longer presence in serum result in significant increase in IGF-1 levels.

### EXAMPLE 7

### Carbohydrate Content and Sialic Acid Content of CTP-modified GH

Analysis of O-glycans is based on a Prozyme kit. O-glycans are chemically and enzymatically cleaved from the protein and separated from peptides using paper chromatography. Sequencing of the O-glycan pool is performed by sequential enzymatic digestions (exo-glycosidases) followed by HPLC analysis compared to standards.

### Glycoprofiling with Sequence Analysis

Glycoprofiling was performed by Ludger Ltd. Two samples (EN648 and RS0708) were taken through triplicate releases and each release was also analyzed by HPLC in triplicate. Triplicate 300µg samples of EN648 and RS0708 and a single 100µl sample of citrate/sodium chloride buffer, plus a positive control fetuin (250µg) and a 100µl water negative control, were ultra-filtrated by centrifugation using a molecular weight cut off membrane of 10,000Da to replace the buffer with water, then taken through hydrazinolysis under O-mode conditions (6 h at 60°C). Released glycans were re-*N*-acetylated and cleaned up by LudgerClean CEX cartridges. An aliquot of the released glycans was then labeled with 2-aminobenzamide (2AB), cleaned up with Ludger Clean S cartridges and analyzed by LudgerSep-N2 HILIC-HPLC.

### Monosaccharide Content

Analysis of neutral monosaccharides requires hydrolysis of glycans to their constituent monosaccharide components. The hydrolysis was performed by Ludger Ltd, on intact glycoprotein samples. Specifically, 50µg of intact glycoprotein was acid hydrolyzed, 2-AB (2-aminobenzamide) labeled and run on a reverse phase HPLC column. This method hydrolyzes all glycans present on the glycoprotein inclusive of N and O linked types.

### Sialic Acid Profiling

Two samples (EN648 and RS0708) and a buffer control were analyzed. Sialic acid analysis requires mild acid release of the monosaccharides followed by DMB fluorophore labeling and HPLC analysis on a LudgerSep-R1 column. 50µg of intact glycoprotein was acid hydrolyzed for each analysis.

### Glyco analysis of CTP-hGH-CTP-CTP

**Table 7. Glycan analysis. Structural assignments and percentage areas of peaks are based upon HPLC and enzyme array digests.**

| **Peak ID^{a}** | **GU^{b}** | **Structure^{c}** | **name** | **Percent from total glycans^{e}** | | | |
|---|---|---|---|---|---|---|---|
| | | | | **Und**^{d} | **NAN1** | **ABS** | **ABS BTG** |
| 1^{f} | 0.92 | 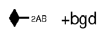 | GalNAc | 0.4 | 0.4 | 0.6 | 53.0 |
| 2^{f} | 1.02 | 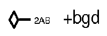 | galactose | 1.9 | 9.7 | 23.8 | 26.5 |
| * | 1.72 | | | 4.3 | 4.6 | 2.3 | |
| 3 | 1.79 | 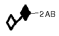 | Galβ1-3GalNAc | 2.3 | 67.7 | 69.4 | 17.1 ^{h} |
| 4^{g} | 2.25 | 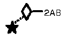 | NeuNAcα2-3Gal | 19.8 | 13.0 ^{h} | | |
| * | 2.57 | | | 1.5 | 1.9 | 1.1 | 1.1 |
| 5 | 2.90 | 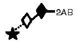 | NeuNAcα2-3Galβ1-3 GalNAc | 70.6 | | | |
| * | 3.58 | | | 0.6 | 0.7 | 0.6 | |
| 6 | 3.22 | 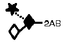 | Galβ1-3[NeuNAcα2-6] GalNAc | 0.9 | 2.3 | | |
| 7 | 4.42 | 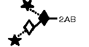 | NeuNAcα2-3Galβ1-3 [NeuNAcα2-6]GalNAc | 1.8 | | | |

The monosaccharide profiles indicate that the CTP-hGH-CTP-CTP glycoprotein samples contain predominantly O-link type glycans. The major O-glycan peak is sialylated core 1 (Neu5Acα2-3Galβ1-3GalNAc). The major sialic acid is Neu5Ac and there are some minor peaks suggesting the presence of 3-4% of di-acetylated sialic acid *N*-acetyl-9-O-acetylneuraminic acid (Neu5, 9Ac2) and less than 1% N-glycolylneuraminic acid. There are also small amounts of Neu5Acα2-6(Galβ1-3)GalNAc.

### EXAMPLE 8

### PharmacokineticlToxicokinetic Analysis of CTP-modified GH in Rhesus monkeys

Serum concentrations versus time curves were generated for each animal. Non-compartmental analysis was performed with WinNonlin professional version 5.2.1 (Pharsight Corporation, Mt View CA.). The estimated pharmacokinetic parameters are shown in Table 8 below:

**Table 8: Estimates of CTP-hGH-CTP-CTP Pharmacokinetic Parameters (Mean ± SD) from Non-compartmental Analysis Following A Single Subcutaneous Injection in Rhesus Monkeys**

| **Parameter** | **1.8 mg/kg** | **90 mg/kg** |
|---|---|---|
| | | |
| Cmax (µg/mL) | 2.073 ± 0.417 | 108.7 ± 46.0 |
| Tmax (hr) | 4 ± 0 | 11 ± 7 |
| AUC₀₋ₜ (µg-hr/mL) | 38.7 ± 7.4 | 2,444 ± 394 |
| AUC_{0-∞} (µg-hr/mL) | 39.0 ± 7.3 | 2,472 ± 388 |
| CL/F (mL/hr/kg) | 47.5 ± 9.0 | 37.04 ± 4.78 |
| T_{1/2} (hr) | 10.00 ± 1.47 | 9.85 ± 1.07 |
| Vz/F (mL/kg) | 701 ± 236 | 529 ± 104 |

The AUCo-t and the AUC_{0-∞} were very similar suggesting the duration of sampling was adequate to characterize the pharmacokinetic profiles. Cmax was proportional to dose. Tmax was later at the higher dose. Tmax was at 4 hours for all animals in the low dose group and was at 8 or 24 hours in the high dose group. Terminal half-lives are similar for the two dose groups.

AUC was approximately proportional to dose with a slightly larger than proportional AUC at the higher dose producing a slightly lower estimate for CL/F and Vz/F compared to the lower dose. It is not possible to say if CL and Vz are lower at the higher dose or if F is lower at the lower dose. There was overlap between the groups so it is questionable that this represents a meaningful difference in CL/F and Vz/F.

Pharmacokinetic parameters estimated by the model were very similar to those from non-compartment analysis. Absorption and elimination half-lives are shown in Table 9 below:

**Table 9: Estimates of CTP-hGH-CTP-CTP Absorption and Elimination Half-lives (Mean ± SD) Following Subcutaneous Injection Derived From Pharmacokinetic Modeling in Rhesus Monkeys**

| **Dose** | **T_{1/2 abs} (hr)** | **T_{1/2 el} (hr)** |
|---|---|---|
| 1.8 mg/kg | 1.17 ± 0.40 | 10.41 ± 2.36 |
| 90 mg/kg | 6.49 ± 1.87 | 7.26 ± 1.85 |

The data indicate that the elimination rates are fairly similar between the groups with a slightly longer T1/2 el in the lower dose group. The absorption, however, is more than 5-fold slower following subcutaneous administration of 90 mg/kg compared to that following 1.8 mg/kg. As in the case of the non-compartmental analysis, modeling indicated a later Tmax at the high dose.

Although GH supplementation is effective in the treatment of GH deficiency in children and adults, the disadvantages of daily injections over extended periods of time limit its use by physicians in certain patient populations as well as increase the risk of dosing error, the number of care givers, the cost of treatment and/noncompliance. Especially important in certain populations, such as children of short stature who may not understand the implications of not following the prescribed GH dosing regimen, is the necessity of compliance to achieve the optimal benefit from GH therapy. The issue of finding a more suitable alternative to daily GH injections and subsequent compliance gains further importance as GH-deficient children transition into adults with a continuing need for GH treatment. The requirement of daily therapy is largely due to recombinant GH's short plasma half-life and has led to the development of a sustained release form of GH (Reiter EO. Attire KM., Mashing TJ. Silverman BL. Kemp SF. Neolith RB. Ford KM. and Sanger P. A multimember study of the efficacy and safety of sustained release GH in the treatment of naive pediatric patients with GH deficiency. J. Clin. Endocrinol. Metab. 86 (2001), pp. 4700-4706.).

GH-CTP, a recombinant human growth hormone-CTP fusion protein, as described herein, has a pharmacokinetic profile in the rat that is longer in duration than that of GH. This unique pharmacokinetic profile allows for intermittent administration of GH-CTP to achieve pharmacodynamic effects in growth-hormone-deficient rat as evidenced by growth and elevations in plasma IGF-1 levels, respectively.

GH-CTP offers a superior pharmacokinetic profile compared with that of GH when administered s.c. in the rat. There are substantial differences in plasma clearance of GH-CTP compared to GH. Specifically, plasma is cleared of GH-CTP at more than 6 times more slowly than GH following s.c. dosing. The terminal half-life and mean residence time of GH-CTP were approximately six times longer than that of GH in rats following s.c. administration. In addition, the Cl/F following s.c. dosing is 10 times lower for GH-CTP than for GH.

In an effort to examine whether the pharmacokinetic advantages in the rat translated to a pharmacodynamic benefit, the possibility that GH-CTP might stimulate growth in GH-deficient hypophysectomized rats with dosing regimens less frequent than daily was tested at equimolar CTP-hGH-CTP-CTP and GH dose levels. Single SC injection of GH-CTP promoted incremental weight gain which was equal to 4 daily consecutive injections of GH. In addition, the every fourth day administration schedule for GH-CTP shows enhanced body weight gain over GH.

Pharmacodynamically, the long circulation time of GH-CTP relative to GH in the hypophysectomized rats resulted in a prolonged IGF-1 response measured in blood plasma following a single s.c. injection. Subcutaneous administration of a single dose of GH-CTP increased circulating IGF-1 concentrations in a dose-dependent manner in the hypophysectomized rats. At the highest albutropin dose, IGF-1 concentrations were elevated above baseline for as long as 75 hours after a single administration. Thus, the enhanced circulation time of a single dose of GH-CTP resulted in substantial pharmacodynamic improvement over a single dose of GH, raising the possibility that GH-CTP could offer similar growth enhancement with reduced dosing frequency compared with standard GH treatment regimens.

Single CTPs modified hGH- dose of 90 mg/kg in Rhesus and 180mg/kg in rats were well tolerated in both species. The allometric factor between rats and primates is approximately X2 which is based on the anticipated clearance of proteins in these animals. In-line with industry-accepted extrapolation models for therapeutic proteins' half-life increase between species (FDA Guidance). 90 mg/kg in Primates has a PK profile slightly better than 180mg/kg of CTPs modified hGH in Rat. Thus, allometric extrapolation to humans supports weekly or once/2w injection.

The present concept utilizing a CTP-GH construct, reduced dosing frequency compared to the commercial GH recombinant product. Nutropin Depot® is a sustained release formulation of GH approved for use in pediatric populations; however, comparisons to historical controls have revealed that 1- and 2-year growth rates are significantly (p<0.001) lower in children given Nutropin Depot® (1-year growth rate 8.2±1.8 cm/year) than in children treated with GH (one-year growth rate 10.1±2.8 cm/year) (Silverman BL, et al. J. Pediatr. Endocrinol. Metab. 15 (2002), pp. 715-722.). The local effects of subcutaneously administered Nutropin Depot® include nodules, erythema, pain at the injection site, headache and vomiting. Preclinical toxicology studies in both rat and monkey have shown that s.c. administration of CTP-hGH-CTP-CTP produces no local reactions compared to vehicle. Given the medical need for a less frequently administered form of GH, the pharmacologic properties of CTP-hGH-CTP-CTP in this study in rats suggest that this product is favorable also in terms of toxicology and patient compliance. The sustained activity of CTP-hGH-CTP-CTP in the rat support its potential utility as an agent that requires only intermittent administration to attain a therapeutic benefit that is currently achieved with daily dosing.

### EXAMPLE 9

### Long-acting CTP-modified version of human growth hormone (hGH-CTP) was highly effective in growth hormone deficient adults - Phase II Clinical Trial

A randomized, open-label, Phase II Clinical Trial was conducted to evaluate the safety, tolerability, pharmacokinetics and pharmacodynamic properties of hGH-CTP injected either weekly or twice-monthly in patients who currently receive daily injections of growth hormone. The trial was conducted at multiple sites in six countries. The three main cohorts in the trial received a single weekly dose of hGH-CTP, containing 30%, 45% or 100% of the equivalent cumulative commercial hGH dose that growth hormone-deficient adult patients receive over the course of seven days in the form of daily injections (referred to as the "30%, "45%" and "100%" cohorts, respectively). The data reflect results from 39 patients, 13 in each cohort. 2 females were included in each cohort.

In addition to the three main cohorts, growth hormone deficient adults were enrolled in an experimental fourth cohort, which is conducted outside of the formal Phase II trial. The patients in the experimental fourth cohort receive a single injection of hGH-CTP once every two weeks that contains 50% of the cumulative commercial dose of that growth hormone-deficient adult patients receive over a two-week period in the form of daily injections.

Efficacy for the three main cohorts receiving a single weekly injection of hGH-CTP is defined by measuring daily insulin-like growth factor 1 (IGF-1) levels within the desired therapeutic range over a period of seven days (during the last week of treatment in the study). The desired therapeutic range is defined as between +2 standard deviations through - 2 standard deviations from the average IGF-1 levels expected in a normal population, stratified by age group and gender. In addition, the trial measured IGF-1 levels within a narrower range of +/-1.5 standard deviations for the purpose of observing the variance of the patients within the normal range.

### Results:

Table 10 contains the average percent of days within the normal therapeutic range (+/- 2 SD), average percent of days within a narrower normal therapeutic range (+/- 1.5 SD), and average Cmax (highest concentration level) of IGF-1 for males, measured during the last treatment week, expressed in standard deviations from the normal population mean IGF-1 levels.

**Table 10: Human Phase II Clinical Trial Results.**

| Cohort | % Days Within Narrow Normal Range of IGF-1 (+/- 1.5 SD) | % Days Within Normal Range of IGF-1 (+/- 2 SD) | Avg. Cmax of IGF-1 (preferred below +2 SD) |
|---|---|---|---|
| 30% | 57% | 100% | -0.9 |
| 45% | 100% | 100% | 0.1 |
| 100% | 86% | 100% | 0.4 |

Two mg per week of hGH-CTP, containing 50% of the cumulative weekly hGH dose that an adult patient would usually be prescribed as the initial treatment dose, has a high likelihood of being defined as the starting dose for males and females in the adult Phase III.

There was no evidence of safety and/or tolerability issues, and no indication that hGH-CTP, when used in high doses, induced excessive levels of IGF-1 in patients or even levels above the normal range.

### Phase II -IGF-1 Summary and Perspectives

### MOD-4023 Phase II study design and objectives:

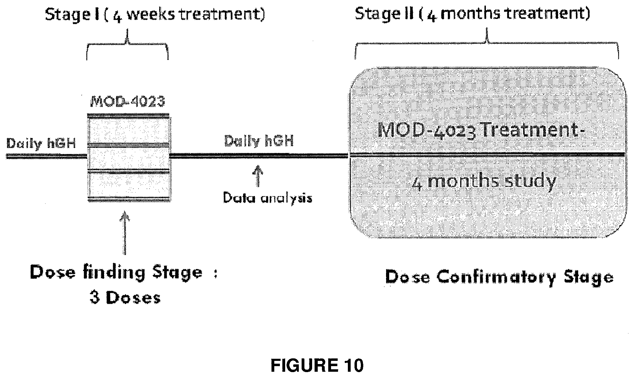

A two stage Phase II study confirming CTP-hGH-CTP-CTP (MOD-4023) weekly administration regimen was completed (see Fig 10). The trial was a switch over study performed in growth hormone deficient (GHD) patients currently on a daily hGH treatment that were considered normalized on their daily treatment prior to MOD-4023 administration, as reflected by IGF-1 SDS levels within the normal range (±2SDS). Stage I of the study was a 4 week dose-finding study (4 injections) supported by a full pharmacokinetics-pharmacodynamics (PK-PD) analysis during the week following the 4^{th} dose of MOD-4023. The major objective of this part was to identify a therapeutic dose range in which the IGF-1 level is kept within a defined range. Another objective was to evaluate the PK-PD profile of MOD-4023 at 3 different doses/multipliers, and confirming a dose-dependent response. The second stage of the study (Stage II) was a 16-week MOD-4023 treatment and dose titration period. All patients who continued to Stage II began with the same MOD-4023 dose level (61.7% of their personal, optimized, weekly cumulative r-hGH dose), but could have their dose modified based on their monitored IGF-1 levels.

In the first part of the study the doses were administered based on percentage of the weekly accumulated hGH in order to evaluate the initial response following a weekly regimen of MOD-4023. For example: A patient receiving 1mg / day of hGH who was randomized to the 55% cohort was injected with a MOD-4023 dose of 1_{mg}*7_{days}*0_{.}55 on weekly basis.

### Results

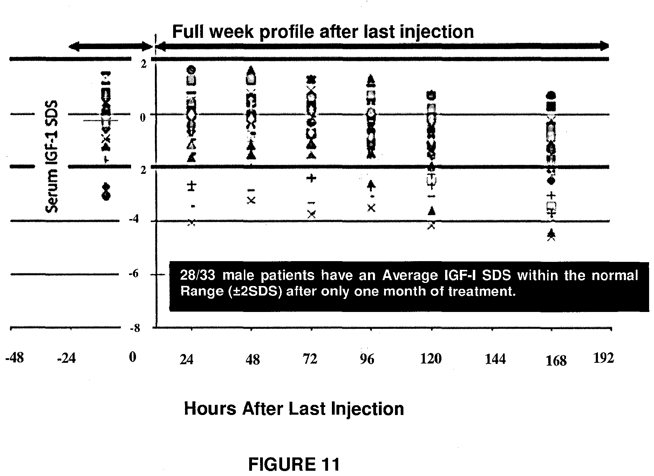

The primary efficacy endpoint of this study was the mean time interval of IGF-1 levels that lay within normal range after the last dose administration during Stage I, expressed in hours. In the final analysis the IGF-1 levels of most of the patients during that week were within the normal range for the entire week (Table 11). Patients who were within the specified SDS range at the final time point were assigned a time interval of 168 hours. None of the patients exceeded +2 SDS at Cmax, indicating that there are no excessive IGF-1 levels. Eighty-five percent of males (28/33 males) had an average IGF-1 SDS within the normal range (±2 SDS) (Figure 11). The mean time interval of IGF-1 levels that lay within ± the normal range of all three cohorts did not show a significant change as all mean time intervals were within 1 standard deviation of one another.

**Table 11: Time interval of IGF-1 that lay within ±2SDS after the 4th dose administration during Stage I**

| | | |
|---|---|---|
| Cohort 2 37% of weekly hGH | Cohort 3 55% of weekly hGH | Cohort 1a 123.4% of weekly hGH |
| **156.37hr** 120.9±66.24 (hr) | **168hr** 146.49±50.62(hr) | **151.17hr** 119.9±66.51(hr) |

As anticipated, administration of 37% of the weekly hGH led to IGF-1 SDS values at the lower part of the normal range and shorter duration within the normal range. A significant improvement in IGF-1 levels as reflected by duration within the normal range was observed when higher dose (55% of the weekly accumulated hGH dose) was administered.

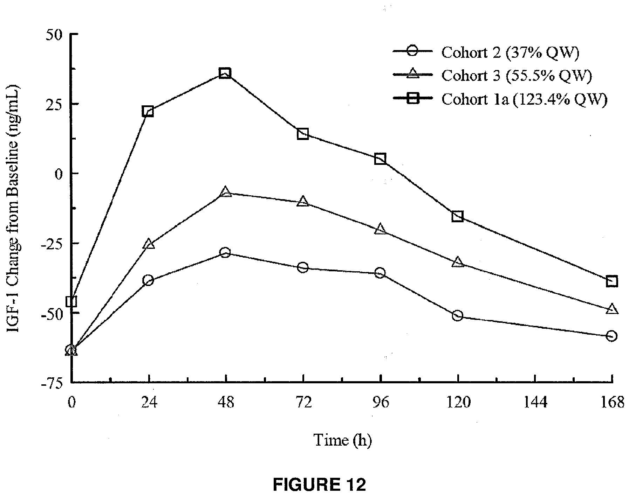

A dose dependent IGF-1 response as compared to baseline is shown in Figure 12. The results presented in Fig. 12, support the notion that IGF-1 levels increase in a MOD-4023 dose dependent manner enabling the adjustment of the IGF-1 weekly profile. Additionally, the mean change from baseline of IGF-1 values 120-168 hr post dosing returns to baseline values, suggesting that IGF-1 trough levels are stable with no deterioration in this normalized growth hormone deficient adults (GHDA) population (Fig. 12).

The Cavg (AUC /Time) which represents the mean IGF-1 exposure, of the daily treated normalized patients was compared to that of the weekly MOD-4023 treatment of the same patients (AUC 0-24/24hr vs. AUC 0-168/168hr respectively). Weekly doses of 55-123.4% of the cumulative weekly hGH dose provided comparable IGF-1 exposure as reflected by Cavg, while for patients treated with 37% of the weekly dose a reduced Cavg was observed, which was aligned with our expectations due to the relatively low weekly dose (Tables 12 &13).

**Table 12: Summary of pharmacodynamic parameters for IGF-1 after subcutaneous administration of r-hGH to growth hormone deficient adults prior to MOD-4023 administration (Stage 1; 4w)**

| **Parameter** | **Cohort* (optimized hGH treatment)** | | |
|---|---|---|---|
| | **2** | **3** | **1a** |
| C_{avg} (ng/mL) | 174 ± 57.0 (11) | 178 ±43.1 (11) | 154 ± 28.5 (11) |

| | | | |
|---|---|---|---|
| *Mean ± standard deviation (N). | | | |

**Table 13: Summary of pharmacodynamic parameters for IGF-1 after subcutaneous administration of MOD-4023 to growth hormone deficient adults (Stage I; 4w)**

| **Parameter** | **Cohort* (MOD-4023 treatment)** | | |
|---|---|---|---|
| | **2 (37%)** | **3 (55.5%)** | **1a (123.4%)** |
| C_{avg} (ng/mL) | 117 ±32.5 (11) | 147 ± 50.9 (11) | 50.4 132 (11) |

Based on the PD analysis of Phase II Stage I the following was concluded: 1) Although the study objective was not to optimize patients IGF-1 levels namely, targeting IGF-1 SDS value to 0, (since IGF-1 SDS optimization requires relatively long titration period), still therapeutic dose range for weekly administration of MOD-4023 could be established: around 56%-123% of the weekly cumulative dose of daily hGH. 2) IGF-1 profile following a weekly MOD-4023 administration is relatively flat, as reflected by fairly small difference between Cmax and Ctrough. 3) The Cavg (AUC 0-168/168hr) which represents the mean weekly IGF-1 exposure correlated to Day 4 values. Therefore, day 4 post MOD-4023 administration was chosen as the monitoring day for IGF-1 levels.

### Phase II Stage II (4 months extension) results and perspectives:

The ability of weekly administration of MOD-4023 to maintain IGF-1 within the normal range at an optimal dose and for a longer period of time was addressed during the second part of the study (Stage II- 4 months extension period; Figure 10). In this study, the same patient population from the first stage was administered with 61.7% of their hGH weekly dose and IGF-1 was monitored every two weeks. The majority of the patients maintain the IGF-1 SDS value within the normal range throughout the study as measured on day 4 post injection. Patients who demonstrated IGF-1 levels below the normal range were further titrated and their MOD-4023 dose was increased (aligned with the clinical practice).

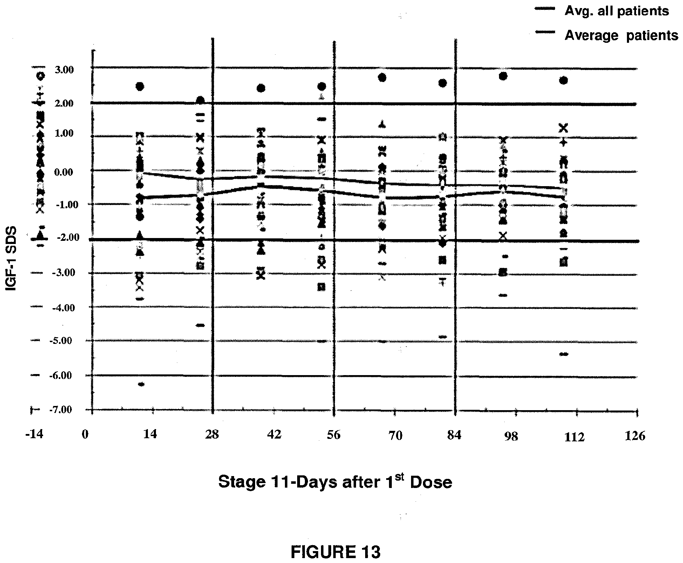

Minority of patients with IGF-1 SDS values below the normal range required further titration but demonstrated remarkable improvement in IGF-1 SDS, indicating that IGF-1 profile can be optimized by MOD-4023 dose increment / decrement. Excellent responsiveness and minimal dose modification were needed as presented in Figure 13 and summarized in Table 14 hereunder.

**Table 14: Summary of required dose modifications during Stage II.**

| **Number of Dose Modifications** | **Males** | **Females** |
|---|---|---|
| No dose modifications | 22 (out of 34) | 3 (of 8) |
| 1 dose modification | 6 (out of 34) | 1 (out of 8) |
| 2 dose modifications | 3 (out of 34) | 3 (out of 8) |
| 3 dose modifications | 3 (out of 34) | 1 (out of 8) |

Based on day 4 IGF-1 SDS values (correlated to Cavg), a significant improvement in IGF-1 levels, as compared to Stage I of the study was observed for the individual patients. This observation further supported the notion that an adjustment period is necessary to reach optimal IGF-1 levels and profile. Females are known to be less sensitive to hGH replacement treatment (MOD-4023 as well) and usually require higher doses and longer period of titration. In addition, IGF-1 SDS levels as measured on day 4 were maintained constantly at a similar values within the normal range during the 4 month extension period, indicating that MOD-4023 can be administered in a weekly regimen. Following consecutive administrations of MOD-4023 no major decrease in IGF-1 levels at day 4 has been observed indicating that the Cmax and Ctrough of the "sinusoidal" behavior of IGF-1 are maintained along the study, confirming again weekly regimen of MOD-4023.

In conclusion, MOD-4023 should obviate the need for the numerous injections now required for the treatment of GHD. The results of this study have demonstrated that MOD-4023 can be injected once per week and achieve the clinical efficacy endpoints assessed, while maintaining a favorable safety profile. A GH treatment regimen that requires less frequent injections may improve compliance and potentially overall outcomes.

Hence, based on the achieved IGF-1 profile and the Phase II safety and tolerability results, the recommended injection frequency and dosing for the Phase III study are: a single weekly injection of MOD-4023 containing 61.7% of the cumulative weekly hGH dose, personalized for each patient.

### EXAMPLE 10

### Administration of a CTP-modified version of human growth hormone (hGH-CTP) improved pharmacokinetics in pre-pubertal growth hormone deficient (GHD) children

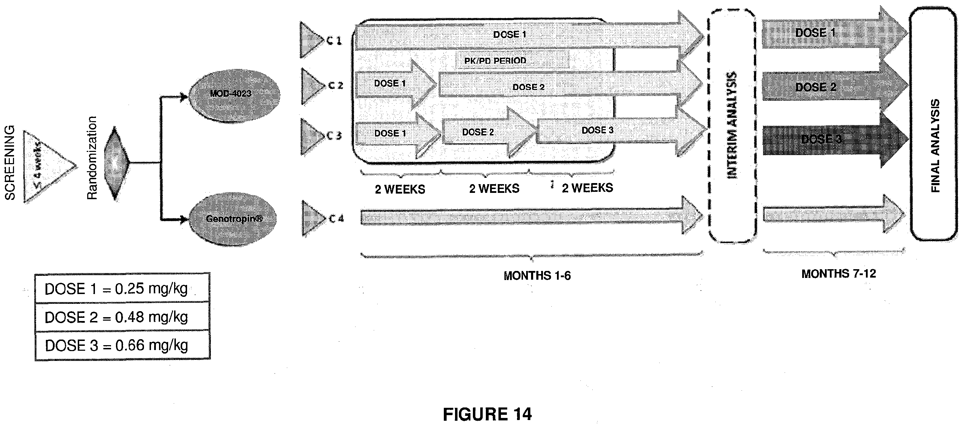

A randomized, open-label, Phase II Clinical Trial was conducted to evaluate the safety, tolerability pharmacokinetics and pharmacodynamics properties of three MOD-4023 doses to that of a commercially available standard daily recombinant human growth hormone. The study consisted of a 6 month screening and two active treatment periods: a 6 month treatment including PK/PD sampling followed by an additional 6 month continuous repeated dosing period as outlined in Figure 14. The secondary objectives were to evaluate the pharmacokinetics (PK) and pharmacodynamics (PD) profiles of three different doses of MOD-4023 in pre-pubertal growth hormone deficient (GHD) children and to select the optimal dose of MOD-4023 for the subsequent phase 3 study on the basis of safety and efficacy.

In order to introduce naive patients to the allocated MOD-4023 dose (see Table 15) in a gradual manner, a stepwise dose increase was implemented. All patients randomized to receive one of the three MOD-4023 doses started treatment for 2 weeks with the low MOD-4023 dose (0.25 mg/kg). Based on the patient's dose allocation, this was followed by a dose increase to the next dose level every two weeks until the final allocated dose has been reached.

**Table 15: Dose Cohorts**

| **Cohort** | **MOD-4023/Genotropin Dose** |
|---|---|
| 1 | 0.25 mg MOD-4023 protein/kg/week equivalent to 0.18 mg hGH/kg weekly injection. |
| 2 | 0.48 mg MOD-4023 protein/kg/week equivalent to 0.35 mg hGH/kg weekly injection. |
| 3 | 0.66 mg MOD-4023 protein/kg/week equivalent to 0.48 mg hGH/kg weekly injection. |
| 4 | Genotropin: 0.034 mg/kg/day. |

Subsequent to the second dose administration of the targeted dose, limited (population based) PK and PD sampling was performed as described in Table 16.

**Table 16: Dose Increase Scheme for MOD-4023 Cohorts**

| **Cohort** | **Dosing Scheme** | | | | | |
|---|---|---|---|---|---|---|
| | Week 1 | Week 2 | Week 3 | Week 4 | Week 5 | Week 6 |
| Cohort 1 | 0.25 mg | | | | | |
| | protein/kg/week | | | | | |
| | | PK/PD sampling | | | | |
| Cohort 2 | 0.25 mg | | **0.48 mg** | | | |
| | protein/kg/week | | **protein/kg/week** | | | |
| | | | | PK/PD sampling | | |
| Cohort 3 | 0.25 mg | | 0.48 mg | | **0.66 mg** | |
| | protein/kg/ /week | | protein/kg/week /week | | **protein/kg/week** /week | |
| | | | | | | PK/PD sampling |

Patients allocated to a MOD-4023 dose cohort were randomized within the cohort into one of three blocks and undergone limited PK/PD sampling (4 samples per patient over a period of one week), according to Table 17 below.

**Table 17: MOD-4023 Population PK and PD Sampling Scheme**

| Visit (V2, V3, V4) | a | | | b | c | d | e | f | g |
|---|---|---|---|---|---|---|---|---|---|
| Time after dosing(h)/ Block number | 0h | 6h | 12h | 24h | 48h | 72h | 96h | 120h | 168h |
| Block 1 | | | | | | | | | |
| Block 2 | | | | | | | | | |
| Block 3 | | | | | | | | | |

Patients allocated to Cohort 1 underwent limited PK/PD sampling following the 2nd dose of MOD-4023 (V2a-g - week 2) and returned to the medical centers for a single visit 4 days after dosing during week 6 (V4h).

Patients allocated to Cohort 2 came to the medical centers for a single visit 4 days after dosing during week 2 (V2h), underwent limited PK/PD sampling following the 4th dose of MOD-4023 (week 4: the second dose at the allocated dose level; V3a-g) and returned to the medical centers for a single visit 4 days after dosing during week 6 (V4h).

Patients allocated to Cohort 3 came to the medical centers for a single visit 4 days after dosing during weeks 2 and 4 (V2h and V3h) and underwent limited sampling following the 6th dose of MOD-4023 (week 6: the second dose at the allocated dose level, V4a-g).

At visits 2a and 2h, 3a and 3h, and 4a and 4h the patients underwent physical examination, vital signs, AEs, local tolerability, concomitant medications, parameters of glucose metabolism (fasting glucose and insulin; HbA1C only at V4), other hormonal levels (TSH, fT4, T3, cortisol), routine safety biochemistry and hematology (visits 2a and 2h, 4a and 4h), patient's height and weight, parameters of lipid metabolism, and IGF-1 and IGFBP-3 serum levels.

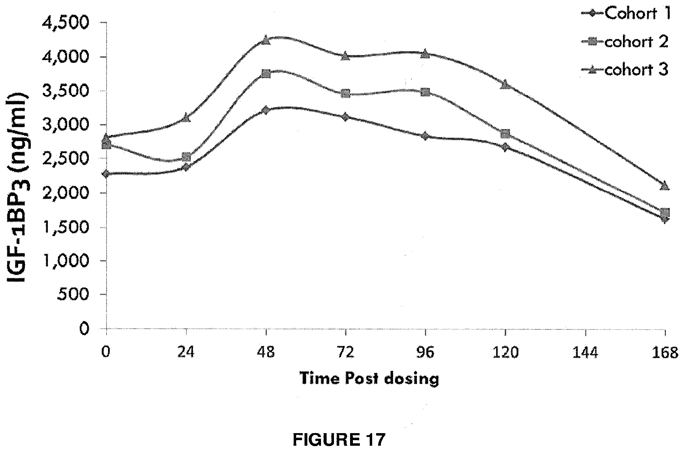

Patients allocated to the Genotropin cohort (cohort 4) returned to the medical centers for visits 2 and 4, during the 2nd and 6th week of treatment. The following procedures were performed: physical examination, vital signs, AEs, local tolerability, concomitant medications, parameters of glucose metabolism (fasting glucose and insulin; HbA1C only at V4), other hormonal levels (TSH, fT4, T3, cortisol), routine safety biochemistry and hematology, patient's height and weight, parameters of lipid metabolism, IGF-1 and IGF-1 BP-3 serum levels (Figure 17).

In addition, after the 8th Genotropin dose (start of week 2 of dosing), the patients allocated to the Genotropin cohort were randomized into one of three blocks and underwent limited PK/PD sampling (4 samples per patient over a period of 24 hours), according to Table 18 below:

**Table 18: Genotropin Population PK and PD Sampling Scheme (Visit 2)**

| Time after dosing(h)/ Block number | 0h | 1h | 2h | 4h | 6h | 12h | 16h | 20h | 24h |
|---|---|---|---|---|---|---|---|---|---|
| Block 1 | | | | | | | | | |
| Block 2 | | | | | | | | | |
| Block 3 | | | | | | | | | |

Following the first 6 weeks of the study, all patients visited the hospital on a monthly basis (weeks 10, 14, 18, 22 and 26). Patients allocated to the MOD-4023 dose cohorts (cohorts 1-3) were asked to return 4 days after MOD-4023 dosing in order to obtain MOD-4023, IGF-1 and IGFBP-3 levels and conduct routine safety assessments. In addition, after 5 months of dosing, patients allocated to MOD-4023 dosing were asked to return to the medical center in the morning hours prior to dosing in order to obtain a trough level MOD-4023 and PD (IGF-1 and IGF-1BP-3) samples. Patients allocated to the Genotropin dose cohort (cohort 4) were asked to return on any day during the relevant dosing week.

### Results:

Study demographic is presented of all patients in Table 19 below:

**Table 19: Phase 2 trial baseline demographic:**

| | **Cohort 1** | | **Cohort 2** | | **Cohort 3** | | **Cohort 4** | |
|---|---|---|---|---|---|---|---|---|
| **Dose** | **0.25 mg/kg/w MOD-4023** | | **0.48 mg/kg/w MOD-4023** | | **0.66 mg/kg/w MOD-4023** | | **0.034 µg/Kg/d Genotropin** | |
| **N** | 9 | | 9 | | 10 | | 7 | |

| | **Mean** | **SD** | **Mean** | **SD** | **Mean** | **SD** | **Mean** | **SD** |
|---|---|---|---|---|---|---|---|---|
| **Age (y)** | 6.44 | 2.3 | 6.33 | 2.1 | 6.10 | 2.2 | 5.43 | 1.9 |
| **Peak GH (ng/ml)*** | 2.84 | 2.9 | 3.58 | 1-7 | 4.41 | 3.2 | 2.92 | 2.4 |
| **HV SDS** | -3.05 | 2.0 | -2.82 | 1.1 | -3.11 | 1.8 | -3.36 | 2.0 |
| **HT SDS** | -3·99 | 0.9 | -3.82 | 0.8 | -3.91 | 1.1 | -4.79 | 1.7 |
| **HtSDS - TH SDS** | -3.47 | 0.9 | -3.23 | 0.7 | -3.25 | 1.3 | -4.20 | 1.8 |
| **Screening IGF-1 SDS** | -2.48 | 0.8 | -2.28 | 0.7 | -1.81 | 0.7 | -2.34 | 1.2 |
| **Gender (%)** | F 1 (11.1) | M 8(88.8) | F 4 (44.4) | M 5(55.6) | F 3(30) | M 7(70) | F 3 (42.9) | M 4 (57.1) |

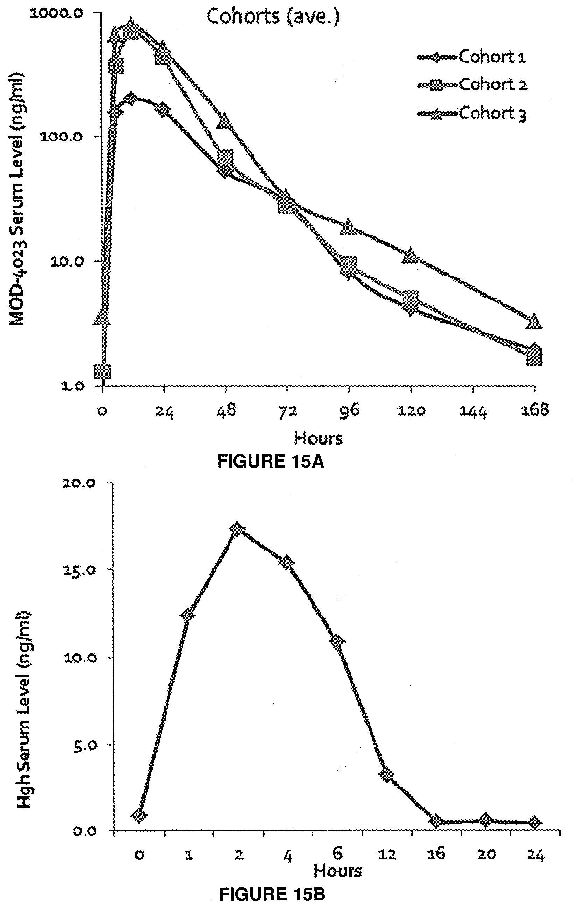

The average PK profile of MOD-4023 administrated to naive GHD at their final administered dose is provided in Figure 15 while the PK parameters are provided on Table 20 below:

**Table 20: Comparative Average PK Parameters**

| | **Units** | **MOD-4023** | | | **hGH** |
|---|---|---|---|---|---|
| | | **C1(n=13)** | **C2(n=12)** | **C3(n=13)** | **C4(n=11)** |
| T_{1/2} | hr | **36.1** | **29.2** | **29.1** | **3.6** |
| Tₘₐₓ | hr | 12 | 12 | 12 | 2 |
| Cₘₐₓ | ng/ml | 459.9 | 810.2 | 795.5 | 17.3 |
| AUC 0-inf_obs | ng/ml*hr | 10943.4 | 20050.3 | 25503.1 | 135.7 |
| Cl/F_obs | (mg/kg)/(ng/ml)/hr | 2.28E-05 | 2.39E-05 | 2.59E-05 | 2.51E-04 |

As anticipated MOD-4023 administered once a week demonstrated an extended half-life which was shown to be 8 fold higher compared to daily hGH. In addition a dose dependent response was observed as reflected by the AUC values of each MOD-4023 dose.

The Dose dependent response was maintained throughout the first 6 months of weekly administration of MOD-4023 (data not shown).

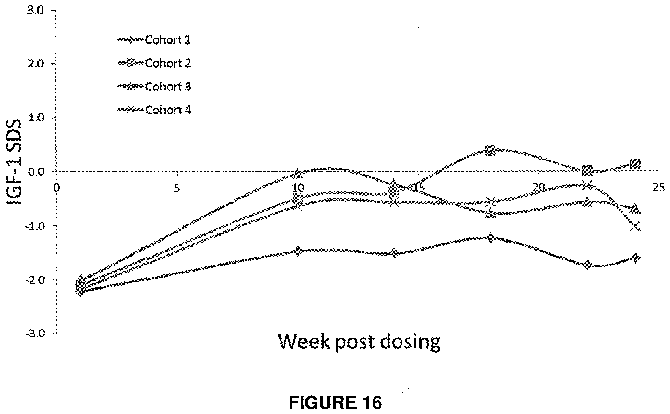

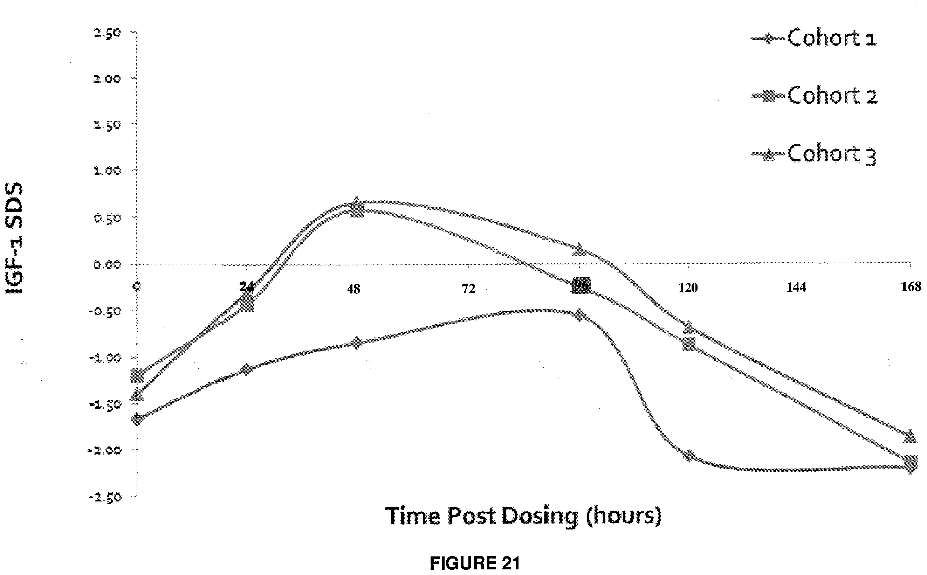

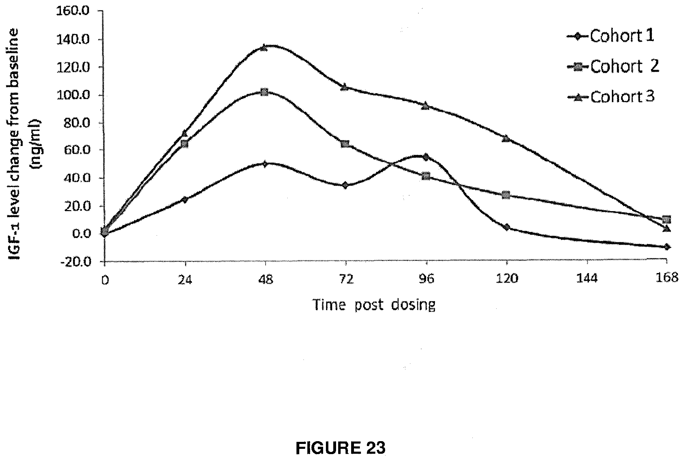

IGF-1 which is a validated surrogate marker for hGH activity was also increased at a dose dependent level (Figure 23) maintaining the target values (above -2 height SD score [SDS]) for the majority of the week without excessive levels (>2SDS, Figure 21). The IGF-1 SDS levels continued to moderately elevate at a dose dependent manner during the first 6 month of the study, without reaching excessive levels which are above 2SDS (Figure 16).

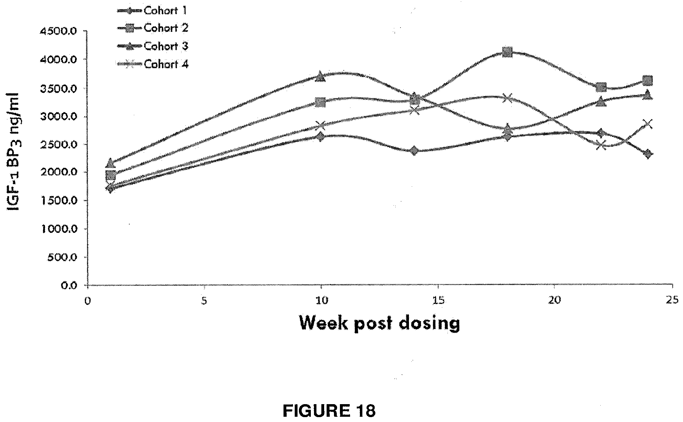

The hGH treated arm (cohort 4) also demonstrated an elevation in IGF-1 SDS values that were very similar to the trend observed with two highest cohorts of MOD-4023. Furthermore IGF-1 BP-3 values also increased in a dose dependent manner upon MOD-4023 administration reaching steady-state values around week 15 (Figures 17 and 18 respectively). Altogether, the two pharmacodynamics profiles of IGF-1 and IGF1BP-3 confirm that MOD-4023 single weekly injection can replace 7 daily injections at a similar dose range

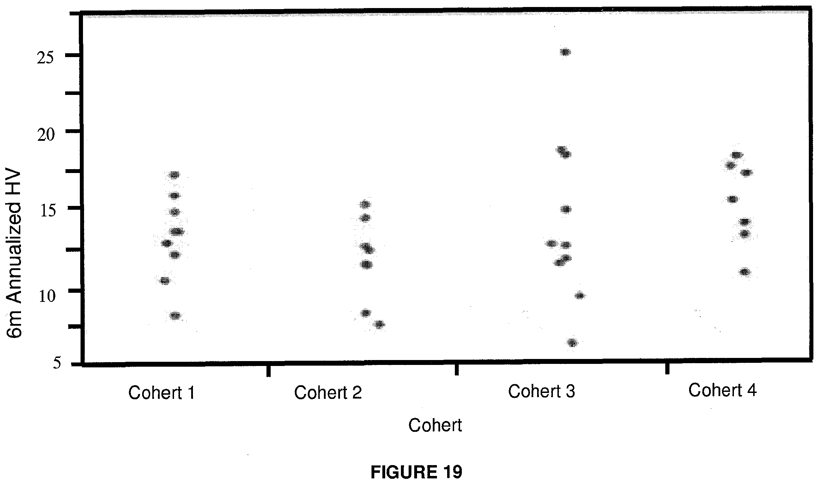

Height velocity was monitored at pre dose and 6 month post weekly dosing of MOD-4023 or daily dosing of hGH. For all cohorts an excellent growth response was obtained with no statistical difference between the different cohorts (Table 21 and Figure 19), further confirming that weekly injection of MOD-4023 can enable proper growth as daily hGH.

**Table 21 MOD-4023 6m Annualized Height Velocity - all patients completing 6m treatment**

| Cohort | Dose | N | Mean (CM) | Std Dev |
|---|---|---|---|---|
| Cohort 1 | 0.25 mg/kg/w MOD-4023 | 9 | 13.48 | 2.71 |
| Cohort 2 | 0.48 mg/kg/w MOD-4023 | 9 | 12.25 | 2.64 |
| Cohort 3 | 0.66 mg/kg/w MOD-4023 | 10 | 14.37 | 5.26 |
| Cohort 4 | 0.034 µg/kg/d Genotropin | 7 | 15.46 | 2.68 |
| | Historical data - daily hGH | | ∼10 | |

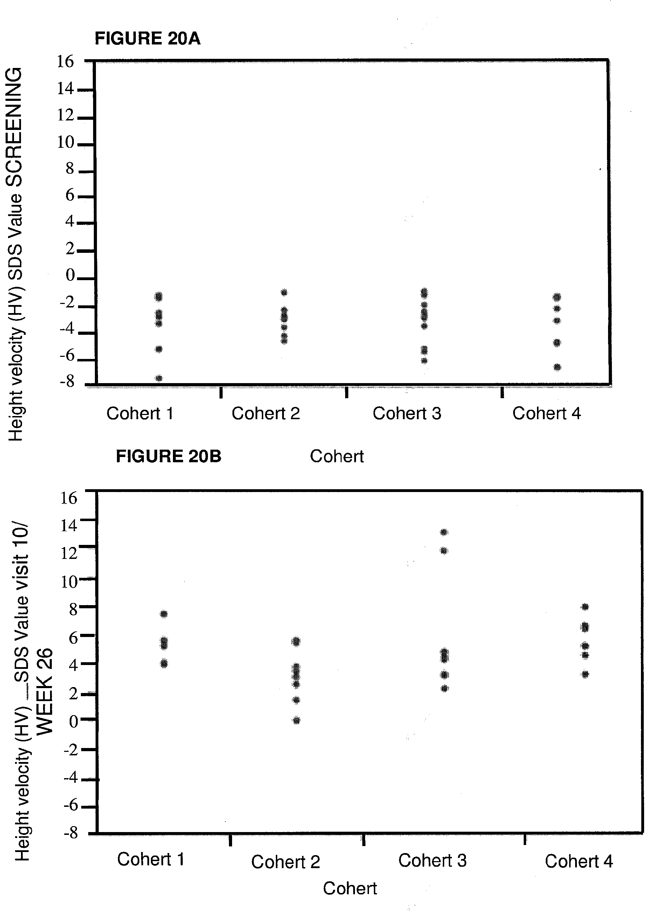

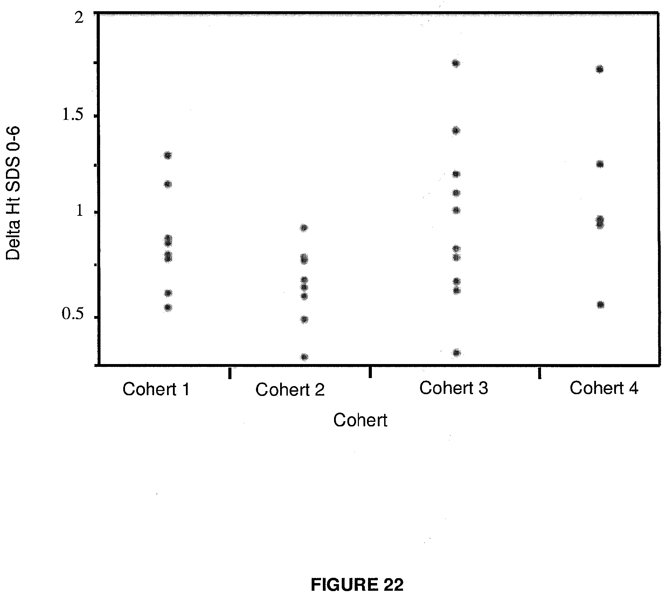

In parallel, an excellent increase in height velocity SDS was also obtained (Table 22, Table 23, and Figure 20). Finally delta height SDS demonstrated excellent correlation to patients' catch up growth (Figure 22).

**Table 22: MOD-4023 Ped. Phase 2 - Pre-Study HV SDS Results**

| Cohort | **Dose** | **N** | **Mean** | **Std Dev** | **SE Mean** |
|---|---|---|---|---|---|
| Cohort 1 | 0.25 mg/kg/w MOD-4023 | 8 | -3.21 | 2.05 | 0.72 |
| Cohort 2 | 0.48 mg/kg/w MOD-4023 | 8 | -2.94 | 1.14 | 0.40 |
| Cohort 3 | 0.66 mg/kg/w MOD-4023 | 10 | -3.11 | 1.79 | 0.56 |
| Cohort 4 | 0.034 µg/kg/d Genotropin | 7 | -3.36 | 1.98 | 0.75 |

**Table 23: MOD-4023 Ped. Phase 2 - 6m HV SDS Results**

| Cohort | Dose | **N** | **Mean** | **Std Dev** | **SE Mean** |
|---|---|---|---|---|---|
| Cohort 1 | 0.25 mg/kg/w MOD-4023 | 8 | 5.03 | 1.23 | 0.44 |
| Cohort 2 | 0.48 mg/kg/w MOD-4023 | 8 | 3.23 | 1.88 | 0.67 |
| Cohort 3 | 0.66 mg/kg/w MOD-4023 | 10 | 5.73 | 3.72 | 1.18 |
| Cohort 4 | 0.034 µg/kg/d Genotropin | 7 | 5.67 | 1.53 | 0.58 |

### Conclusion

All doses provided a good catch-up growth response. Preliminary statistical analysis indicates that there are no statistically significant differences between the cohorts but there are some limitations as to the limited number of patients per cohort and the relatively severe GHD patients.

### EXAMPLE 11

### Formulation development of MOD-4023

**The protein:** MOD-4023 is a long-acting recombinant human Growth Hormone (hGH) for subcutaneous administration. MOD-4023 consists of hGH fused to three copies of the C-terminal peptide (CTP) of the beta chain of human Chorionic Gonadotropin (hCG); The CTP includes four O-glycosylation sites and therefore, the protein is a single chain of 275 amino acids with up to twelve O-linked carbohydrates. The protein is manufactured in CHO cells from a producing clone.

**Producing Clone:** Clone#2 was the original clone used for the early toxicological studies, Phase I and Phase II (adults). Stability data for the DS and DP for this clone are available for up to 2 years at -20°C and 5°C. Conversion to a new producing clone (Clone #28) was carried out to improve productivity and clone stability. Clone #28 DP supported the long term toxicological studies and Phase II in children, and will support all further clinical activities and commercial manufacturing. Stability data for the DP for this clone are available for up to 1 year at -20°C and 5°C.

**Manufacturing CMO:** The manufacturing of MOD-4023 was executed by Xcellerex (USA) at early stages and supported non-clinical studies up to Phase II. The process was transferred to Rentschler Biotechnologie (RB), (Germany). Two GMP batches were already produced at RB.

### Additional Information

### Physicochemical Properties -

Highly glycosylated and negatively charged with pI = 3-4.5
Density: 1.0216 g/ml
Soluble in aqueous solution
Liquid formulation for both DS and DP: 10mM Citrate, 147 mM NaCl pH 6.
Final concentration of DS: 40 mg/ml
Final concentration of DP: 5, 10, 20 and 40 mg/ml
Primary Packaging -
2R vials (Schott)
Stoppers (West)
Aluminum Seals (West)
Future Primary Packaging - PEN Device

### Objective of Formulation Optimization

### To develop a stable liquid formulation for MOD-4023:

1. First objective: 2 years stability at 5°C in vials
2. Second objective: 2 years stability at 5°C in cartridges

### Analytical tests needed:

RP-HPLC ( Validated method)
SEC-HPLC ( Validated method)
CZE (TBD) ( Established method)

### Timeline:

Based on the stability data, 2w at 25°C can be employed to predict product stability at 5°C to enable an initial assessment for comprehensive matrix formulation study.

### Stability Data

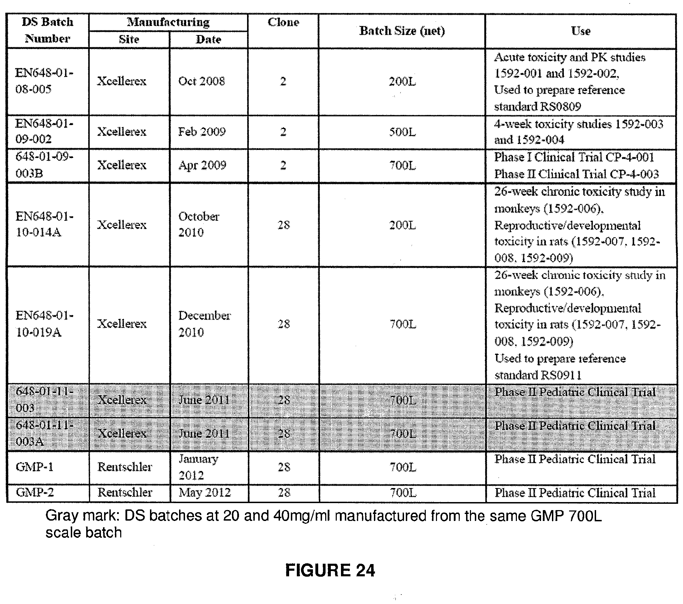

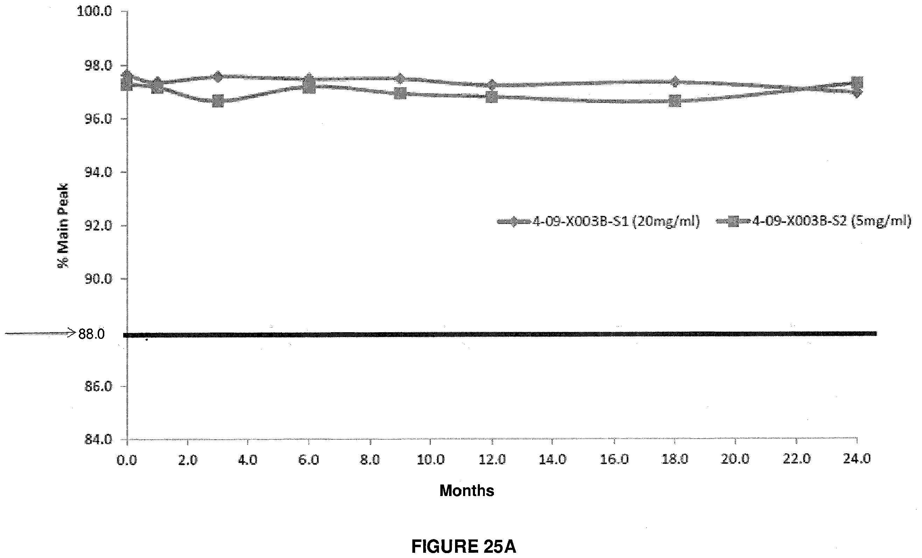

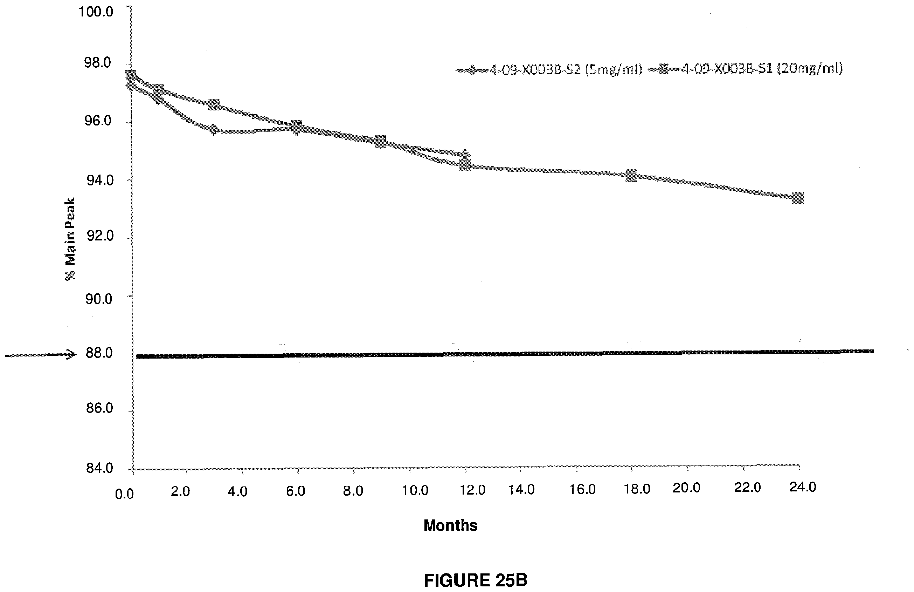

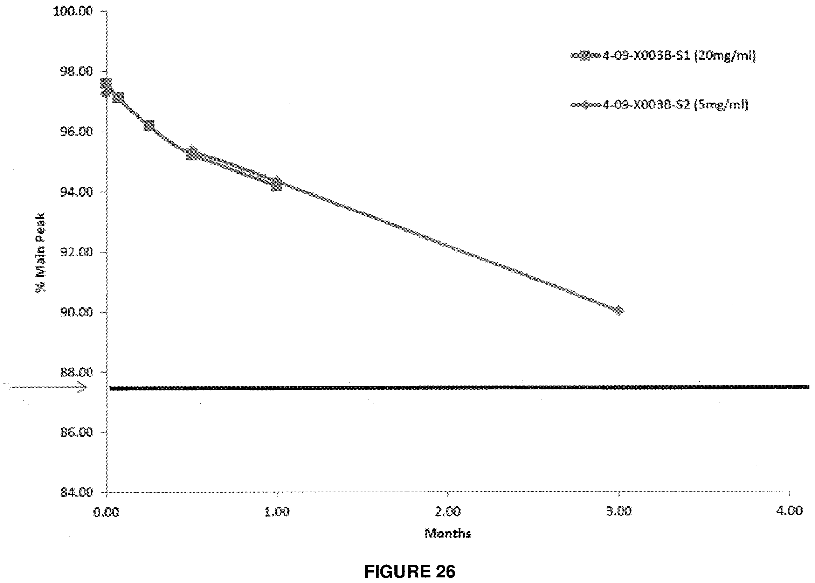

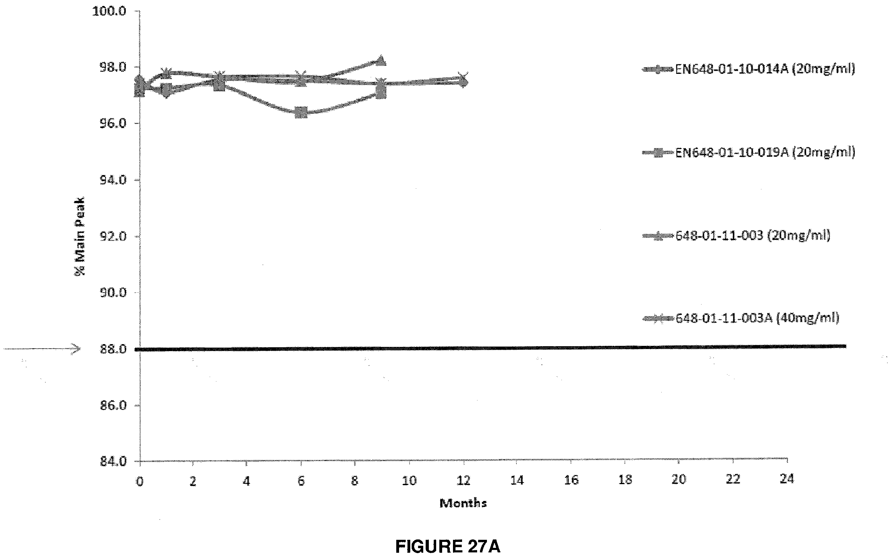

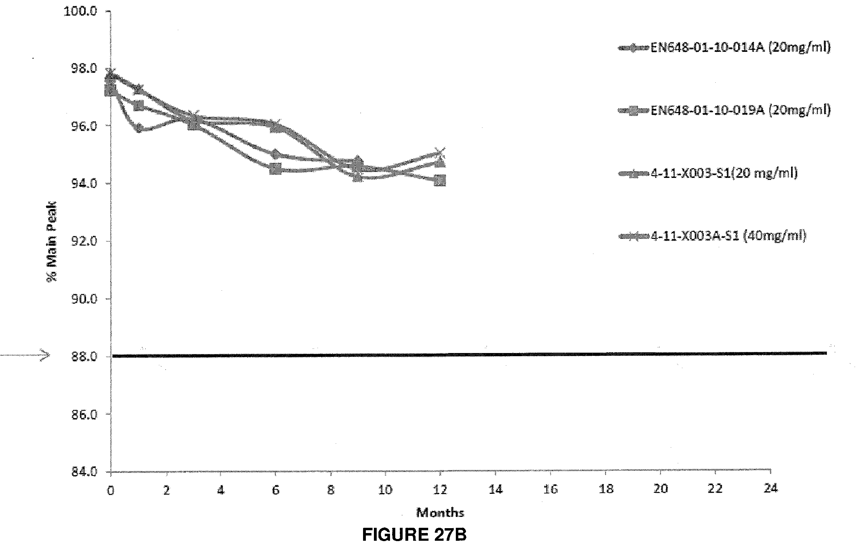

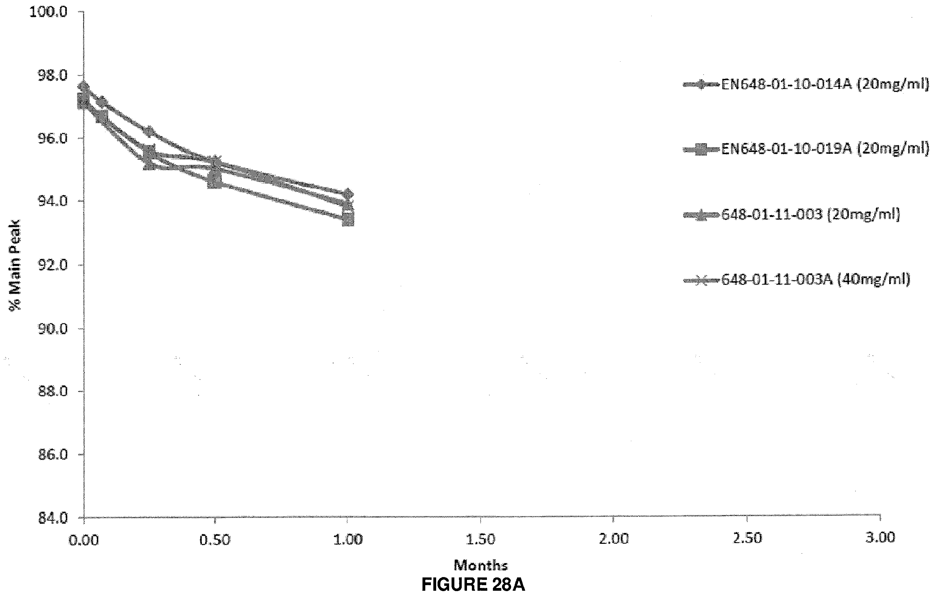

Data shows non-GMP and GMP batches produced in 10 mM Citrate, 147 mM NaCl pH 6 (Fig. 24). Data also shows that MOD-4023 Clone 2 is stable at 5°C for 24 months in 20 mg/m and that there's a similar stability profile between 5mg/ml and 20mg/ml (see Figs. 25A and 25B). Moreover, MOD-4023 Clone 2 is stable for 3 months at room temperature (see Fig. 26). MOD-4023 Clone 28 is stable at 5°C for 12 months at 20 mg/ml or 40mg/ml; DP Main Peak Specification >88% (Fig. 27A and Fig. 27B). MOD-4023 Clone 28 is stable for at least one month at room temp. (Fig. 28A).

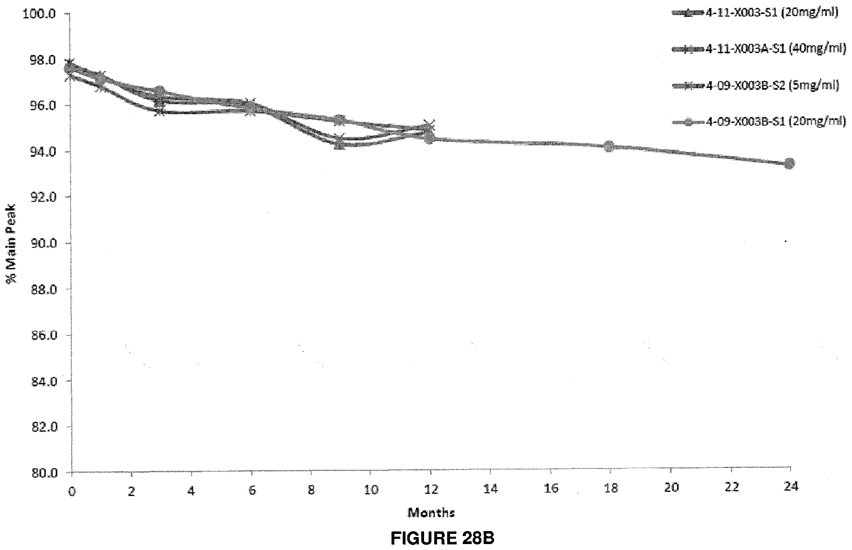

Based on the similarity between Clone 28 and Clone 2, which is stable at 5°C for 24m, it is expected that clone 28 produced at Xcellerex (XC) will be stable for 24m at 5°C; DP Main Peak Specification >88% (Fig. 28B).

### Manufacturing of MOD-4023

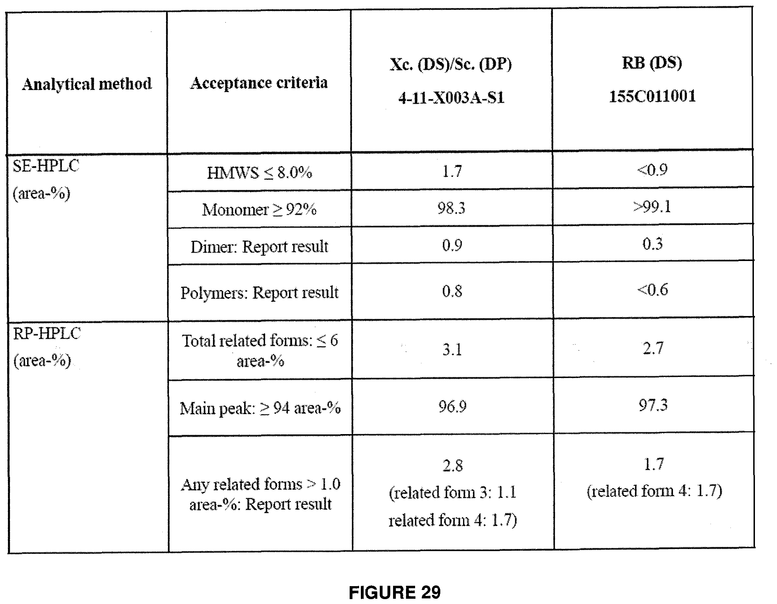

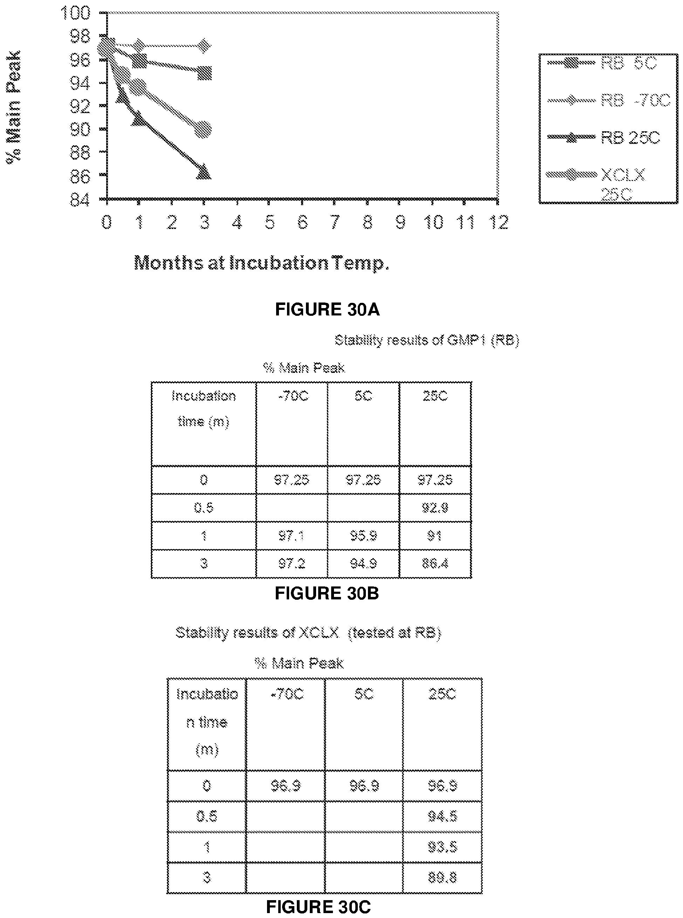

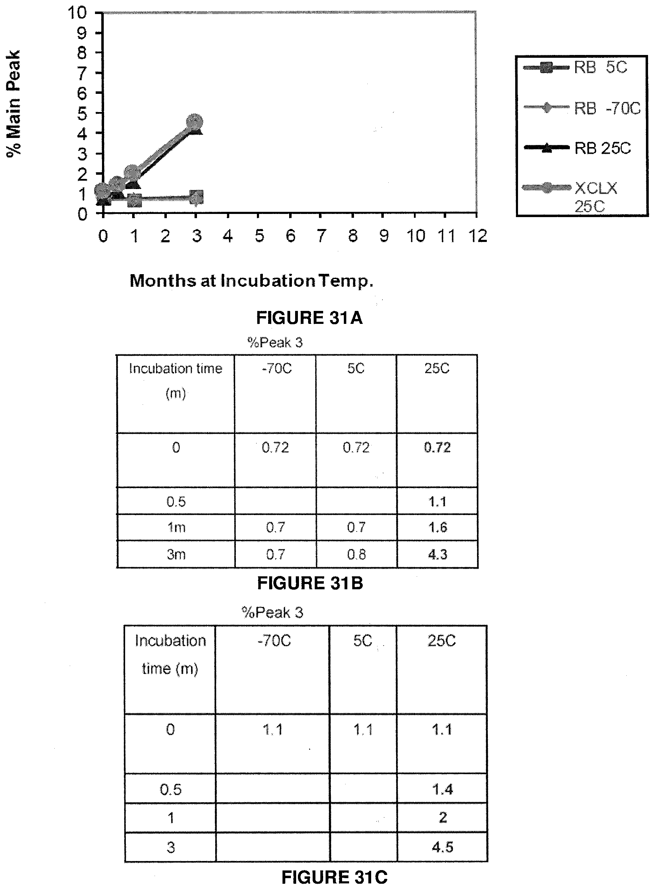

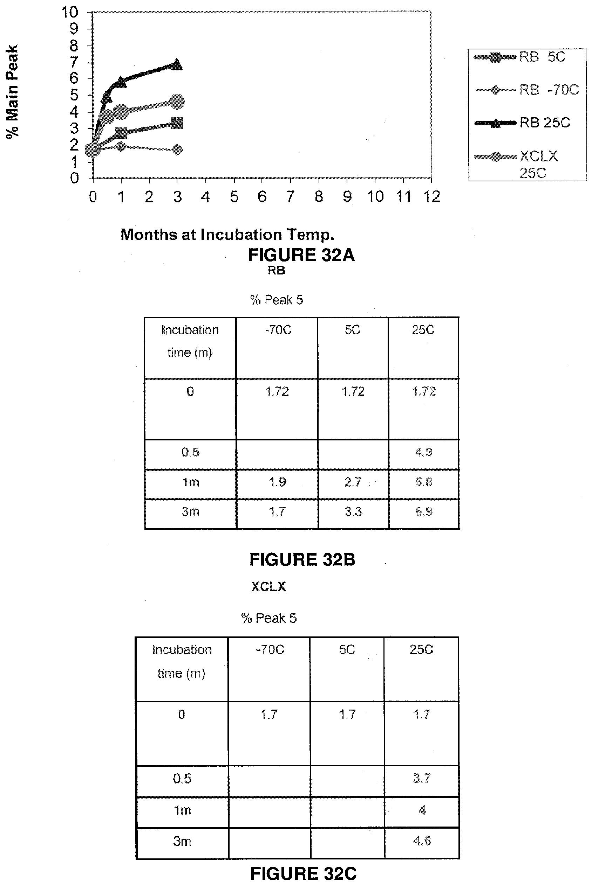

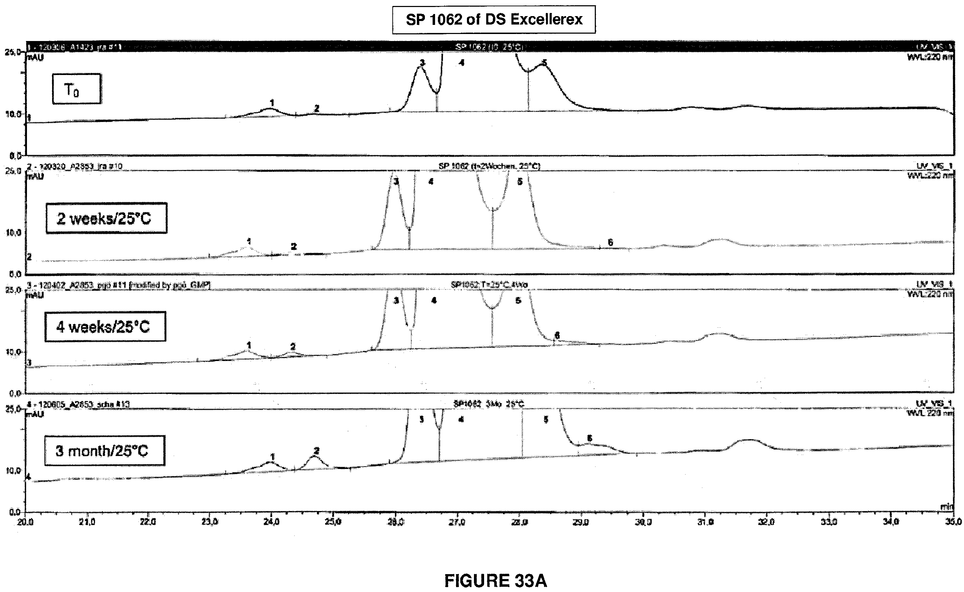

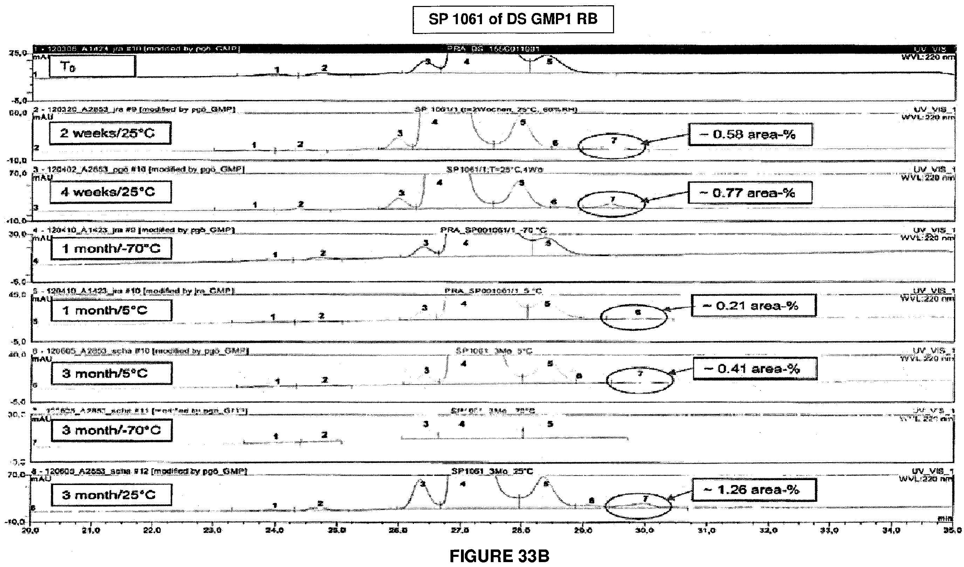

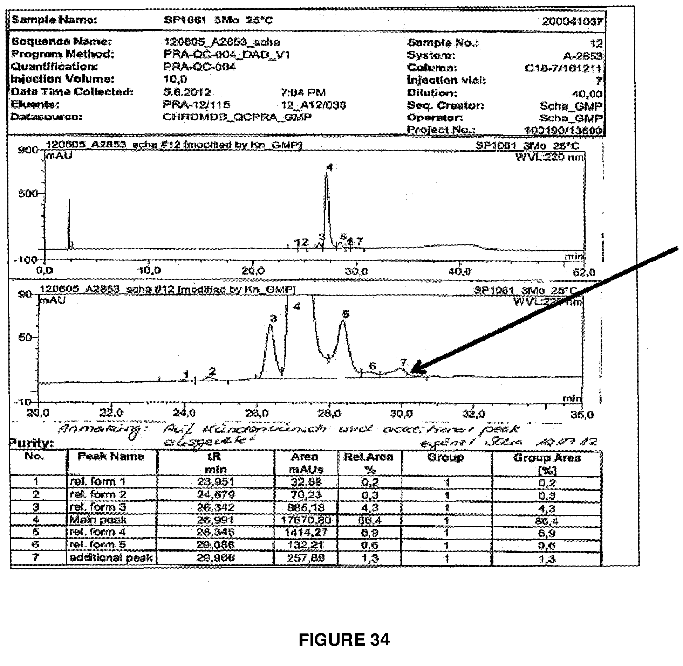

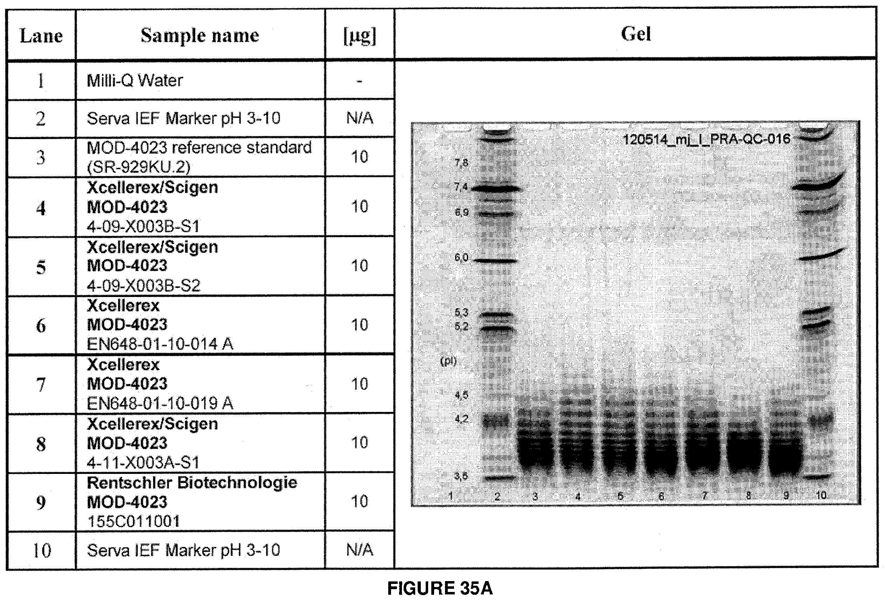

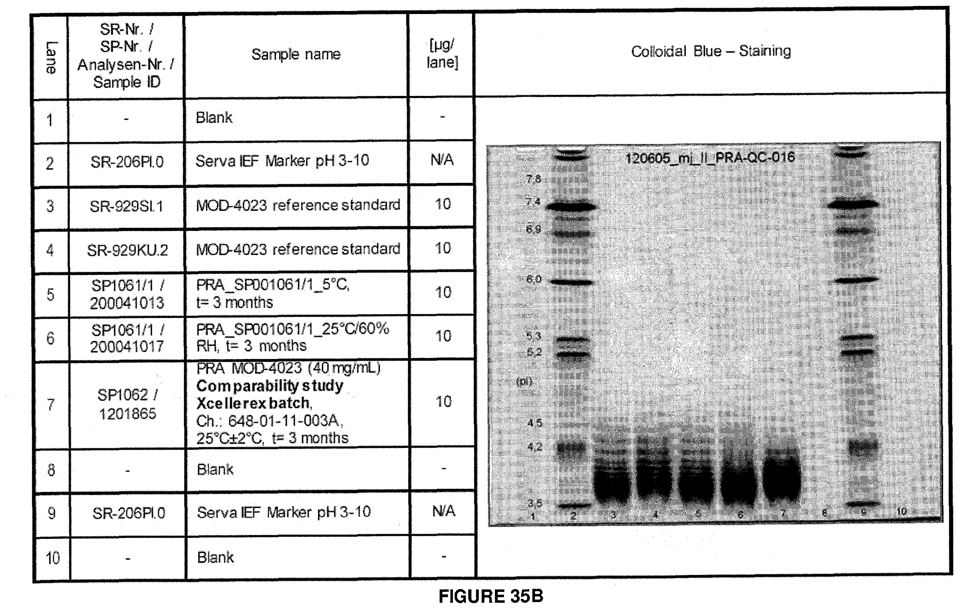

Comparable profile at T=0 between Xcellerex (XC) to Rentschler (RB) DS (Fig. 29). Figures 30-34 show differences in stability between XC and RB. Isoelectric focusing (IEF) demonstrates that there's a similar band pattern in a pI-value range from 3.5 to 4.2. In one XC batch there are less faint isoforms in the high pI boundary and in an RB batch there are more faint isoforms in the low pI boundary (Fig. 35A). In addition, there are more diffuse bands in XC sample as compared to the RB sample (Fig. 35B).

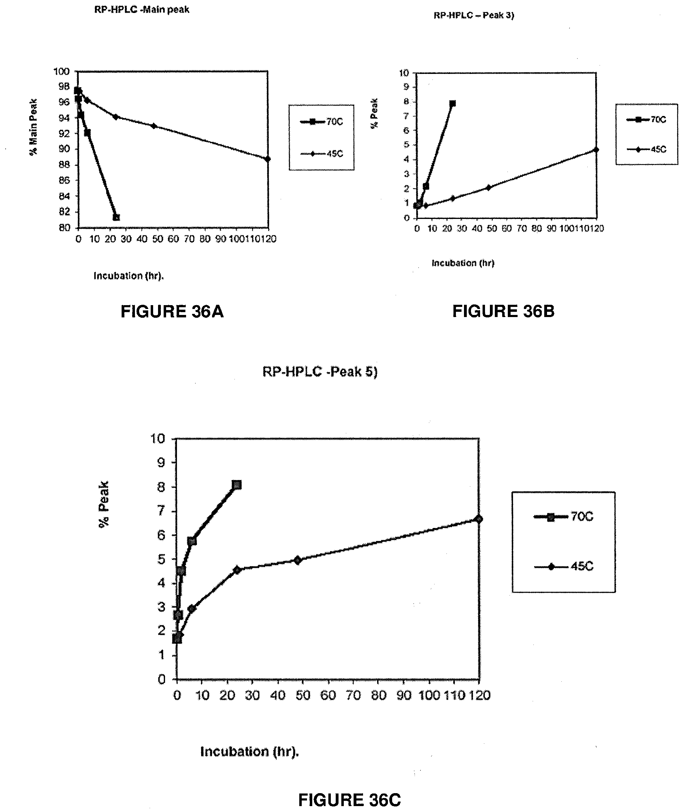

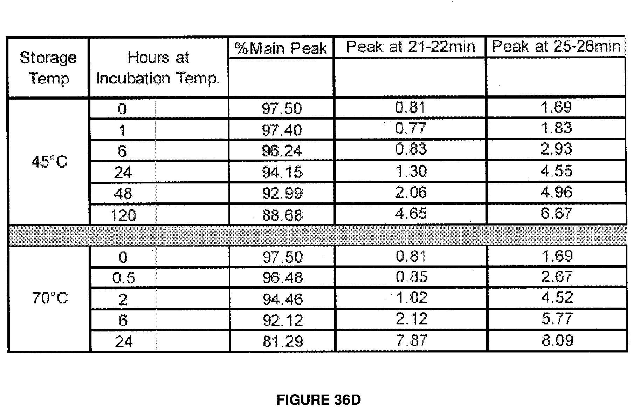

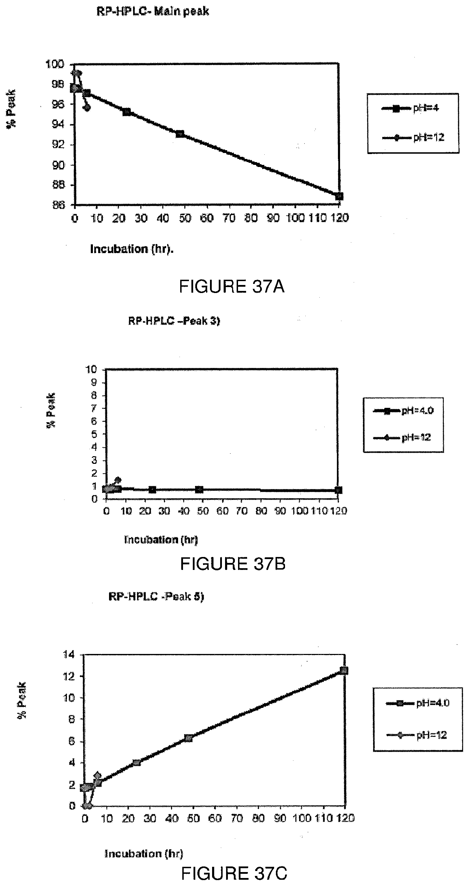

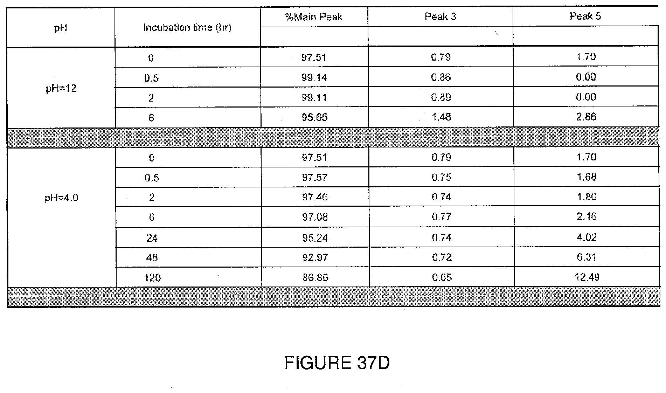

Additional observations showed that the formation of both peaks (3 and 5 - see below for peak definitions) is temperature dependent and accelerates at high temperature (Fig. 36A-D)). Further, there was no change in the % of peak 3 after incubation for up to 5 days at pH=4 and up to 2 h at pH=12 (Fig. 37B, 37D) and there was no change in the % of the peak after incubation for up to 6 h at pH=4. However, following 6 h a sharp increase in the peak % was observed; at pH 12 incubation for up to 2 h - the peak disappears (Fig. 37A-D).

### Forced degradation studies at RB (clone 28)

A stressed sample of MOD-4023 drug substance was prepared (65°C for about three days) for analysis of related form 5 in MOD-4023 drug substance as the peak is below the LOQ for the unstressed sample.

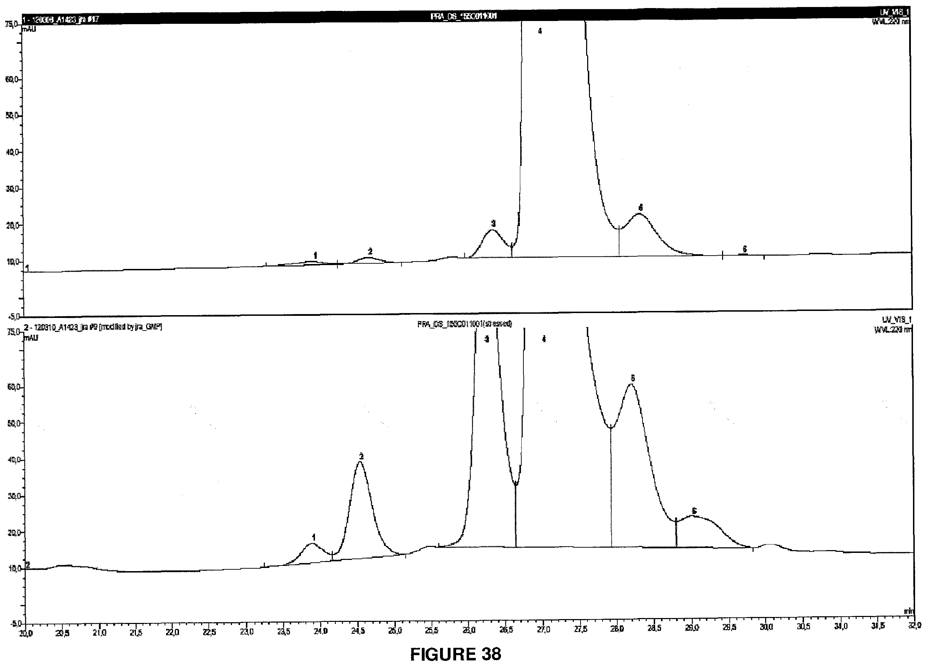

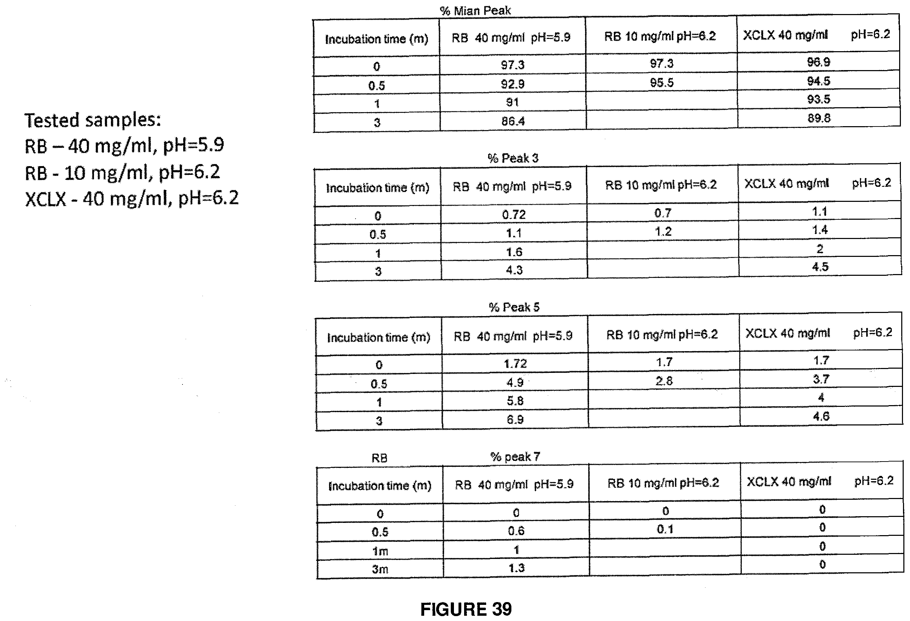

In order to test pH effect on RP-HPLC related forms three sample were tested:
RB - 40 mg/ml, pH=5.9.
RB - 10 mg/ml, pH=6.2.
XC- 40 mg/ml, pH=6.2.
Results are provided in Figures 38 and 39.

### Isolation and characterization of related form peaks (1-7) - M-Scan

Peak 1 -Oxidation of deamidated MOD-4023
Peak 2- Deamidation of MOD-4023
Peak 3- Partially oxidation of MOD-4023
Peak 5- Peptide bond cleavage between amino acid residues 167 and 168 and between amino acid residues 171 and 172 of MOD-4023. disulphide
Peak 6 and peak 7- Truncated forms

### Conclusions (see Figs 24-39)

### Stability

Clone 2 derived product is stable for up to 2 years at 5°C.

Clone 28 derived product manufactured at XC is stable for at least 1 year at 5°C with similar profile as clone 2.

Clone 28 derived product manufactured at RB has altered stability profile with accelerated generation of related forms (mainly peak 5) and a generation of new peak (peak 7), not previously observed.

Previous studies show that Peak 3 and 5 have a similar Mw as the main peak and react with anti-hGH corresponding MOD-4023 band.

Both peaks: 3 and 5 are temperature dependent (the % of the peaks increases when the temperature is increased).

No change in the percentage of the HMW forms was observed during incubation at - 20°C and 5°C.

Stability of RB product GMP1 (after incubation of 2 weeks) at different temperatures: 5, 25, 37, 50 and 65°C demonstrated that Peak 7 formation is accelerated at 25°C and 37°C but it is not observed at 50 and 65°C.

Incubation of RB samples for 10 min at 65°C followed by incubation at 25°C eradicated the generation of peak 7 (after 2 week at 25°C).

## Claims

1. A chorionic gonadotropin carboxy terminal peptide (CTP)-modified polypeptide comprising a growth hormone (GH), wherein one CTP is attached to the amino terminus of said growth hormone, and two chorionic gonadotropin CTPs are attached in tandem to the carboxy terminus of said growth hormone, optionally including a signal peptide attached to the amino terminus of said one CTP,
for use in maintaining insulin-like growth factor (IGF-1) levels within a normal therapeutic range of ±2 SDS in a human subject for six months,
wherein the subject is a growth hormone deficient (GHD) child, and said CTP-modified growth hormone is administered once a week at a dose of 0.66 mg/kg/week.

2. The CTP-modified polypeptide for use according to any of the preceding claims, wherein at least one CTP is glycosylated.

3. The CTP-modified polypeptide for use according to any of the preceding claims, wherein at least one CTP is truncated.

4. The CTP-modified polypeptide for use according to any of the preceding claims, wherein at least one CTP is optionally attached to said growth hormone via a linker, wherein the linker may be a peptide bond.

5. The CTP-modified polypeptide for use according to any of the preceding claims, wherein the administration results in an annualized height velocity increase of at least about 14.37 cm, based on an interim analysis after 6 months treatment.

6. The CTP-modified polypeptide for use according to any of the preceding claims, wherein the subsequent doses of the CTP-modified polypeptide may be modified from the starting dose based on the IGF-1 levels as measured 4 days after the previous dose.

7. The CTP-modified polypeptide for use according to any of the preceding claims, wherein the IGF-1 levels comprise IGF-1 levels of samples tested four days post-administration of said CTP-modified polypeptide.

8. The CTP-modified polypeptide for use according to any of the preceding claims, wherein the subsequent doses of the CTP-modified polypeptide are provided weekly.

9. The CTP-modified polypeptide for use according to any of the preceding claims, wherein the growth hormone is a human growth hormone (hGH).

10. The CTP-modified polypeptide for use according to any of the preceding claims, wherein said subject is a naive subject.

11. The CTP-modified polypeptide for use according to any of the preceding claims, wherein the sequence of at least one CTP consists of the amino acid sequence of SEQ ID NO: 17 or SEQ ID NO: 18.

12. The CTP-modified polypeptide for use according to any of the preceding claims, wherein the amino acid sequence of the signal peptide is set forth in SEQ ID NO: 49.

13. The CTP-modified polypeptide for use according to any of claims 1-11, wherein the amino acid sequence of said CTP-modified polypeptide comprises the sequence set forth in amino acids 27-301 of SEQ ID NO: 39 or amino acids 27-285 of SEQ ID NO: 40.

14. The CTP-modified polypeptide for use according to any of claims 1-12, wherein the amino acid sequence of said CTP-modified polypeptide comprises the sequence set forth in SEQ ID NO: 39 or SEQ ID NO: 40.

15. The CTP-modified polypeptide for use according to any one of the preceding claims, wherein the CTP-modified polypeptide is encoded by the nucleic acid sequence set forth in SEQ ID NO: 44 or SEQ ID NO: 45.

16. The CTP-modified polypeptide for use according to any one of the preceding claims, wherein the CTP-modified polypeptide is administered in a step-wise dose increase at two-week intervals until a dose of 0.66 mg/kg/week is reached.

## Patentansprüche

1. Durch carboxy-terminales Peptid (CTP) von Choriongonadotropin modifiziertes Polypeptid, umfassend ein Wachstumshormon (GH), wobei ein CTP an dem Amino-Terminus des Wachstumshormons angehängt ist und zwei Choriongonadotropin-CTPs zusammen an dem Carboxy-Terminus des Wachstumshormons angehängt sind, wahlweise einschließlich eines Signalpeptids, das an dem Amino-Terminus des einen CTP angehängt ist,
zur Verwendung beim Aufrechterhalten von insulinähnlichen Wachstumsfaktor (IGF-1)-Spiegeln innerhalb eines normalen therapeutischen Bereichs von ±2 SDS bei einem menschlichen Subjekt für eine Dauer von sechs Monaten,
wobei das Subjekt ein Kind mit Wachstumshormonmangel (GHD) ist und das CTP-modifizierte Wachstumshormon einmal pro Woche in einer Dosis von 0,66 mg/kg/Woche verabreicht wird.

2. CTP-modifiziertes Polypeptid zur Verwendung nach einem der vorhergehenden Ansprüche, wobei mindestens ein CTP glykosyliert ist.

3. CTP-modifiziertes Polypeptid zur Verwendung nach einem der vorhergehenden Ansprüche, wobei mindestens ein CTP trunkiert ist.

4. CTP-modifiziertes Polypeptid zur Verwendung nach einem der vorhergehenden Ansprüche, wobei mindestens ein CTP wahlweise über einen Linker an das Wachstumshormon angehängt ist, wobei der Linker eine Peptidbindung sein kann.

5. CTP-modifiziertes Polypeptid zur Verwendung nach einem der vorhergehenden Ansprüche, wobei die Verabreichung basierend auf einer Zwischenanalyse nach einer 6-monatigen Behandlung zu einer annualisierten Erhöhung der Größenwachstumsgeschwindigkeit von mindestens etwa 14,37 cm führt.

6. CTP-modifiziertes Polypeptid zur Verwendung nach einem der vorhergehenden Ansprüche, wobei die nachfolgenden Dosen des CTP-modifizierten Polypeptids basierend auf den IGF-1-Spiegeln, gemessen jeweils 4 Tage nach der vorherigen Dosis, in Bezug auf die Anfangsdosis modifiziert werden können.

7. CTP-modifiziertes Polypeptid zur Verwendung nach einem der vorhergehenden Ansprüche, wobei die IGF-1-Spiegel IGF-1-Spiegel von Proben umfassen, die vier Tage nach der Verabreichung des CTP-modifizierten Polypeptids getestet wurden.

8. CTP-modifiziertes Polypeptid zur Verwendung nach einem der vorhergehenden Ansprüche, wobei die nachfolgenden Dosen des CTP-modifizierten Polypeptids wöchentlich bereitgestellt werden.

9. CTP-modifiziertes Polypeptid zur Verwendung nach einem der vorhergehenden Ansprüche, wobei das Wachstumshormon ein menschliches Wachstumshormon (hGH) ist.

10. CTP-modifiziertes Polypeptid zur Verwendung nach einem der vorhergehenden Ansprüche, wobei das Subjekt ein naives Subjekt ist.

11. CTP-modifiziertes Polypeptid zur Verwendung nach einem der vorhergehenden Ansprüche, wobei die Sequenz von mindestens einem CTP aus der Aminosäuresequenz von SEQ ID NO: 17 oder SEQ ID NO: 18 besteht.

12. CTP-modifiziertes Polypeptid zur Verwendung nach einem der vorhergehenden Ansprüche, wobei die Aminosäuresequenz des Signalpeptids in SEQ ID NO: 49 angegeben ist.

13. CTP-modifiziertes Polypeptid zur Verwendung nach einem der Ansprüche 1-11, wobei die Aminosäuresequenz des CTP-modifizierten Polypeptids die Sequenzen umfasst, die in den Aminosäuren 27-301 von SEQ ID NO: 39 oder den Aminosäuren 27-285 von SEQ ID NO: 40 angegeben sind.

14. CTP-modifiziertes Polypeptid zur Verwendung nach Anspruch 1-12, wobei die Aminosäuresequenz des CTP-modifizierten Polypeptids die Sequenz umfasst, die in SEQ ID NO: 39 oder SEQ ID NO: 40 angegeben ist.

15. CTP-modifiziertes Polypeptid zur Verwendung nach einem der vorhergehenden Ansprüche, wobei das CTP-modifizierte Polypeptid durch die Nukleinsäuresequenz codiert wird, die in SEQ ID NO: 44 oder SEQ ID NO: 45 angegeben ist.

16. CTP-modifiziertes Polypeptid zur Verwendung nach einem der vorhergehenden Ansprüche, wobei das CTP-modifizierte Polypeptid in einer schrittweisen Dosiserhöhung in Zeitabständen von jeweils zwei Wochen verabreicht wird, bis eine Dosis von 0,66 mg/kg/Woche erreicht ist.

## Revendications

1. Polypeptide de gonadotrophine chorionique à peptide à carboxy terminaux (CTP) modifiés comprenant une hormone de croissance (GH), dans lequel un CTP est attaché à l'extrémité amino-terminale de ladite hormone de croissance et deux CTP gonadotrophine chorionique sont attachés en tandem à l'extrémité carboxy terminale de ladite hormone de croissance, comprenant éventuellement un séquence-signal attaché à l'extrémité amino-terminale dudit CTP,
destiné à être utilisé pour maintenir des niveaux de facteur de croissance ressemblant à l'insuline (IGF-1) dans une plage thérapeutique normale de ±2 ET chez un sujet humain pendant six mois,
dans lequel le sujet est un enfant déficient en hormone de croissance (GHD), et ladite hormone de croissance à CTP modifiés est administrée une fois par semaine à une dose de 0,66 mg/kg/semaine.

2. Polypeptide à CTP modifiés à utiliser selon l'une quelconque des revendications précédentes, dans lequel au moins un CTP est glycosylé.

3. Polypeptide à CTP modifiés à utiliser selon l'une quelconque des revendications précédentes, dans lequel au moins un CTP est tronqué.

4. Polypeptide à CTP modifiés à utiliser selon l'une quelconque des revendications précédentes, dans lequel au moins un CTP est facultativement attaché à ladite hormone de croissance via un lieur, dans laquelle le lieur peut être une liaison peptidique.

5. Polypeptide à CTP modifiés à utiliser selon l'une quelconque des revendications précédentes, dans lequel l'administration entraîne une hausse annuelle de vitesse de croissance d'au moins environ 14,37 cm, sur la base d'une analyse intermédiaire après 6 mois de traitement.

6. Polypeptide à CTP modifiés à utiliser selon l'une quelconque des revendications précédentes, dans lequel les doses ultérieures du polypeptide à CTP modifiés peuvent être modifiées à partir de la dose de départ sur la base des niveaux d'IGF-1 mesurés 4 jours après la dose précédente.

7. Polypeptide à CTP modifiés à utiliser selon l'une quelconque des revendications précédentes, dans lequel les niveaux d'IGF-1 comprennent des niveaux d'IGF-1 d'échantillons testés quatre jours après l'administration dudit polypeptide à CTP modifiés.

8. Polypeptide à CTP modifiés à utiliser selon l'une quelconque des revendications précédentes, dans lequel les doses ultérieures du polypeptide à CTP modifiés sont fournies chaque semaine.

9. Polypeptide à CTP modifiés à utiliser selon l'une quelconque des revendications précédentes, dans lequel l'hormone de croissance est une hormone de croissance humaine (hGH).

10. Polypeptide à CTP modifiés à utiliser selon l'une quelconque des revendications précédentes, dans lequel ledit sujet est un sujet naïf.

11. Polypeptide à CTP modifiés à utiliser selon l'une quelconque des revendications précédentes, dans lequel la séquence d'au moins un CTP consiste en des acides aminés de séquence SEQ ID NO:17 ou SEQ ID NO:18.

12. Polypeptide à CTP modifiés à utiliser selon l'une quelconque des revendications précédentes, dans lequel les acides aminés de la séquence-signal sont présentés dans SEQ ID NO:49.

13. Polypeptide à CTP modifiés à utiliser selon l'une quelconque des revendications 1 à 11, dans lequel la séquence d'acides aminés dudit polypeptide à CTP modifiés comprend les acides aminés 27-301 présentés dans SEQ ID NO:39 ou les acides aminés 27-285 de SEQ ID NO:40.

14. Polypeptide à CTP modifiés à utiliser selon l'une quelconque des revendications 1 à 12, dans lequel la séquence d'acides aminés dudit polypeptide à CTP modifiés comprend la séquence présentée dans SEQ ID NO:39 ou SEQ ID NO:40.

15. Polypeptide à CTP modifiés à utiliser selon l'une quelconque des revendications précédentes, dans lequel le polypeptide à CTP modifiés est codé par la séquence d'acides nucléiques présentée dans SEQ ID NO:44 ou SEQ ID NO:45.

16. Polypeptide à CTP modifiés à utiliser selon l'une quelconque des revendications précédentes, dans lequel le polypeptide à CTP modifiés est administré en une dose progressivement augmentée à intervalles de deux semaines jusqu'à atteindre une dose de 0,66 mg/kg/semaine .
